# EUROPEAN PATENT SPECIFICATION

(11) **EP 3 488 015 B1**
(45) Date of publication and mention of the grant of the patent: **21.02.2024**
(21) Application number: 17755068.8
(22) Date of filing: 24.07.2017
(51) Int. Cl.: C12Q 1/689

(54) **HIGHLY POLYMORPHIC AND MODULAR EXTRAGENIC (H.P.M.E.) MARKERS WITHIN SPECIFIC TAXA OF MICROORGANISMS AND USE THEREOF FOR THEIR DIFFERENTIATION, IDENTIFICATION AND QUANTIFICATION**
HOCHPOLYMORPHE UND MODULARE EXTRAGENE MARKER INNERHALB SPEZIFISCHER TAXA VON MIKROORGANISMEN UND IHRE VERWENDUNG ZUR DIFFERENZIERUNG, IDENTIFIZIERUNG UND QUANTIFIZIERUNG
MARQUEURS EXTRAGÈNES MODULAIRES ET HAUTEMENT POLYMORPHES (H.P.M.E.) À L'INTÉRIEUR DE TAXONS SPÉCIFIQUES DE MICRO-ORGANISMES ET LEUR UTILISATION POUR LEUR DIFFÉRENCIATION, LEUR IDENTIFICATION ET LEUR QUANTIFICATION

(30) Priority: 22.07.2016 WO PCT/EP2016/067597
(43) Date of publication of application: 29.05.2019
(73) Proprietor: Microbion S.r.l., 37126 Verona (IT)
(72) Inventor: FRACCHETTI, Fabio, 37066 Sommacampagna (IT); TORRIANI, Sandra, 29121 Piacenza (IT); DEL CASALE, Antonio, 66054 Vasto (IT); FELIS, Giovanna, 37123 Verona (IT)
(74) Representative: Zaccaro, Elisabetta
(86) International application number: PCT/EP2017/068673
(87) International publication number: WO 2018/015572

(56) References cited:
- S. AHMAD ET AL: "A protein-based phylogenetic tree for Gram-positive bacteria derived from hrcA, a unique heat-shock regulatory gene", INTERNATIONAL JOURNAL OF SYSTEMATIC BACTERIOLOGY, vol. 49, no. 4, 1 October 1999 (1999-10-01), pages 1387-1394, XP055419790, GB ISSN: 0020-7713, DOI: 10.1099/00207713-49-4-1387
- WANG TAO ET AL: "Multilocus sequence typing and pulsed-field gel electrophoresis analysis ofOenococcus oenifrom different wine-producing regions of China", INTERNATIONAL JOURNAL OF FOOD MICROBIOLOGY, ELSEVIER BV, NL, vol. 199, 12 January 2015 (2015-01-12), pages 47-53, XP029217160, ISSN: 0168-1605, DOI: 10.1016/J.IJFOODMICRO.2015.01.006
- B. DE LAS RIVAS ET AL: "Allelic Diversity and Population Structure in Oenococcus oeni as Determined from Sequence Analysis of Housekeeping Genes", APPLIED AND ENVIRONMENTAL MICROBIOLOGY, vol. 70, no. 12, 1 December 2004 (2004-12-01), pages 7210-7219, XP055361984, ISSN: 0099-2240, DOI: 10.1128/AEM.70.12.7210-7219.2004
- ANDREAS LECLERQUE ET AL: "Multilocus sequence typing (MLST) for the infra-generic taxonomic classification of entomopathogenic Rickettsiella bacteria", FEMS MICROBIOLOGY LETTERS, vol. 324, no. 2, 20 September 2011 (2011-09-20), pages 125-134, XP055362674, GB ISSN: 0378-1097, DOI: 10.1111/j.1574-6968.2011.02396.x
- Cile Cé ET AL: "Genetic Organization of the mle Locus and Identification of a mleR-Like Gene from Leuconostoc oenos", /96/$04.00ϩ0 Copyright, 1 January 1996 (1996-01-01), pages 4493-4498, XP55361760, Retrieved from the Internet: URL:http://aem.asm.org/content/62/12/4493. full.pdf#page=1&view=FitH
- STEFANIE P. GLAESER ET AL: "Multilocus sequence analysis (MLSA) in prokaryotic taxonomy", SYSTEMATIC AND APPLIED MICROBIOLOGY., vol. 38, no. 4, 1 June 2015 (2015-06-01), pages 237-245, XP055361515, AMSTERDAM, NL ISSN: 0723-2020, DOI: 10.1016/j.syapm.2015.03.007
- L. SOLIERI ET AL: "Development of a Sequence-Characterized Amplified Region Marker-Targeted Quantitative PCR Assay for Strain-Specific Detection of Oenococcus oeni during Wine Malolactic Fermentation", APPLIED AND ENVIRONMENTAL MICROBIOLOGY, vol. 76, no. 23, 1 December 2010 (2010-12-01), pages 7765-7774, XP055419282, ISSN: 0099-2240, DOI: 10.1128/AEM.00929-10
- GROISILLIER A ET AL: "Comparison of partial malolactic enzyme gene sequences for phylogenetic analysis of some lactic acid bacteria species an relationships with the malic enzyme", INTERNATIONAL JOURNAL OF SYSTEMATIC BACTERIOLOGY, SOCIETY FOR GENERAL MICROBIOLOGY, READING, GB, vol. 49, 1 January 1999 (1999-01-01), pages 1417-1428, XP002964486, ISSN: 0020-7713

## Description

### FIELD OF THE INVENTION

The present invention concerns the field of nucleic acid based methods suitable for the generation of tools for biomedical research and biotechnological applications, which allow to differentiate, identify and quantify microorganisms

### STATE OF THE ART

Molecular methods based on DNA sequence are becoming more and more the reference standard in the post-genomic era, thanks to the availability of an increasing number of DNA sequences of prokaryotic and eukaryotic microorganisms for genotyping and for the identification (Gil-Lamaignere *et al.,* 2003; Moore *et al.,* 2010).

The key for applying DNA sequences for the characterization, the identification and quantification of any microorganism is the possibility to find in a limited region that is commonly defined "marker" bringing biological information. Nature Magazine (https://www.nature.com/subjects/genetic-markers) defines genetic markers as "DNA sequences with known physical locations on chromosomes. They are points of variation that can be used to identify individuals or species, or may be used to associate an inherited disease with a gene through genetic linkage with nearby but possibly unidentified or uncharacterised genes". The genetic (DNA) markers applied "to identify individuals or species" share high similarity within a defined "target" group of microorganisms and contemporarily nucleotide variations to differentiate the "target" group from any other.

In the post-genomic era, a paradigm shifting from "genetic marker" to "genomic marker", as well as from "phylogenetics" to "phylogenomics", is in progress, due to the availability of an increasing number of whole genome sequences and also to the availability of new bioinformatics tools for data mining (Capella-Gutierrez *et al.,* 2014). The concept of "genetic marker" and "genomic marker" can be used indeed as synonyms, as they relate on DNA sequences "used to identify individuals or species, or may be used to associate an inherited disease with a gene through genetic linkage".

The size and the characteristics of said DNA region depends on the characteristics of the diagnostic technological platform which the found DNA sequences (marker) will be applied to. For instance Quantitative Real Time PCR (qPCR) to identify and quantify microorganisms requires the amplification of short sequences, possibly in the range of 100 bp and 200 bp (Bustin *et al.,* 2009), while ITS-RFLP to characterize and identify different yeast species is based on DNA sequence sizes usually from 400 bp and 1 050 bp.

The last two examples introduce the concept of "adaptability" of a certain DNA marker, that can be adapted and used for DNA-based fingerprinting, by implementing different protocols that work on diverse technological platforms such as Real Time qPCR and ITS-RFLP.

As it is not possible to verify the specificity of a certain marker candidate for the identification of the putative target group by applying the prototype to all the known microorganisms, the effectiveness and the robustness of the genetic markers need to be verified also by *in-silico* analysis with the support of phylogenomics and phylogenetics. Even if they are different disciplines, phylogenomics and phylogenetics play both a key role, respectively, to find markers and to support the choice of the best candidate marker to differentiate and identify the target microorganisms (Assiss *et al.,* 2014; Capella-Gutierrez *et al.,* 2014).

DNA-based molecular methods target different markers to characterize, identify and quantify microorganisms, depending on the characteristics of the microorganisms and also depending on the characteristics of the marker (Lamaignere *et al.,* 2003; Moore *et al.,* 2010).

The sequence variability is generated by events that can be classified in two main categories: a) nucleotide substitutions which generate Single Nucleotide Polymorphisms (SNPs); b) insertions and/or deletions of nucleotides, mobile elements, tandem repeats which generate length polymorphisms (Shaw, 2013).

The sequence analysis of the genes coding for the small subunit ribosomal RNA (SSU RNA) and large subunit ribosomal RNA (LSU RNA), namely 16S rRNA and 23S rRNA for prokaryotes and 18S rRNA and 26S rRNA for eukaryotes are generally considered the most effective and robust marker for the identification to the species level. Despite this general rule closely phylogenetically related species are characterized by SSU RNA and LSU RNA close to 100% of sequence similarity.

In order to improve the capacity to differentiate and to identify phylogenetically related species and to obtain a higher resolution of the phylogenetic relationship, protein coding genes have been increasingly applied as a molecular markers (Glaeser *et al.,* 2015) in MultiLocus Sequence Analysis (MLSA) and MultiLocus Sequence Typing (MLST). Usually an MLST scheme is constituted by a number ranging from five to ten of ubiquitous genes coding for housekeeping proteins such as enzymes involved in DNA replication, in RNA transcription or as molecular chaperones (Rong *et al.,* 2014).

In some cases different genes coding for enzymes involved in the characteristic metabolism of a microorganism group, or for a gene bringing a well-defined biological information have been chosen. The case-study of the *Rickettsiella genus,* of the *Bifidobacterium genus, Oenococcus oeni* are reported below.

MLST data has been obtained for the classification of bacteria of the genus *Rickettsiella* (Leclerque *et al.* 2011), in which new genetic markers were identified.

MLST was also used to investigate the genetic variation within the *O*. *oeni* species isolated from wines of different origins (de las Rivas *et al.,* 2004). In both these cases, sequence polymorphism markers were analysed.

Also in the new genome-based identification approach for the identification of members of the genus *Bifidobacterium* reported by Ferrario *et al.* (2015) the targets used for the identification are protein coding genes.

The characteristic length polymorphism of the Internal Transcribed Sequence of the ribosomal RNA operon, i.e. a polymorphic region comprised between the genes coding for SSU RNA e LSU RNA is used to differentiate and identify prokaryotic (Thanh *et al.,* 2013) and eukaryotic microorganisms (Esteve & Zarzoso, 1999).

Only Clustered Regularly Interspaced Short Palindromic Repeats (CRISPRs) are a known extragenic molecular markers that combine high sequence variability and high length polymorphism due to the insertion/deletion of nucleotide stretches. CRISPRs have been successfully applied as a molecular marker for typing *Salmonella* spp. isolates (Shariat *et al.,* 2013), but they are limited only to intraspecific typing, because the CRISPRs loci are conserved only between strains of the same species. New molecular markers comprising also highly polymorphic extragenic regions but conserved in different species belonging to the same genus or also in higher taxonomical units would give many advantages because of the possibility to apply the same marker with different approaches and on different technological platform.

### Bioinformatic tools to identify and compare DNA sequence marker

Specific bioinformatics tools are required to identify and compare any DNA sequence marker. From this viewpoint, functional annotation of bacterial genomes is an obligatory and crucially important step of information processing from the genome sequences toward insights into cellular mechanisms and putative ecological roles of a given organism or microbial community. Numerous software packages, databases, platforms, and score filters involve computational pipelines that assign functions to the genes, as DNA sequence information is much more useful when it has been functionally characterized. The function of genes is indeed central for all biological insights, including interpretation and design of experiments, the input data for metabolic and regulatory models development, and comparative genomic analysis to find any genetic marker candidate for microorganisms identification and quantification,. The gene finding problem is therefore an important part of the process for genome annotation but several solutions have been found (Jun *et al.,* 2017). Several gene functions have been universally recognized by the scientific community and stored in specific databases.

A fundamental database for the scientific community is Pfam (Protein families): a large collection of protein families, each represented by multiple sequence alignments and hidden Markov models (HMMs). As proteins are generally composed of one or more functional regions, commonly termed domains, different combinations of domains give rise to the diverse range of proteins found in nature. The identification of domains that occur within proteins can therefore provide insights into their function. Pfam also generates higher-level groupings of related entries, known as clans. A clan is a collection of Pfam entries which are related by similarity of nucleotide and/or aminoacid sequence, structure or profile-HMM (Bateman & Finn, 2007; Mistry *et al.,* 2007; Schaeffer *et al.,* 2017).

A class of proteins that is better functionally characterized in microorganisms is represented by gene regulator, such as Lys-R, CtsR and HrcA.

The LysR family of transcriptional regulators represents the most abundant type of transcriptional regulator in the prokaryotic kingdom. Members of this family have a conserved structure with an Nterminal DNA-binding helix-turn-helix motif and a C-terminal co-inducer-binding domain. LysR-type transcriptional regulators (LTTRs) regulate a diverse set of genes, including those involved in virulence, metabolism, quorum sensing and motility (Maddocks *et al.,* 2008).

The gene regulator HrcA and CtsR have been in-depth characterized in *Bacillus subtilis* and they were found to be play a key role in heat shock regulation (Schumann, 2003) Several other specialized Databases are public and available for discovering possible gene marker (e.g. KEGG, CRISPR web server), but none of the above cited database gives details about the robustness and the effectiveness of the stored genetic markers as a possible target for the identification and quantification of a specific group of microorganisms.

From this viewpoint the molecular markers reported by Ferrario *et al.,* (2015) do not share with all the previously reported markers the possibility to be directly compared one each other by sequence alignment, therefore each new possible *Bifidobacterium* species included in the database required to compare newly the whole genome sequences of the *Bifidobacterium* species to find species-specific as a candidate marker.

The need and importance are increasingly felt for the identification of specific target sequences, which are useful for the identification and quantification of microorganisms and viruses for biomedical and biotechnological purposes.

The present invention therefore regards the identification and development of molecular markers suitable for these purposes. They are characterized by adaptability to diverse technological platforms and can be stored and compared analyzed in specialized databases..

### SUMMARY OF THE INVENTION

The problem underlying the present invention is that of making available methods for the identification of microorganisms.

This problem is resolved by the present finding by the use of compounds capable of functionally blocking one or more genes chosen from the group identified in the attached claims.

The present invention therefore concerns a Highly Polymorphic and Modular Extragenic (HPME) marker, said HPME being a microbial genome (prokaryotic sequence) nucleotide sequence having a length of less than or equal to 2000bp, and said HPME comprising at least one primary protein encoding gene and a secondary flanking extragenic region,

As used herein, the term "Highly Polymorphic and Modular Extragenic marker" or "HPME" refers to a nucleotide sequence comprising at least one Open Reading Frame (ORF) complete or partial and a flanking extragenic region, that is conserved in more than one species, but it shares with the sequence of the other species a similarity of sequence lower than 78%, or it is characterized by insertion/deletion of at least one nucleotide, or both the conditions (a microbial genome nucleotide sequence having a length of less than or equal to 2000bp).

By the term "extragenic region" as used herein a nucleotide sequence that is not comprised in an Open Reading Frame or in a non coding conserved sequence such as rRNA, tRNA, pseuodogenes is intended.

The present invention also relates to the use of the HPME marker, for the identification of the genus and/or the species and/or subspecies or any of the Operational Taxonomic Unit (OTU) of a microorganism.

According to one aspect, the described invention provides a use of the HPME marker, for designing primers and probes and adaptors for the generation of tools for biomedical research and biotechnological applications.

In a further aspect the invention provides a use of the HPME marker for the identification and quantification of a target microorganisms.

In a preferred aspect the invention provides a use of the HPME marker, wherein said identification and quantification of microorganisms is carried out with:
- a target marker consisting of the nonamer sequence TTTGACTAT within the H.P.M.E. *mleA-mleR* marker, for the *Lactobacillales* order;
- the target markers consisting of the sequences ACAAGCCGG, GAGTGCTAAT (SEQ ID NO: 25), AACACGCCAAA (SEQ ID NO: 23), ATTGGAAGGAAAGTA (SEQ ID NO:24), ATTGTATTAGCACTC (SEQ ID NO: 26), within the H.P.M.E. *tkt-hrcA* marker, for the *Bifidobacterium* genus;
- the target markers consisting of the sequence TTTGTTTTTCTTCT, TGTGTTCACCTCCCT within the H.P.M.E. *hrcA-grpE* marker; for the *Bacillus* genus.

A further aspect of the present invention is a target marker consisting of the nonamer sequence TTTGACTAT.

As will be further described in the detailed description of the invention, the HPME of the present invention has the advantages of being variable because of the presence of Single Nucleotide Polymorphisms (SNPs), or because of insertions/deletions, but it is present in different species or different genus and therefore it is possible to compare by means of sequence alignment and is also possible to store in a specific database. It is flexible because it can be adapted and successfully applied to different techniques, protocols and technology platforms. Moreover it is often characterized by two level of specificity, as usually it is polymorphic both regarding the nucleotide content and the length. This is useful for designing DNA-based assays for the identification and the quantification of microorganisms also in mixed cultures.

There is very much a need, therefore, to identify new markers useful in nucleic acid based methods suitable for the generation of tools for biomedical research and biotechnological applications.

### BRIEF DESCRIPTION OF THE DRAWINGS

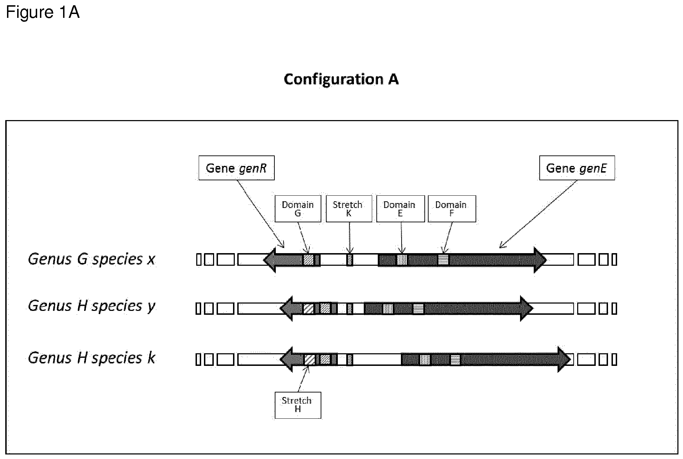

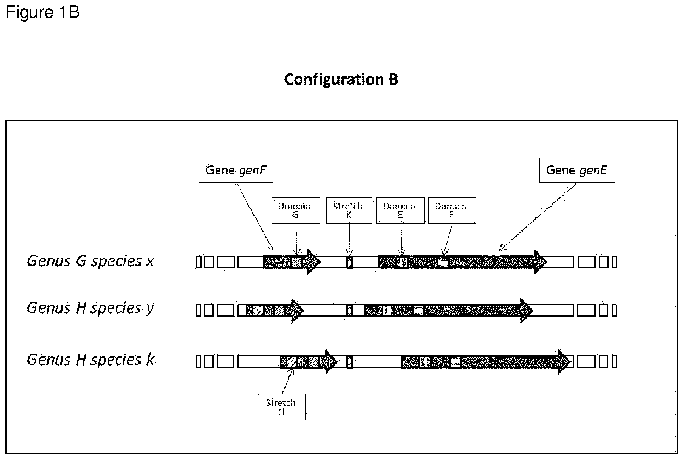

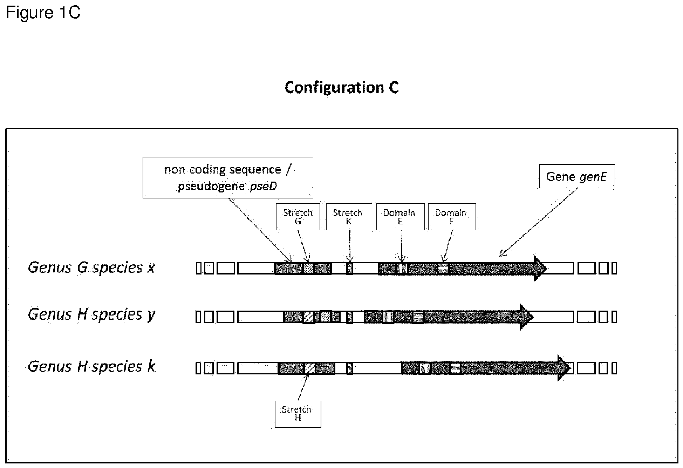

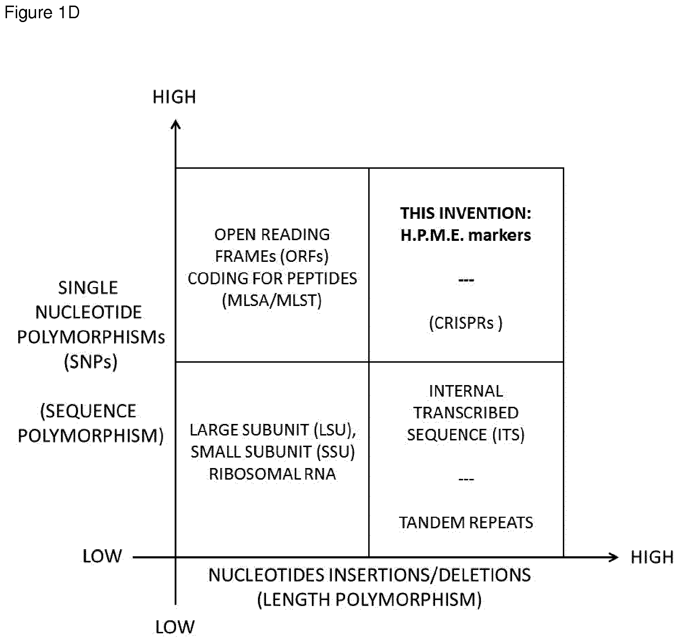

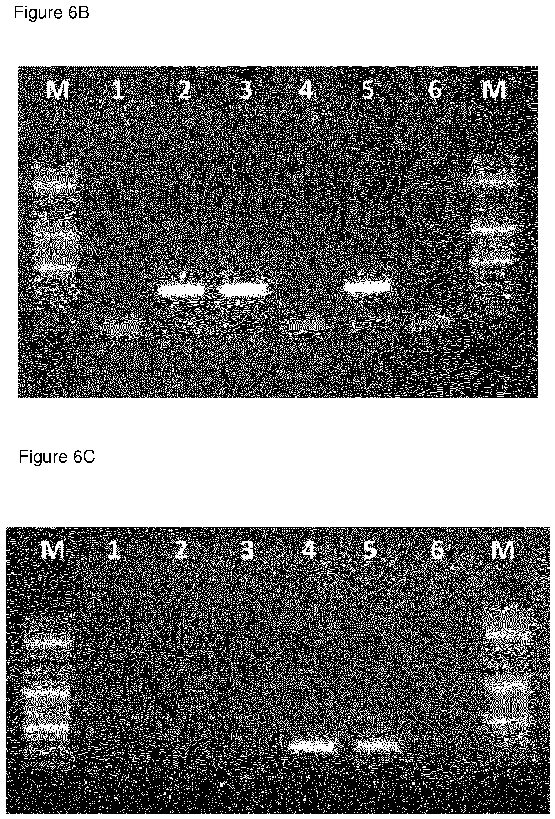

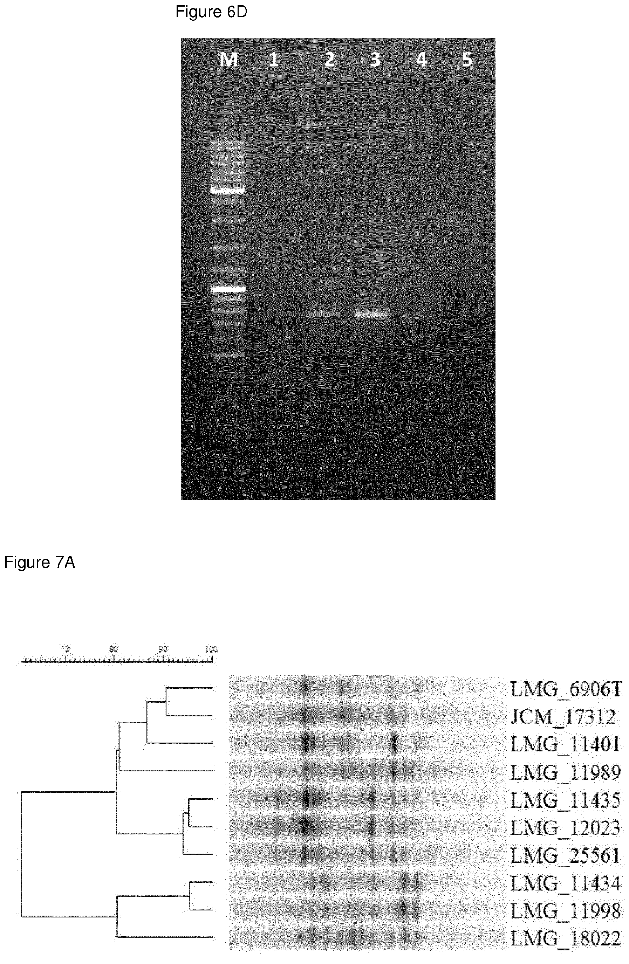

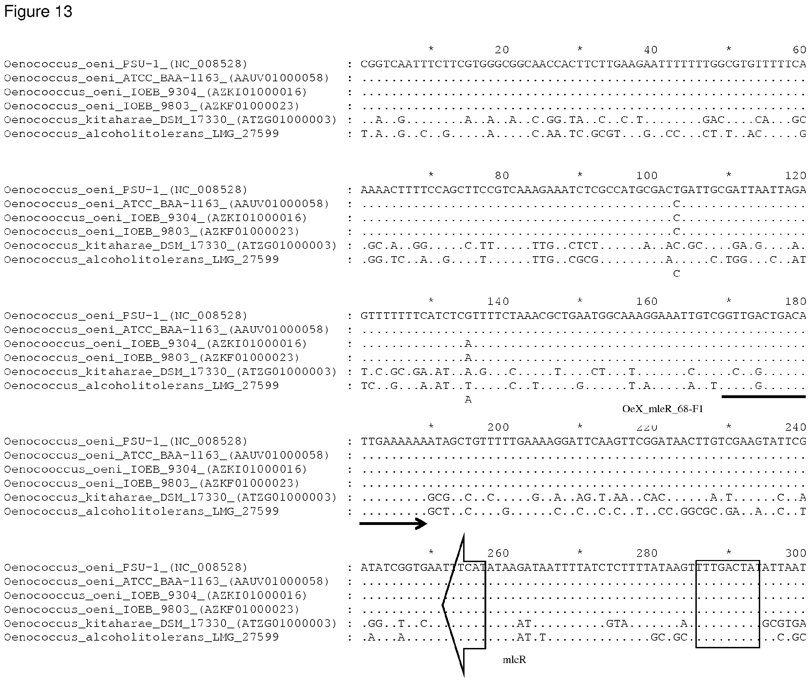

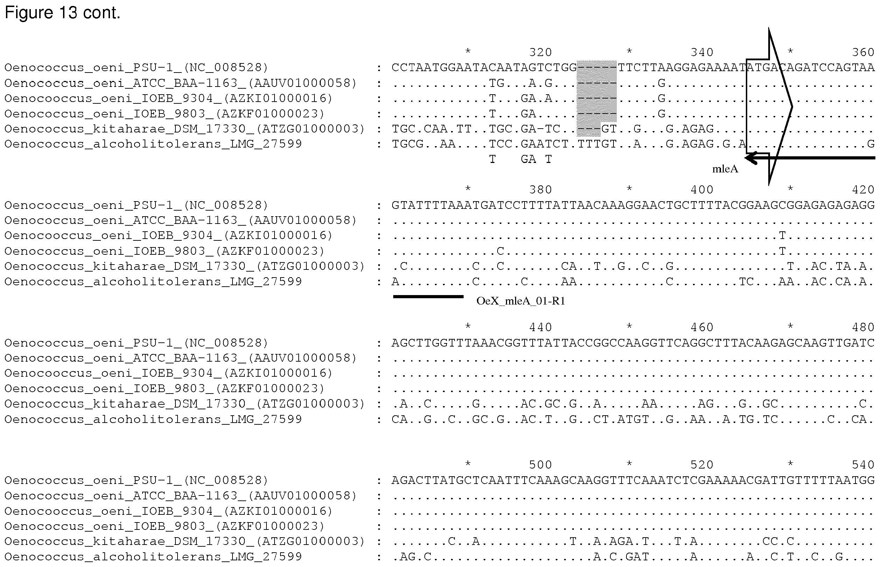

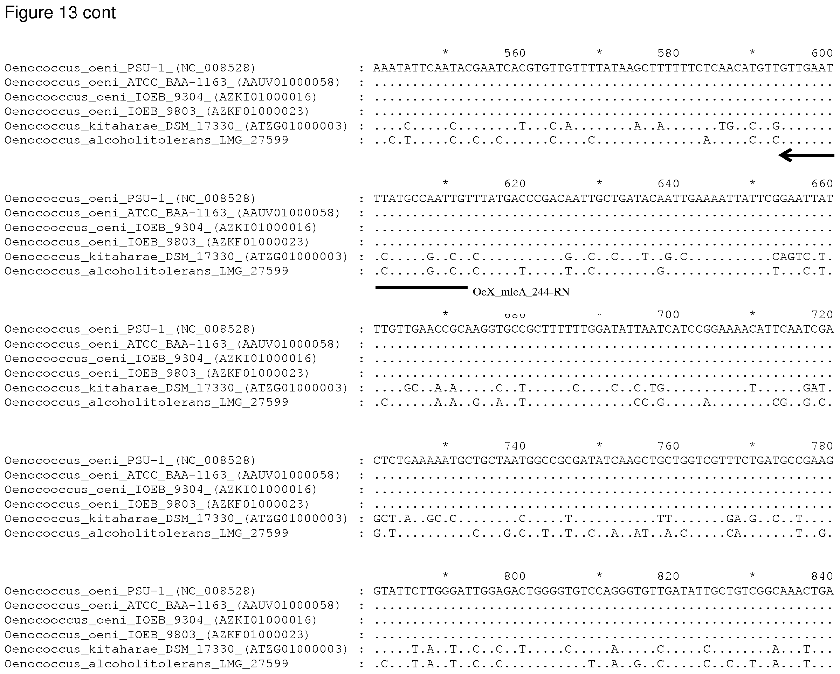

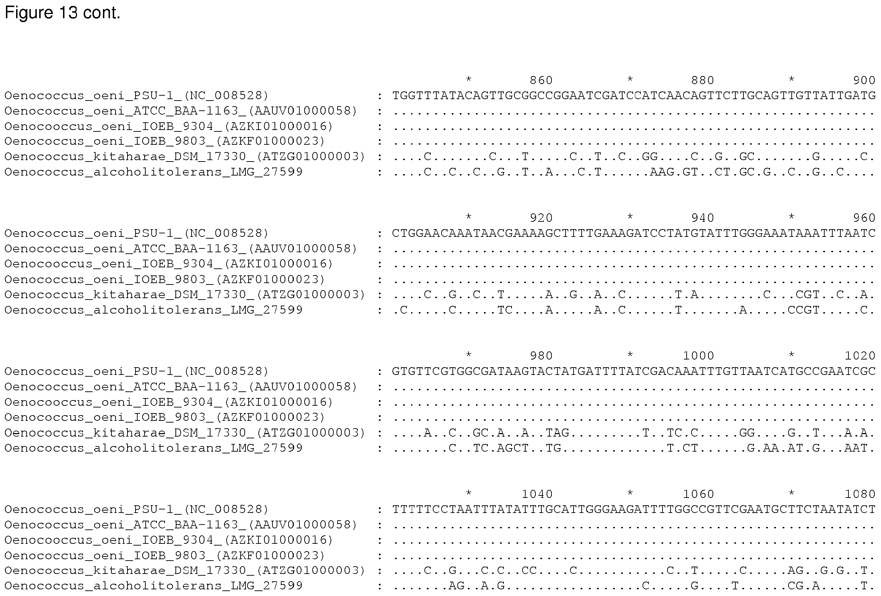

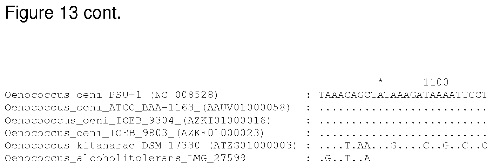

The characteristics and advantages of the invention will now be described in detail from the detailed description which follows, from the Examples given for illustrative and nonlimiting purposes, and in reference to the annexed Figures 1-8, wherein:
**Figure 1** shows schematic representations of the possible configurations of the Highly Polymorphic and Modular Extragenic (H.P.M.E.) markers, which are the subjects of the invention, and their positioning with respect to other well-known markers. In this comparison their effectiveness in differentiating and identify microorganism thanks to both sequence polymorphisms (Single Nucleotide Polymorphisms - SNPs) and length polymorphism (insertions/deletions) has been considered.
**Figure 1A.** In one aspect of the invention (configuration A) the genetic elements comprised in the H.P.M.E. are two genes (Gene *genR* and *genE*) oriented in opposite directions on the two complementary strands of the DNA. This configuration is shared by different microorganisms (and viruses) belonging to different taxa, such as different genera (*Genus G* and *Genus H*) and different species within the same genus (*Genus H species y* and *Genus H species k*)*.* The comparison between the H.P.M.E. marker harboured both by closely related and far related species, by sequence alignment, reveals the presence of conserved domains (Domain G, Domain E and Domain F) coding for specific amino acid sequence and also the presence of conserved nucleotide stretches in both coding (stretch H) and non coding (stretch K) regions. The domains and the stretches, as defined above, can be modularly applied to differentiate, identify and quantify species belonging to the same genus or also to different genus (or higher taxa) according their conservation grade within a specific taxon.
**Figure 1B****.** In another aspect of the invention (configuration B) the genetic elements comprised in the H.P.M.E. are two genes (Gene *genF* and *genE)* oriented in opposite directions on the two complementary strands of the DNA.
**Figure 1C****.** In another aspect of the invention (configuration C) only one gene is comprised in the H.P.M.E. are two genes (*genE*)*,* as the other element is a conserved sequence non coding for a peptide, e.g. a conserved tRNA, rRNA or a pseudogene *(pseD).*
**Figure 1D****.** Scheme of the positioning of the H.P.M.E. markers with respect to other well-known markers already applied. More in detail, it has been considered the effectiveness of H.P.M.E. to differentiate and to identify microorganisms and viruses thanks to both sequence polymorphisms (Single Nucleotide Polymorphisms - SNPs) and length polymorphism (insertions/deletions). H.P.M.E. markers share with protein coding genetic markers an high sequence variability, that is useful to differentiate and to identify very closely related species and subspecies and also strains belonging to the same species thanks to MultiLocus Sequence Typing (MLST) or MultiLocus Sequence Analysis (MLSA). Moreover H.P.M.E. markers share with other extragenic markers such as Internal Transcribed Sequence, an hypervariable region in the ribosomal RNA operon of both prokaryotes and eukaryotes, the possibility to accumulate insertions/deletions and therefore to efficiently applied to differentiate and identify microorganisms thanks to length polymorphism. Also bacterial Clustered Regularly Interspaced Short Palindromic Repeats (CRISPRs) are characterized both by high sequence and length polymorphism, but in their case, the presence of insertions/deletions depends on the history of each strain, therefore they are potentially good markers for strain differentiation and identification, but they are too variable to be compare across different species and different genera.

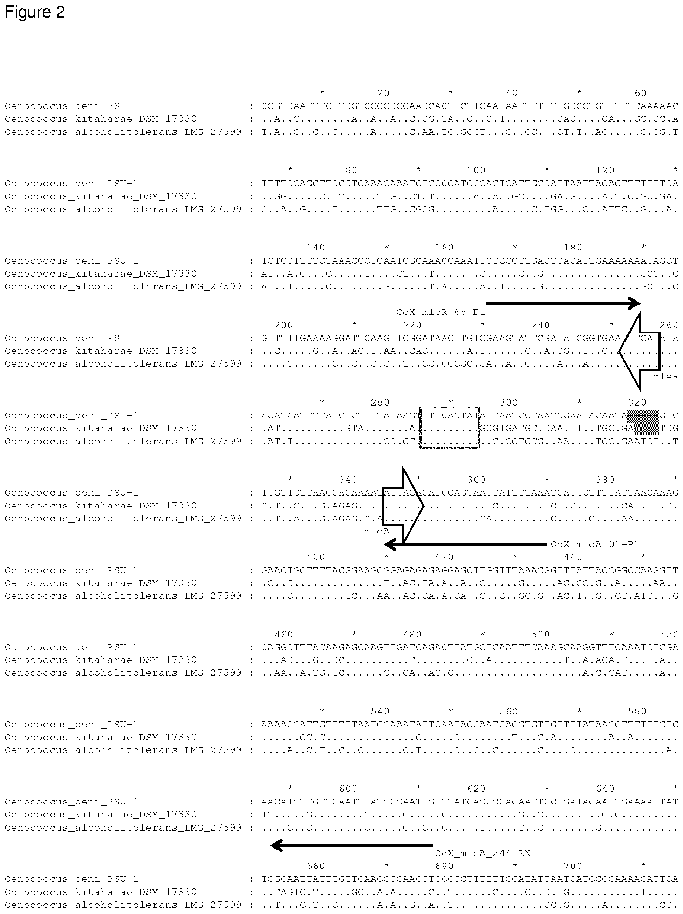

**Figure 2** shows the alignment of the H.P.M.E. *mleA-mleR* marker, constituted by the partial sequence of the *mleR* gene and the *mleA* gene and the complete sequence of the promoter from *Oenococcus oeni, Oenococcus kitaharae* and *Oenococcus alcoholitolerans.* The sequence of the strain *Oenococcus alcoholitolerans* LMG 27599 was obtained with the primers OE_mleR_F1_seq OEX_mleR_F1_seq and OEX_mleR_FN_seq newly designed.
   The alignment is displayed in "similarity mode", therefore in the second and third sequence, the common nucleotides with the first sequence are shown as a dot, whereas the SNPs are shown as a nucleotide. The start codon of the *mleR* and *mleA* genes are included in an empty arrow showing the orientation of the gene, therefore the H.P.M.E. *mleA-mleR* marker is one preferred representation of one H.P.M.E. marker in the Configuration A (Figure A1). The gaps in the promoter region which allow to differentiate the three species by length polymorphism are marked in light grey. The regions that have been used to design the primers (OeX_mleR_68-F1, OeX_mleA_01-R1, OeX_mleA_244-RN) to be applied in different protocols and procedure are located within of the full black arrows and they correspond to the "Domain G", "Domain E" and "Domain F" depicted in the Figure 1A. The conserved sequence "TTTGACTAT" from the position 86 to the position 94 of the alignment and surrounded by a grey frame it is known to be shared also by other Lactic Acid Bacteria, e.g. *Lactobacillus, Leuconostoc* and *Pediococcus,* and corresponds to the "stretch K" of the Figure 1A and it is used to design probes constituted by DNA oligonucleotides or other XNA oligonucleotides.

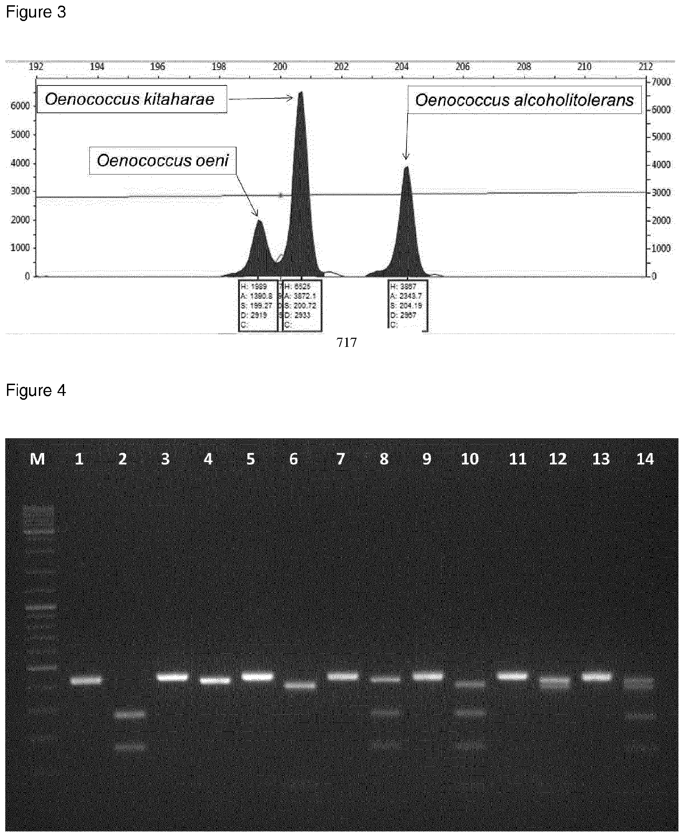

**Figure 3** shows the size difference between the PCR products of *Oenococcus oeni, Oenococcus kitaharae* and *Oenococcus alcoholitolerans* obtained with the primers OeX_mleR_68-F1 and OeX_mleA_01-R1, designed respectively on the sequence of the *mleR* gene and *mleA* gene. The forward primer OeX_mleR_68-F1 was labelled with FAM and the purified PCR products were separated by capillary electrophoresis to estimate the size of the PCR products that are 199 bp, 200 bp and 204 bp for *Oenococcus oeni, Oenococcus kitaharae* and *Oenococcus alcoholitolerans,* as expected according the alignment in Figure 2.
**Figure 4** shows the result of the Hae III PCR-RFLP assay applied to the H.P.M.E. *mleA-mleR* marker after the electrophoresis migration on a 3% weight/volume agarose in TAE buffer. More in detail, the PCR products obtained for *Oenococcus oeni, Oenococcus kitaharae* and *Oenococcus alcoholitolerans* with the primers OeX_mleR_68-F1 and OeX_mleA_244-RN were followed by the digestion with the Hae III Restriction Enzyme. The three species have been differentiated by electrophoresis on agarose gel thanks to the sequence variability of the H.P.M.E. *mleA-mleR* marker displayed by means of the Hae III digestion. The three species can be differentiated also when they are present together in a mixture.
   The sample list is as follows: M) size marker O'GeneRuler DNA Ladder Mix, Ready-to-Use 100 to 10.000 bp (Thermo Scientific); 1) *O. oeni* DSM 20477; 2) *O. oeni* DSM 20477 digested with Hae III; 3) *O. kitaharae* DSM 17330; 4) *O. kitaharae* DSM 17330 digested with Hae III; 5) *O. alcoholitolerans* LMG 27599; 6) *O. alcoholitolerans* LMG 27599 digested with Hae III; 7) *O. oeni* DSM 20477 and *O. kitaharae* DSM 17330; 8) *O. oeni* DSM 20477 and *O. kitaharae* DSM 17330 digested with Hae III; 9) *O. oeni* DSM 20477 and *O. alcoholitolerans* LMG 27599; 10) *O. oeni* DSM 20477 and *O. alcoholitolerans* LMG 27599 digested with Hae III; 11) *O*. *kitaharae* DSM 17330 *O. alcoholitolerans* LMG 27599; 12) *O. kitaharae* DSM 17330 *O. alcoholitolerans* LMG 27599 digested with Hae III; 13) *O*. *oeni* DSM 20477, *O*. *kitaharae* DSM 17330 and *O*. *alcoholitolerans* LMG 27599; 14) *O. oeni* DSM 20477, *O. kitaharae* DSM 17330 and *O. alcoholitolerans* LMG 27599 digested with Hae III;

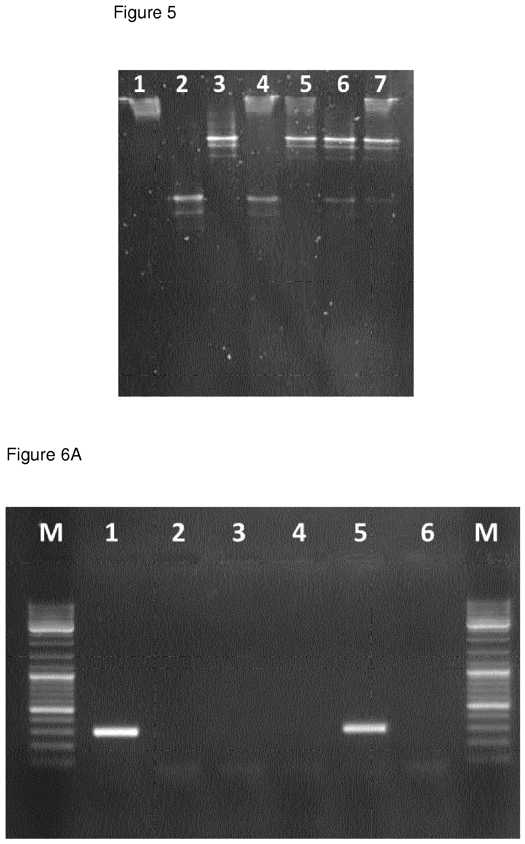

**Figure 5** shows the result of the PCR-DGGE, after the electrophoresis migration on the Denaturating Gel with Formammide concentration ranging from 20% to 40%. The PCR products loaded were obtained with the primer Oex_mleR_68_F2-GC (with the addition of the GC-clamp at the 5'-end) and Oex_mleA_01_R1. The migration profile of the PCR products depends on their sequence as the double-helix of the PCR product is denaturated by formammide depending on their nucleotide composition. The three species can be clearly differentiated also when they are present together in a mixture. This experiment clearly shows that the H.P.M.E. *mleA-mleR* marker can be applied for culture-independent ecological studies and for Metagenomics (also referred to as environmental and community genomics).
   The sample list is as follows: *O*. *oeni* DSM 20477; 2) *O. kitaharae* DSM 17330; 3) *O*. *alcoholitolerans* LMG 27599; 4) *O. oeni* DSM 20477 and *O*. *kitaharae* DSM 17330; 5) *O*. *oeni* DSM 20477 and *O. alcoholitolerans* LMG 27599; 6) *O. kitaharae* DSM 17330 *O*. *alcoholitolerans* LMG 27599; 7) *O. oeni* DSM 20477, *O. kitaharae* DSM 17330 and *O*. *alcoholitolerans* LMG 27599
**Figure 6** shows the results of the electrophoresis migration of the PCR products obtained for the four species of lactobacilli belonging to the *Lactobacillus casei group* (*Lactobacillus casei, Lactobacillus paracasei* subsp. *paracasei, Lactobacillus paracasei* subsp. *tolerans, Lactobacillus rhamnosus*) according three different PCRs developed by Ward & Timmins (1999) and one PCR using primers designed on H.P.M.E. *mleA-mleR* marker in the framework of this invention.
**Figure 6A** shows the results of the electrophoresis migration on a 1,5% weight/volume agarose in TAE buffer of the PCR product obtained with the primer CASEI - Y2 specific for *Lactobacillus casei.* The sample list is: M) size marker O'GeneRuler DNA Ladder Mix, Ready-to-Use 100 to 10.000 bp (Thermo Scientific); 1) *L. casei* DSM 20011, 2) *L. paracasei* subsp. *paracasei* DSM 5622; 3) *L. paracasei* subsp. *tolerans* DSM 20258; 4) *L. rhamnosus* DSM 20021; 5) *L. casei* DSM 20011, *L paracasei* subsp. *paracasei* DSM 5622; *L. paracasei* subsp. *tolerans* DSM 20258, *L. rhamnosus* DSM 20021; 6) No Template Control.
**Figure 6B** shows the results of the electrophoresis migration on a 1,5% weight/volume agarose in TAE buffer of the PCR product obtained with the primer PARA - Y2 specific for *Lactobacillus casei.* The sample list is: M) size marker O'GeneRuler DNA Ladder Mix, Ready-to-Use 100 to 10.000 bp (Thermo Scientific); 1) *L. casei* DSM 20011, 2) *L. paracasei* subsp. *paracasei* DSM 5622; 3) *L. paracasei* subsp. *tolerans* DSM 20258; 4) *L. rhamnosus* DSM 20021; 5) *L. casei* DSM 20011, *L paracasei* subsp. *paracasei* DSM 5622; *L. paracasei* subsp. *tolerans* DSM 20258, *L. rhamnosus* DSM 20021; 6) No Template Control.
**Figure 6C** shows the results of the electrophoresis migration on a 1,5% weight/volume agarose in TAE buffer of the PCR product obtained with the primer RHAMN - Y2 specific for *Lactobacillus casei.* The sample list is: M) size marker O'GeneRuler DNA Ladder Mix, Ready-to-Use 100 to 10.000 bp (Thermo Scientific); 1) *L. casei* DSM 20011, 2) *L. paracasei* subsp. *paracasei* DSM 5622; 3) *L. paracasei* subsp. *tolerans* DSM 20258; 4) *L. rhamnosus* DSM 20021; 5) *L. casei* DSM 20011, *L paracasei* subsp. *paracasei* DSM 5622; *L. paracasei* subsp. *tolerans* DSM 20258, *L. rhamnosus* DSM 20021; 6) No Template Control.
**Figure 6D** shows the results of the electrophoresis migration on a 1,5% weight/volume agarose gel in TAE buffer of the PCR product obtained with only one PCR with the primer mleR_casG_F1 and mleA_casG_R1, designed on the sequences of the H.P.M.E. *mlea-mleR* marker of the species *Lactobacillus casei, Lactobacillus paracasei* subsp. *paracasei, Lactobacillus paracasei* subsp. *tolerans, Lactobacillus rhamnosus.* The three species *L. casei, L paracasei* and *L. rhamnosus* can be differentiated with only one PCR. As the PCR products show different sizes, these primers guarantee more specificity and they allow to differentiate the three species also in a mixture.
   The sample list is: M) size marker O'GeneRuler DNA Ladder Mix, Ready-to-Use 100 to 10.000 bp (Thermo Scientific); 1) *L. casei* DSM 20011, 2) *L. paracasei* subsp. *paracasei* DSM 5622; 3) *L. paracasei* subsp. *tolerans* DSM 20258; 4) *L. rhamnosus* DSM 20021.

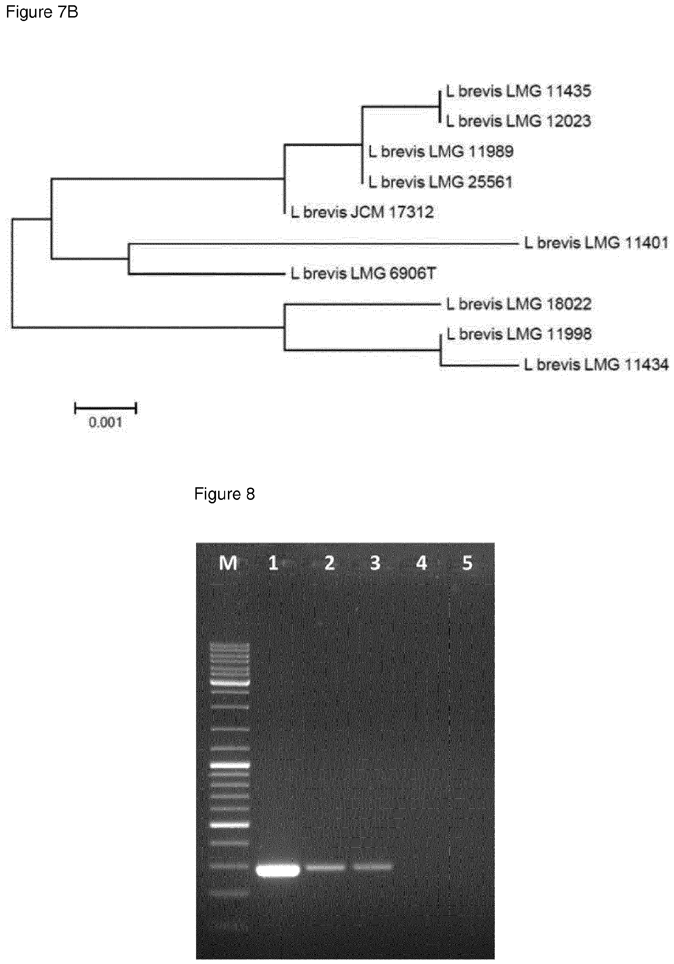

**Figure 7** shows the comparison of two techniques commonly applied for the fingerprinting of different bacterial strains. The first technique is RAPD-PCR, the second is sequencing of a variable genic *locus* followed by alignment and comparison. The fingerprinting profile obtained by sequencing H.P.M.E. *mleA-mleR* marker is compared here with the profile analysis of the RAPD-PCR with the primer 1281 according Akopyanz *et al.* (1991), on a collection of ten different *of Lactobacillus brevis* strains: *L. brevis* strains LMG 6906; *L*. *brevis* JCM 17312; *L. brevis* 11401; *L. brevis* 11989; *L. brevis* 11435; *L. brevis* 12023; *L*. *brevis* 25561; *L. brevis* 11434; *L. brevis* LMG 11998; *L. brevis* LMG 18022.
**Figure 7A** shows the dendrogram obtained by analysing with GelCompar software (AppliedMaths) the RAPD-PCR profiles obtained with the primer 1281 for the ten *L. brevis* strains. Two main clusters are clearly shown and they are divided by a percentage similarity calculated with the UPGMA method lower than 80%. In one cluster the strains *L. brevis* 11434; *L. brevis* LMG 11998; *L. brevis* LMG 18022 are included. In the other main group the remaining seven strains are clustered together.
**Figure 7B** shows the phylogenetic tree obtained from the alignment of the sequences of the H.P.M.E. *mleA-mleR* marker, with the Maximun likelihood statistical method, Tamura-Nei substitution model and complete deletion mode (No. of bootstrap replications: 1000) Two main clusters are clearly shown and two isolated long-branching samples. One group is constituted by the strains *L. brevis* 11434; *L. brevis* LMG 11998; *L. brevis* LMG 18022. The other main group includes the strains *L. brevis* 11435; *L. brevis* 12023; *L. brevis* 11989 and *L. brevis* 25561. The remaining two strains *L. brevis* LMG 11401 and *L. brevis* LMG 6906 are isolated strains.
**Figure 8** shows the results of the electrophoresis migration of the PCR products obtained for three species of bifidobacteria commonly used for industrial purposes (*Bifidobacterium* breve, *Bifidobacterium animalis* subsp. *animalis, Bifidobacterium animalis* subsp. *lactis, Bifidobacterium longum* subsp. *longum,*) according the PCR specific for *Bifidobacterium breve* reported by Matsuki *et al.* (1999).The sample list is: M) size marker O'GeneRuler DNA Ladder Mix, Ready-to-Use 100 to 10.000 bp (Thermo Scientific); 1) *Bifidobacterium breve* DSM 20213; 2) *Bifidobacterium animalis* subsp. *animalis* DSM 2014; 3) *Bifidobacterium animalis* subsp. *lactis* DSM 10140; 4) *Bifidobacterium longum* subsp. *longum;* 5) No Template Control.

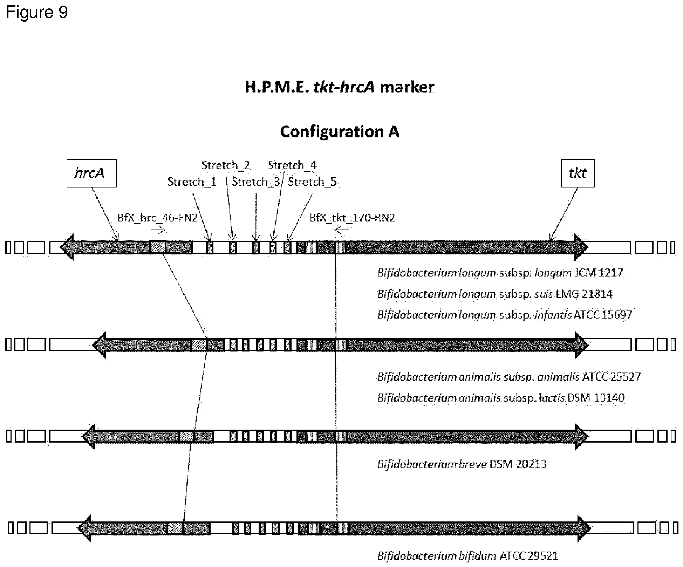

**Figure 9** shows the configuration of the H.P.M.E. *tkt-hrcA* marker, constituted by the partial sequence of the *tkt* gene and the *hrcA* gene and the complete sequence of the gene promoter from *Bifidobacterium breve, Bifidobacterium animalis* subsp. *animalis, Bifidobacterium animalis* subsp. *lactis, Bifidobacterium longum* subsp. *longum, Bifidobacterium longum* subsp. *infantis, Bifidobacterium longum* subsp. *suis* and *Bifidobacterium bifidum.* The genes *tkt* and *hrcA* are oriented in opposite directions on the two complementary strands of the DNA. The DNA sequences BfX_tkt-hrcA_Stretch_1 ACAAGCCGG, BfX_tkt-hrcA_Stretch_2 GAGTGCTAAT, BfX_tkt-hrcA_Stretch_3 AACACGCCAAA (SEQ ID NO: 23), BfX_tkt-hrcA_Stretch_4 ATTGGAAGGAAAGTA (SEQ ID NO:24), BfX_tkt-hrcA_Stretch_5 ATTGTATTAGCACTC (SEQ ID NO: 26) in the promoter region they are also present in the PCR product amplified with the primers BfX_hrc_46-FN2 TARTCYTCYACVAYRGCSCGMAG (SEQ ID NO: 65) and BfX_tkt_170-RN2 TCRTTCGGATCRTGCTTGATG (SEQ ID NO: 66).

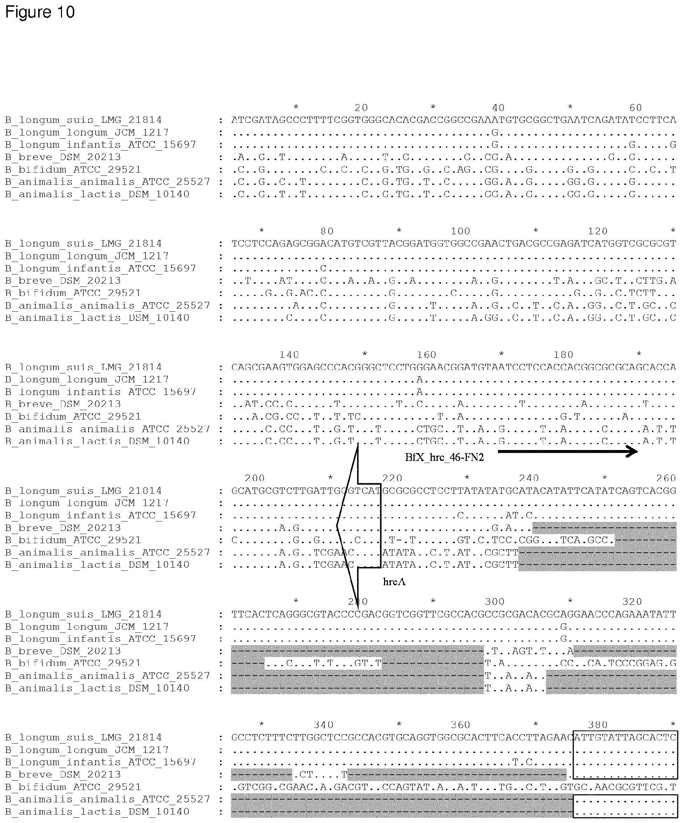

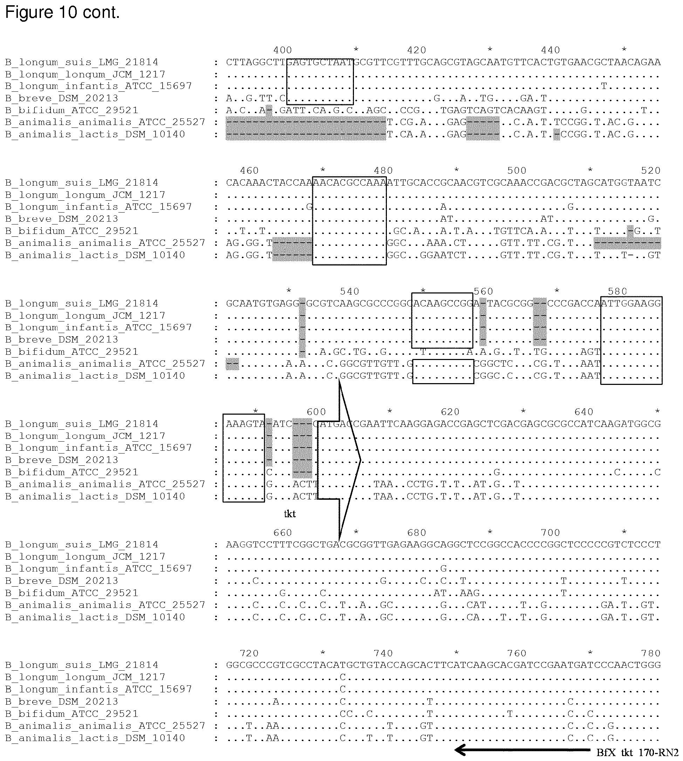

**Figure 10** shows the alignment of the H.P.M.E. *tkt-hrcA* marker, constituted by the partial sequence of the *tkt* gene and the *hrcA* gene and the complete sequence of the gene promoter from *Bifidobacterium breve, Bifidobacterium animalis* subsp. *animalis, Bifidobacterium animalis* subsp. *lactis, Bifidobacterium longum* subsp. *longum, Bifidobacterium longum* subsp. *infantis, Bifidobacterium longum* subsp. *suis* and *Bifidobacterium bifidum.*
   The alignment is displayed in "similarity mode", therefore in the sequence from the second to the sixth, the common nucleotides with the first sequence are shown as a dot, whereas the SNPs are shown as a nucleotide. The start codon of the *hrcA* and *tkt* genes are included in an empty arrow showing the orientation of the gene, therefore the H.P.M.E. *tkt-hrcA* marker is another preferred representation of one H.P.M.E. marker in the Configuration A (Figure A1). The gaps in the promoter region which allow to differentiate the three species by length polymorphism are marked in light grey, while the gaps differentiating *Bifidobacterium animalis* subsp. *animalis, Bifidobacterium animalis* subsp. *lactis* are marked in grey. The preferred regions used to design the primers and probes (BfX_hrc_46-FN2 BfX_tkt_170-RN2) to be applied in different protocols and procedure are located within of the full black arrows and they correspond to the "Domain G", "Domain E" and "Domain F" depicted in the Figure 1A. The conserved sequences surrounded by a grey frame within the positions from 376 to 390, from 400 to 409, from 470 to 480, from 539 to 557 and from 577 to 591 the position of the alignment corresponds to the BfX_tkt-hrcA_Stretch_1, BfX_tkt-hrcA_Stretch_2, BfX_tkt-hrcA_Stretch_3, BfX_tkt-hrcA_Stretch_4, BfX_tkt-hrcA_Stretch_5 of the Figure 9 and it is used to design probes constituted by DNA oligonucleotides or other XNA oligonucleotides.

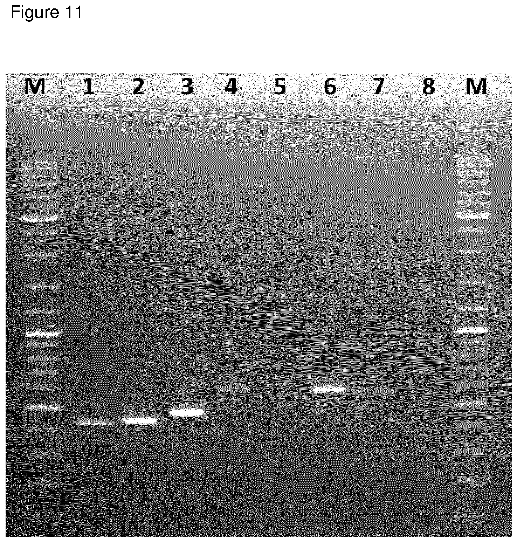

**Figure 11** shows the results of the electrophoresis migration of the PCR products obtained for four species of bifidobacteria commonly used for industrial purposes (*Bifidobacterium* breve, *Bifidobacterium animalis* subsp. *animalis, Bifidobacterium animalis* subsp. *lactis, Bifidobacterium longum* subsp. *longum*,) with the primers BfX_hrc_46-FN2 and BfX_tkt_170-RN2. The sample list is: M) size marker O'GeneRuler DNA Ladder Mix, Ready-to-Use 100 to 10.000 bp (Thermo Scientific); 1) *B. animalis animalis* DSM 20104; 2) *B. animalis lactis* DSM 10140; 3) *B. breve* DSM 20213; 4) *B*. *longum longum* DSM 20097; 5) *B. longum infantis* DSM 20088; 6) *B. longum suis* LMG 21814; 7) *B. bifidum* 20456; 8) No Template Control.

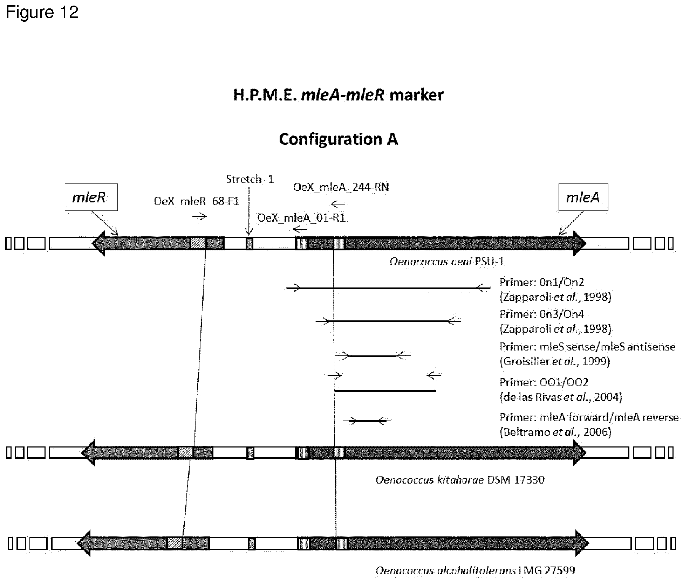

**Figure 12** shows the alignment of the H.P.M.E. *mleA-mleR* marker, constituted by the partial sequence of the *mleR* gene and the complete sequence of the *mleA* gene and the of the promoter from *Oenococcus oeni, Oenococcus kitaharae* and *Oenococcus alcoholitolerans.*
   The alignment is displayed in "similarity mode", therefore in the sequences other than the first (O. oeni PSU-1), the common nucleotides with the first sequence are shown as a dot, whereas the SNPs are shown as a nucleotide. The start codon of the *mleR* and *mleA* genes are included in an empty arrow showing the orientation of the gene. The gaps in the promoter region which allow to differentiate the three species by length polymorphism are marked in light grey. The regions that have been used to design the primers (OeX_mleR_68-F1, OeX_mleA_01-R1, OeX_mleA_244-RN) to be applied in different protocols and procedure are located within of the full black arrows. The conserved sequence TTTGACTAT of the alignment and surrounded by a grey frame it is known to be shared also by other Lactic Acid Bacteria, e.g. *Lactobacillus, Leuconostoc* and *Pediococcus,* and corresponds to the "Stretch_1" of the Figure 13 and it is used to design a LNA TaqMan^{®} probe targeting *Lactobacillales* order as reported in Example 11, Table 6.
**Figure 13** shows the configuration of the H.P.M.E. *mleA-mleR* marker, constituted by the partial sequence of the *mleA* gene and the *mleR* gene and the complete sequence of the gene promoter from *Oenococcus oeni* PSU-1 *Oenococcus kitaharae* DSM 17330 and *Oenococcus alcoholitolerans* LMG 27599. The genes *mleR* and *mleA* are oriented in opposite directions on the two complementary strands of the DNA. In the promoter region the Stretch_1 corresponding to the conserved sequence TTTGACTAT is comprised in the PCR product obtained with the primer pair OeX_mleR_68-F1 and OeX_mleA_01-R1 and with the primer pair OeX_mleR_68-F1 and OeX_mleA_244-RN, but it is not comprised in any other PCR product reported in the prior art and obtained with primers targeting only *mleA* gene.
   Moreover both the primer pair OeX_mleR_68-F1/OeX_mleA_01-R1 and OeX_mleR_68-F1 / OeX_mleA_244-RN allow to differentiate the three species *O*. *oeni, O kitaharae* and *O. alcoholitolerans* both by sequence polymorphisms (SNPs) and by length polymorphisms (insertion/deletion).

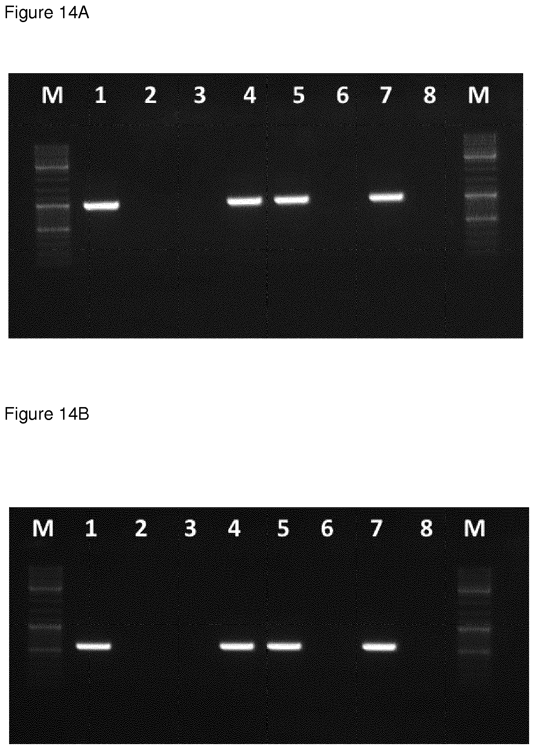

**Figure 14A** shows the results of the electrophoresis migration in 1.5% w/v agarose gel of the products resulting from the first step of the nested-PCR with the primer pair On1 and On2 according Zapparoli *et al.,* (1998) for the three species of the *Oenococcus* genus, using both DNA from pure cultures and mixed DNA. The sample list is as follows: *O. oeni* DSM 20477; 2) *O. kitaharae* DSM 17330; 3) *O. alcoholitolerans* LMG 27599; 4) *O. oeni* DSM 20477 and *O. kitaharae* DSM 17330; 5) *O. oeni* DSM 20477 and *O*. *alcoholitolerans* LMG 27599; 6) *O. kitaharae* DSM 17330 *O. alcoholitolerans* LMG 27599; 7) *O. oeni* DSM 20477, *O. kitaharae* DSM 17330 and *O. alcoholitolerans* LMG 27599; 8) No Template Control.
**Figure 14B** shows the results of the electrophoresis migration in 1.5% w/v agarose gel of the products resulting from the second step of the nested-PCR with the primer pair On3 and On4 according Zapparoli *et al.,* (1998) for the three species of the *Oenococcus* genus, using both DNA from pure cultures and mixed DNA. The sample list is as follows: *O. oeni* DSM 20477; 2) *O. kitaharae* DSM 17330; 3) *O. alcoholitolerans* LMG 27599; 4) *O. oeni* DSM 20477 and *O. kitaharae* DSM 17330; 5) *O. oeni* DSM 20477 and *O*. *alcoholitolerans* LMG 27599; 6) *O. kitaharae* DSM 17330 *O. alcoholitolerans* LMG 27599; 7) *O. oeni* DSM 20477, *O. kitaharae* DSM 17330 and *O. alcoholitolerans* LMG 27599; 8) No Template Control.

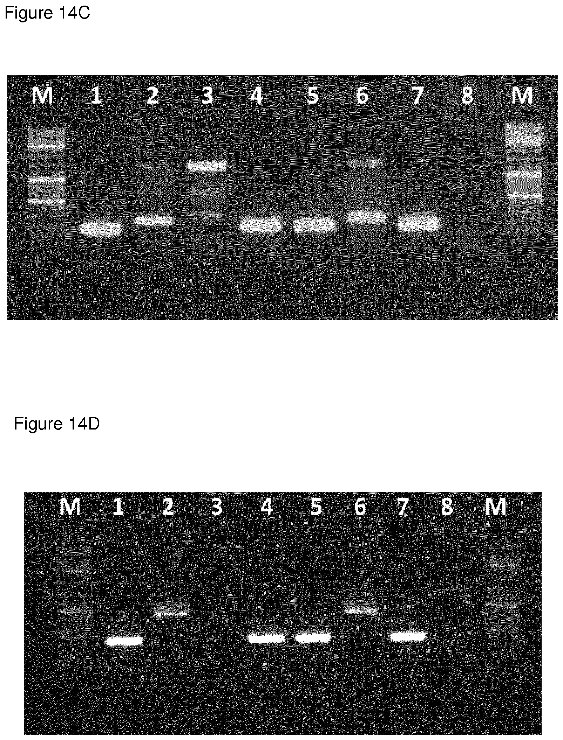

**Figure 14C** shows the results of the electrophoresis migration in 1.5% w/v agarose gel of the PCR products obtained with the primer pair *mleS* sense and *mleS* antisense for the amplification of the gene *mleS* coding for Malolactic Enzyme, according Groisilier *et al*., (1999) for the three species of the *Oenococcus* genus, using both DNA from pure cultures and mixed DNA. The sample list is as follows: *O*. *oeni* DSM 20477; 2) *O*. *kitaharae* DSM 17330; 3) *O. alcoholitolerans* LMG 27599; 4) *O. oeni* DSM 20477 and *O*. *kitaharae* DSM 17330; 5) *O. oeni* DSM 20477 and *O. alcoholitolerans* LMG 27599; 6) *O*. *kitaharae* DSM 17330 *O. alcoholitolerans* LMG 27599; 7) *O. oeni* DSM 20477, *O*. *kitaharae* DSM 17330 and *O. alcoholitolerans* LMG 27599; 8) No Template Control. The electrophoresis migration clearly shows not specific products when the primers are applied to amplify *O. kitaharae* and *O. alcoholitolerans.*
**Figure 14D** shows the results of the electrophoresis migration in 1.5% w/v agarose gel of the PCR products obtained with the primer pair 001 and 002 for the amplification of the gene *mleA* coding for Malolactic Enzyme, according de las Rivas *et al.,* (2004) for the three species of the *Oenococcus* genus, using both DNA from pure cultures and mixed DNA. The sample list is as follows: *O*. *oeni* DSM 20477; 2) *O. kitaharae* DSM 17330; 3) *O. alcoholitolerans* LMG 27599; 4) *O. oeni* DSM 20477 and *O. kitaharae* DSM 17330; 5) *O. oeni* DSM 20477 and *O. alcoholitolerans* LMG 27599; 6) *O. kitaharae* DSM 17330 *O*. *alcoholitolerans* LMG 27599; 7) *O. oeni* DSM 20477, *O. kitaharae* DSM 17330 and *O*. *alcoholitolerans* LMG 27599; 8) No Template Control.
   The electrophoresis migration clearly shows not specific products when the primers are applied to amplify *O*. *kitaharae,* while *O. alcoholitolerans* gives no result.

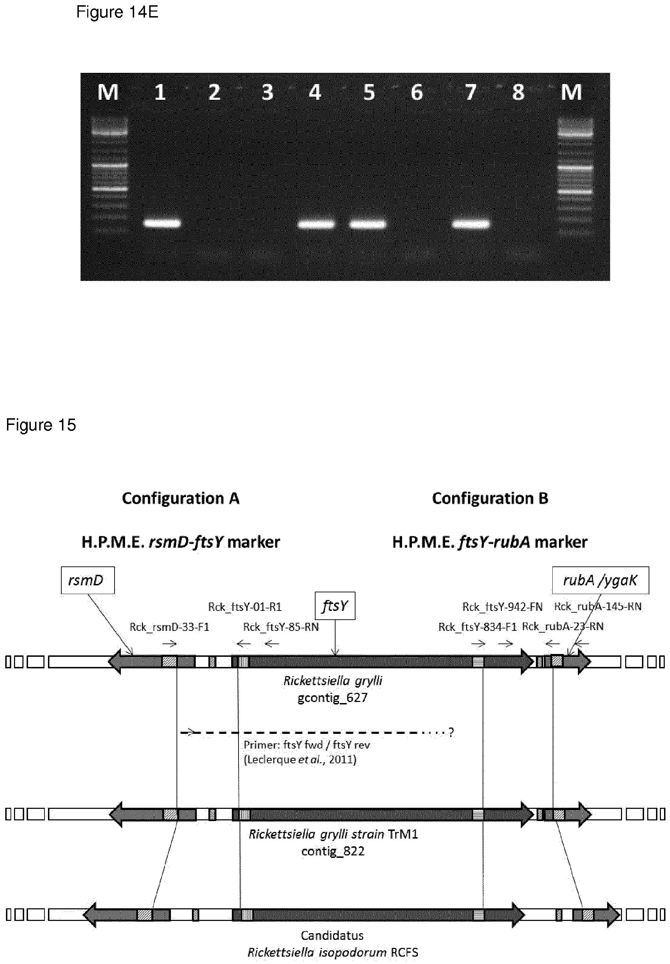

**Figure 14E** shows the results of the electrophoresis migration in 1.5% w/v agarose gel of the PCR products obtained with the primer pair *mleA* "forward primer" and *mleA* "reverse primer" for the amplification of the gene *mleA* coding for Malolactic Enzyme, according Beltramo *et al.* (2006) for the three species of the *Oenococcus* genus, using both DNA from pure cultures and mixed DNA. The sample list is as follows: *O*. *oeni* DSM 20477; 2) *O. kitaharae* DSM 17330; 3) *O. alcoholitolerans* LMG 27599; 4) *O. oeni* DSM 20477 and *O. kitaharae* DSM 17330; 5) *O. oeni* DSM 20477 and *O. alcoholitolerans* LMG 27599; 6) *O. kitaharae* DSM 17330 *O. alcoholitolerans* LMG 27599; 7) *O. oeni* DSM 20477, *O. kitaharae* DSM 17330 and *O. alcoholitolerans* LMG 27599; 8) No Template Control.
   The electrophoresis migration clearly shows that only *O*. *oeni* gives results both when the DNA is present singly and when it is in a mixed solution.
**Figure 15** shows the configuration of two H.P.M.E. markers in *Rickettsiella genus* comprising the *ftsY* gene, that is known as a effective genetic marker for identification and phylogenetic analysis of *Rickettsiella* isolates, and its flanking regions. The first case is H.P.M.E. *rsmD-ftsY* marker, constituted by the partial sequence of the *rsmD* gene coding for 16S rRNA Methyltransferase, oriented in the opposite direction of *ftsY,* which is the gene coding for the signal recognition particle receptor subunit alpha involved in protein translocation. The H.P.M.E. *rsmD-ftsY* marker is characterized by the Configuration A according the definition in Figure 1. The second case is represented by the H.P.M.E *ftsY-rubA* marker, constituted by the gene *ftsY* and the gene coding for Rubredoxin that is identified both with the name *rubA* and *ygaK* and it is characterized by the Configuration B, according the definition in Figure 1.
   These two H.P.M.E. markers allow to differentiate *Rickettsiella grylli* and *Candidatus Rickettsiella isopodorum* on the basis of the *in-silico* comparative analysis of the three available genome sequences for this genus.
   More in detail, it is possible to differentiate the two species thanks to the H.P.M.E. *rsmD-ftsY* marker by analyzing the region comprised within the primer Rck_rsmD-33-FN and Rck_ftsY-01-R1 or within the primer Rck_rsmD-33-FN and Rck_ftsY-85-RN. The regions defined by these two primer pairs are indeed variable both considering the nucleotide sequence and the size, because of the presence of insertion/deletion nucleotide sites. Analogously it is possible to differentiate *Rickettsiella grylli* and *Candidatus Rickettsiella isopodorum* on the basis of both the sequence and the size, thanks to the H.P.M.E. *ftsY-rubA* marker by analyzing the region comprised within the primer pair comprising one of the following three oligonucleotides as the forward primer: Rck_ftsY-834-F1, Rck_ftsY-942-FN or Rck_ftsY-947-FN and one of the two following oligonucleotides as the reverse primer: Rck_rubA_145-RN; Rck_rubA-23-RN. It is not possible to predict the size of the PCR product given by the primer pair ftsY fwd / ftsY rev according leclerque *et al.,* (2011) by *in-silico* analysis.

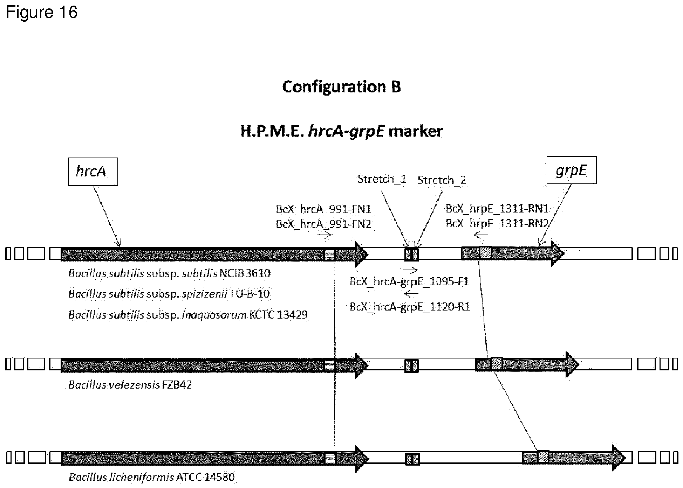

**Figure 16** shows the configuration of the H.P.M.E. *hrcA-grpE* marker, constituted by the partial sequence of the *hrcA* gene and the *grpE* gene and the complete sequence of the gene promoter from *Bacillus subtilis* subsp. *subtilis* NCIB 3610; *Bacillus subtilis* subsp.
   *spizizenii* TU-B-10; *Bacillus subtilis* subsp. *inaquosorum* KCTC 13429; *Bacillus velezensis* FZB42 and *Bacillus licheniformis* ATCC 14580.
   The genes *hrcA* and *grpE* are oriented in the same direction. In the promoter region the the conserved sequences corresponding to Stretch_1 and Stretch_2, corresponding respectively to BcX hrcA-grpE_1095-F1 and BcX_hrcA-grpE_1120-R1 are comprised in the PCR product obtained with the primer pair constituted by BcX_hrcA_991-FN2 or BcX_hrcA_991-FN1, as forward primer, and BcX_hrpE_1311-RN1or BcX_hrpE_1311-RN2, as reverse primer. In both the cases species belonging to the *Bacillus* genus can be differentiated both by sequence polymorphisms (SNPs) and by length polymorphisms (insertion/deletion).

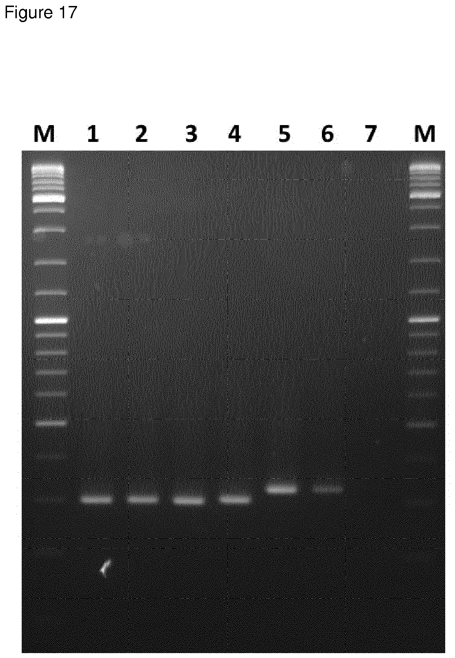

**Figure 17** shows the results of the electrophoresis migration of the PCR products in 2.0% w/v agarose gel obtained for four species belonging to the *Bacillus* genus commonly applied in agriculture, in food and in industrial production processes: *Bacillus subtilis* subsp. subtilis, *Bacillus subtilis* subsp. *spizizenii, Bacillus subtilis* subsp. *inaquosorum, Bacillus velezensis, Bacillus licheniformis* and *Bacillus megaterium.* The PCR product was obtained with the primers BcX_hrcA_991-FN2 and BcX_hrpE_1311-RN2. The sample list is: M) size marker O'GeneRuler DNA Ladder Mix, Ready-to-Use 100 to 10.000 bp (Thermo Scientific); 1) *B. subtilis* DSM 10; 2) *B. subtilis* subsp. *spizizenii* DSM 15029; 3) *B. subtilis* subsp. *inaquosorum* DSM 22148; 4) *B*. *velezensis* DSM 23117; 5) *B*. *licheniformis* DSM 13; 6) *B. megaterium* NRRL B-14308; 7) No Template Control.

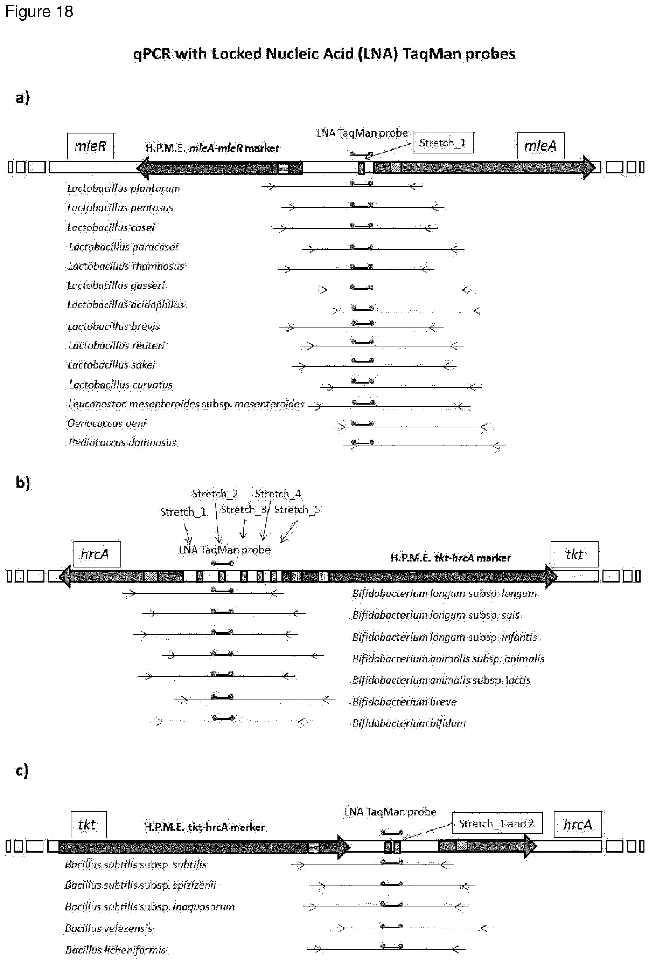

**Figure 18** shows the application scheme of a qPCR protocols based on the LNA TaqMan^{®} probe technology: a) combining short LNA TaqMan probe targeting microorganisms of the *Lactobacillales* order and species-specific primers designed on the H.P.M.E. *mleA-mleR* marker; b) combining short LNA TaqMan probe targeting *Bifidobacterium genus* and species-specific primers designed on the H.P.M.E. *tkt-hrcA* marker; c) a) combining short LNA TaqMan probe targeting microorganisms of the *Bacillus genus* and species-specific primers designed on the H.P.M.E. *hrcA-grpE* marker*.*

### DETAILED DESCRIPTION OF THE INVENTION

The present invention therefore concerns a Highly Polymorphic and Modular Extragenic (HPME) marker, said H.P.M.E. being a microbial genome nucleotide sequence having a length of less than or equal to 2000bp, and said HPME comprising two genetic elements, at least one of which is a predicted Open Reading Frame coding for a protein, a hypothetical protein, or a pseudogene, wherein said two genetic elements are divided by a noncoding nucleotide sequence corresponding to a promoter region, wherein said H.P.M.E. marker allows the identification and quantification of microorganisms, and wherein said two genetic elements are respectively: - the gene mleA coding for the Malolactic enzyme and the gene mleR coding for the mleA transcriptional regulator, belonging to the LysR-type transcriptional regulator family, namely H.P.M.E. mleA-mleR marker, for the identification and quantification of microorganisms within the Lactobacillales order; - the gene tkt coding for the Transketolase enzyme and the gene hrcA coding for the HrcA transcriptional regulator, namely H.P.M.E. tkt-hrcA marker, for the identification and quantification of microorganisms within the Bifidobacterium genus; - the gene grpE coding for the Heat Shock Protein GrpE and the gene hrcA coding for the HrcA transcriptional regulator, namely H.P.M.E. hrcA-grpE marker, for the identification and quantification of microorganisms within the Bacillus genus.

The HPME of the present invention has the advantages of being a very robust new marker for use in nucleic acid based methods suitable for the generation of tools for biomedical research and biotechnological applications.

The present invention relates to the use of nucleotide sequences (HPME target sequences) comprising two genetic elements (*loci*)*,* at least one of which is a predicted Open Reading Frame coding for a protein, a hypothetical protein, or a pseudogene, to differentiate identify and quantify microorganisms,

The two genetic elements in the target sequences are divided by noncoding nucleotide sequence, that correspond to the promoter region of one Open Reading Frame coding for a protein or for an hypothetical protein.

The target sequences are characterized by both conserved and variable nucleotide stretches within specific taxa of microorganisms and viruses and they represent the key information that is exploited to develop new protocols for the differentiation, identification and the quantification of the microorganisms The target sequences are characterized by two levels of polymorphism within the same taxon: sequence polymorphism and length polymorphism. The number of the possible sequence targets and their characteristics guarantees the flexibility required to be applied on different technological platform and with different protocols, as well as two possible levels of specificity to check the robustness of the results.

In one aspect of the disclosure but not part of the invention the genetic elements comprised in the target sequences are divided by a sequence stretch of zero nucleotides, They are therefore united, but this does not hinder to differentiate microorganisms and viruses from other microorganisms and viruses within the same taxon, divided by a longer stretch of nucleotides.

In one aspect of the invention the two genetic elements are oriented in opposite directions on the two complementary strands of the DNA.

In another aspect of the invention the two two genetic elements are oriented in the same direction on the same strand of DNA.

All above considered, the target sequences previously defined guarantee the flexibility required to be applied on different technological platform and with different protocols, as well as two possible level of specificity to check the robustness of the results.

In a preferred aspect, the HPME marker according to the present invention is a bacterial sequence such as eubacteria archea, fungi or a viral sequence. In one aspect, the HPME marker according to the present disclosure but not part of the invention is a bacterial sequence, which belongs to a bacterium of the genus chosen from the group consisting of *Oenococcus, Lactobacillus, Lactococcus, Leuconostoc, Pediococcus, Streptococcus, Fructobacillus, Weisella, Enterococcus, Bifidobacterium, Bacillus, Paenibacillus, Streptomyces, Gluconobacter, Acetobacter, Gluconacetobacter, Komagataeibacter, Saccharomyces, Zygosaccharomyces, Schizosaccharomyces Candida, Penicillium,* and *Aspergillus,*

In a preferred aspect, the HPME marker according to the present invention is a bacterial sequence belonging to a bacterium of the genus chosen from the group consisting of *Lactobacillus, Leuconostoc* and *Pediococcus Streptococcus, Enterococcus, Bifidobacterium* and *Bacillus.*

A further aspect of the disclosure but not part of the invention relates to an HPME marker whose flanking extragenic region is in a non coding region, preferably a conserved tRNA, rRNA or a pseudogene region.

A still further aspect of the invention relates to an HPME marker wherein said secondary flanking region is a gene coding for a gene regulatory protein and said gene coding for a gene regulatory protein is:
- oriented in the same direction of the primary gene;
- oriented in the opposite direction of the primary gene.

A still further aspect of the invention relates to an HPME marker wherein said secondary flanking region is a gene coding for a gene regulatory protein belonging to the lysR-type transcriptional regulator family and to the HrcA family according the PFAM nomenclature. According to one aspect, the described invention provides a H.P.M.E. marker, wherein the said primary protein encoding gene and a secondary flanking extragenic region are respectively:
- the gene *mleA* coding for the Malolactic enzyme and the gene *mleR* coding for the *mleA* transcriptional regulator, belonging to the LysR-type transcriptional regulator family, namely H.P.M.E. *mleA-mleR* marker, for the identification and quantification of microorganisms within the *Lactobacillales* order;
- the gene tktcoding for the Transketolase enzyme and the gene *hrcA* coding for the HrcA transcriptional regulator, namely H.P.M.E. *tkt-hrcA* marker, for the identification and quantification of microorganisms within the *Bifidobacterium* genus;
- the gene *grpE* coding for the Heat Shock Protein GrpE and the gene *hrcA* coding for the HrcA transcriptional regulator, namely H.P.M.E. *hrcA-grpE* marker, for the identification and quantification of microorganisms within the *Bacillus* genus.

The present invention also relates to the use of the HPME marker, for the identification of the genus and/or the species and/or subspecies or any of the Operational Taxonomic Unit (OTU) of a microorganism.

In a further aspect the invention provides a use of the HPME marker for the identification and quantification of a target microorganisms.

According to one aspect, the described invention provides a use of the HPME marker for the identification and quantification of microorganisms carried out with HPME marker specific primers and probes

In a preferred aspect the invention provides a use of the HPME marker, wherein said primers and probes are for molecular biology techniques such as for example: PCR, rtPCR, qPCR, LAMP-PCR, Next Generation Sequencing, molecular applications mediated by hybridization, preferably qPCR, cytofluorimetry, citofluorimetry and cell sorting, Fluorescent In Situ Hybridization (FISH), FISH-FLOW.

In a preferred aspect the invention provides a use of the HPME marker, wherein said identification and quantification of microorganisms is carried out with:
- a target marker consisting of the nonamer sequence TTTGACTAT within the H.P.M.E. *mleA-mleR* marker, for the *Lactobacillales* order;
- the target markers consisting of the sequences ACAAGCCGG, GAGTGCTAAT (SEQ ID NO: 25), AACACGCCAAA (SEQ ID NO: 23), ATTGGAAGGAAAGTA (SEQ ID NO:24), ATTGTATTAGCACTC (SEQ ID NO: 26), within the H.P.M.E. *tkt-hrcA* marker, for the *Bifidobacterium* genus;
- the target markers consisting of the sequence TTTGTTTTTCTTCT, TGTGTTCACCTCCCT within the H.P.M.E. *hrcA-grpE* marker; for the *Bacillus* genus.

A further aspect of the present invention is a target marker consisting of the nonamer sequence TTTGACTAT.

A still further aspect of the present invention is the use of the nonamer sequence TTTGACTAT for the identification and the quantification of the genus and the species of microorganism.

In a preferred aspect the invention provides a use of the nonamer sequence, wherein the microorganism is a bacterium and wherein the microorganism is preferably a bacterium and is chosen from the group consisting of the genera *Oenococcus, Lactobacillus, Lactococcus, Leuconostoc, Pediococcus, Streptococcus, Fructobacillus, Weisella, Enterococcus, Bifidobacterium, Bacillus, ,* In a more preferred aspect the microorganism is a bacterium and is chosen from the group consisting of the genera *Lactobacillus, Leuconostoc* and *Pediococcus Streptococcus, Enterococcus, Bifidobacterium* and *Bacillus.*

The possible applications and uses to differentiate, identify and quantify microorganism and viruses thanks to the information contained in the precise configuration of variable and conserved sequence stretches of the HPME target sequences, are:
i) oligonucleotides to be applied as primers in Polymerase Chain Reaction (PCR) and quantitative PCR (qPCR), both characterized by thermal cycling amplifications;
ii) oligonucleotides or xeno-nucleic acid aptamers such as Locked Nucleic Acid (LNA) oligos to be applied as primers in isothermal amplification (e.g. Loop mediated isothermal amplification);
iii) oligonucleotides or xeno-nucleic acid aptamers such as Locked Nucleic Acid (LNA) oligos to be used as a probe marked with a reporter molecule for colorimetric assays mediated by hybridization (e.g. Horseradish Peroxidase mediated);
iv) oligonucleotides to be used as a probe marked with a reporter molecule for chemiluminescence assays mediated by hybridization;
v) oligonucleotides or xeno-nucleic acid aptamers such as Locked Nucleic Acid (LNA) oligos to be used as a probe marked with a reporter molecule (e.g. Fluorescein, FAM, HEX, NED, TAMRA, R6G, JOE, VIC, Cyanine dyes, AlexaFluor^{(R)} dyes, quantum dots) for fluorescence molecular applications mediated by hybridization such as qPCR, cytofluorimetry, citofluorimetry and cell sorting, Fluorescent In Situ Hybridization (FISH), FISH-FLOW;
vi) polypeptides such as Restriction Enzymes (RE), modified restriction enzymes, specific DNA sequence target antibodies;
vii) any method whereby is possible to detect and report a specific nucleotide sequence or a specific configuration of nucleotide sequences.

In a preferred embodiment, the HPME marker can have a target sequence which is specific for the microbial genera which are to be identified. For example, for the identification of the *Oenococcus* genus and the *Lactobacillus* genus, the mleA-mleR target sequence is used.

**mleA-mleR target sequence for the *Oenococcus* genus and the *Lactobacillus* genus identification** can be chosen from the following:
SEQ ID NO: 1 Oenococcus_alcoholitolerans_LMG_27599_mleA-mleR 
TGATCGATCTCGTCGTGAGCGGCCAAAATCTGCGTAAGGATCCTTTCTGTGTACTT
TTCGAGGATCTTACCGGCTTTCGTCAATTGAACGCGGCCATGCGAATGATTCCTGG
 
TAACTAATTCTTGTTTAAATTCTTTTTCCAATCGCTGGATGGCATAAGAAATAGTTGG
TTGGCTGACATTGAAAAAAGCTGCCGTTTGTGAAAACGACTCCACTTTGGCCAGGC
GCTGAAAATACTCTAAATCAGTGAATTTCATATAAATTTATTTTATCTCTTTTGCAGC
TTTTGACTATATCAGCTGCGATAAAATATCCTGAATCTGTTTGTTTATTAGGAGAGA
GAAATGACAGATCCAGTAGATATTTTAAACGATCCCTTTAAAAACAAAGGAACCGCT
TTTACTCAAGAAGAACGCAAAGCACTGGGCTTGCAGGGACTTTTGCCCTCATGTGT
GCAAACTATTGATCAGCAAGCTGCACAAGCCTATGCTCAATTTCAAAGCAAAGCTG
ATAATCTAGAAAAAAGACTTTTCTTGATGGAACTTTTCAACACCAACCACGTGCTGT
TCTATAAGCTTTTTTCACAACACGTCGTTGAATTCATGCCGATCGTCTATGATCCGA
CTATCGCTGATACGATTGAAAATTATTCTGAACTTTTCGTTGAAACACAGGGAGCTG
CTTTTTTGGATATCCAGCATCCAGAAAACATCGAAGCCAGTTTGAAAAATGCCGCT
GACGGTCGTGACATAAAATTGATCGTCGTCACTGATGCTGAGGGCATTTTAGGTAT
CGGCGACTGGGGTGTTCAAGGGGTCGATATCGCCGTTGGAAAATTGATGGTCTAC
ACCGTGGCTGCAGGACTTGATCCAAAGAGTGTCTTGCCGGTCGTGATCGATGCCG
GAACCAATAATCAAAAACTTTTAAACGATCCTTTGTATTTAGGAAACCGTTTTAACC
GTGTTCGCGGTCAAGCTTATGATGATTTTATCGATACTTTTGTTGAAAAATCGGAAA
ATCTTTTTCCAGATATGTATTTGCATTGGGAAGACTTTGGGCGTTTGAATGCCGCAA
ATATTTGAATAGA
SEQ ID NO: 2 Lactobacillus_brevis _LMG 11989_mleA-mleR 
CCATACAGAGCCGCCCTCAACAGTTTGCAGTCCTTCCAGCAGATTCTCAGCCTGCA
ATCGTGCGGCAATTCGTGGAAAATAATAATTTTCGATAATTGGTGGCAACCCCAGC
GTGGTTTGGCGAGCAGCTAAATGGGCTAGTTCTTGGTGGGCTAGCGTTAATTCACT
GAGGACGACTTGGGCGTGAATGGCTAATTGCTCACCGCTCGGGGTAATTTGAATA
TCATTGTGTACCCGATCCCGGATGACTAATTTGGTTTCAAATTCGGTCTCTAATCGT
TTGATAGCCGTGGTGATCGTGGGCTGAGTCACACCGAArAAGGCTGCTACCTTAGA
AAAATTTTTTTGTAGAATAAGCTGCTGGAAATAATCCAAATCTTTTGTATTCATATTT
TCTCCTGTCAAATCAAGGCTGATTACTACATTATAAAATAATTTTATAACAATCGCTA
CGTTTGACTATAAAATTTTCAGGGCGTTATAGTGCAATATGTAAATAAGAGCTATCC
AAATTGGAGGAAAATCATGATGACGCAAACAGCAGATACGATTTTAAATAACCCCTT
CTTAAATAAAGGAACCGCGTTCACACAAGCAGAACGGCGGGAACTTGGCTTGGTT
GGGACATTGCCAGCCCAAGTTCAAACGATTGATGAACAAGCCACTCAGGCCTATG
 
CCCAATACCAAAGCAAGCCAAGTGACCTGGAAAAGCGGATTTTCTTAATGAATCTC
TTTAACGAAAACCGGACATTATTCTTCCATTTAATGGATGAACACGTGGTTGAATT
SEQ ID NO: 3, Lactobacillus_brevis_LMG_25561_mleA-mleR 
CCATACAGAGCCGCCCTCAACAGTTTGCAGTCCTTCCAGCAGATTCTCAGCCTGCA
ATCGTGCGGCAATTCGTGGAAAATAATAATTTTCGATAATTGGTGGCAACCCCAGC
GTGGTTTGGCGAGCAGCTAAATGGGCTAGTTCTTGGTGGGCTAGCGTTAATTCACT
GAGGACGACTTGGGCGTGAATGGCTAATTGCTCACCGCTCGGGGTAATTTGAATA
TCATTGTGTACCCGATCCCGGATGACTAATTTGGTTTCAAATTCGGTCTCTAATCGT
TTGATAGCCGTGGTGATCGTGGGCTGAGTCACACCGAAGAAGGCTGCTACCTTAG
AAAAATTTTTTTGTAGAATAAGCTGCTGGAAATAATCCAAATCTTTTGTATTCATATT
TTCTCCTGTCAAATCAAGGCTGATTACTACATTATAAAATAATTTTATAACAATCGCT
ACGTTTGACTATAAAATTTTCAGGGCGTTATAGTGCAATATGTAAATAAGAGCTATC
CAAATTGGAGGAAAATCATGATGACGCAAACAGCAGATACGATTTTAAATAACCCC
TTCTTAAATAAAGGAACCGCGTTCACACAAGCAGAACGGCGGGAACTTGGCTTGG
TTGGGACATTGCCAGCCCAAGTTCAAACGATTGATGAACAAGCCACTCAGGCCTAT
GCCCAATACCAAAGCAAGCCAAGTGACCTGGAAAAGCGGATTTTCTTAATGAATCT
CTTTAACGAAAACCGGACATTATTCTTCCATTTAATGGATGAACACGTGGTTGAATT
SEQ ID NO: 4 Lactobacillus_brevis_LMG_18022_mleA-mleR 
CCATACAGAGCCGCCCTCGACAGTTTGCAGTCCTTCCAGCAGATTCTCAGCCTGC
AATCGTGCGGCAATTCGTGGAAAATAATAATTTTCGATAATTGGTGGCAACCCCAG
CGTGGTTTGGCGAGCAGCTAAATGTGCTAGTTCTTGGTGGGCTAGCGTTAATTCAC
TGAGGACGACTTGGGCGTGAATGGCTAGTTGCTCACCGCTCGGGGTAATTTGAAT
ATCATTGTGTACCCGATCCCGGATGACTAATTTGGTTTCAAATTCGGTCTCTAATCG
TTTGATAGCCGTGGTGATCGTGGGCTGAGTCACACCGAAGAAGGTTGCTACCTTA
GAAAAATTTTTTTGTAGAATAAGCTGCTGGAAATAATCCAAATATTTTGTATTCATAT
TTTCTCCTGTCAAATCAAGGCTGATTGCTACATTATAAAATAATTTTATAACAATCGC
TACATTTGACTATAAAATTTCCAGGGCGTTATAGTGCAATATGTAAATAAGAGCTAT
CCAAATTGGAGGAAAATCATGATGACGCAAACAGCAGATACGATTTTAAATAACCC
CTTCTTAAATAAAGGAACCGCGTTCACACAAGCAGAACGGCGGGAACTTGGCTTG
GTTGGGACATTGCCAGCCCAAGTTCAAACGATTGATGAACAAGCCACTCAGGCCT
ATGCCCAATACCAAAGCAAGCCAAGTGACCTGGAAAAGCGGATTTTCTTAATGAAT
 
CTCTTTAACGAAAACCGGACATTATTCTTCCATTTAATGGATGAACACGTGGTTGAA
TT
SEQ ID NO: 5 Lactobacillus_brevis_JCM_17312_mleA-mleR 
CCATACAGAGCCGCCCTCAACAGTTTGCAGTCCTTCCAGCAGATTCTCAGCCTGCA
ATCGTGCGGCAATTCGTGGAAAATAATAATTTTCGATAATTGGTGGCAACCCCAGC
GTGGTTTGGCGAGCAGCTAAATGGGCTAGTTCTTGGTGGGCTAGCGTTAATTCACT
GAGGACGACTTGGGCGTGAATGGCTAATTGCTCACCGCTCGGGGTAATTTGAATA
TCATTGTGTACCCGATCCCGGATGACTAATTTGGTTTCAAATTCGGTCTCTAATCGT
TTGATAGCCGTGGTGATCGTGGGCTGAGTCACACCGAAGAAGGCTGCTACCTTAG
AAAAATTTTTTTGTAGAATAAGCTGCTGGAAATAATCCAAATCTTTTGTATTCATATT
TTCTCCTGTCAAATCAAGGCTGATTACTACATTATAAAATAATTTTATAACAATCGCT
ACGTTTGACTATAAAATTTTCAGGGCGTTATAGTGCAATATGTAAATAAGAGCTATC
CAAATTGGAGGAAAATCATGATGACGCAAACAGCAGATACGATTTTAAATAACCCC
TTCTTAAATAAAGGAACCGCGTTCACACAAGCAGAACGGCGGGAACTTGGCTTGG
TTGGGACATTGCCAGCCCAAGTTCAAACGATTGATGAACAAGCCACTCAGGCCTAT
GCCCAATACCAAAGCAAGCCAAGTGACCTGGAGAAGCGGATTTTCTTAATGAATCT
CTTTAACGAAAACCGGACATTATTCTTCCATTTAATGGATGAACACGTGGTTGAATT
SEQ ID NO: 6 Lactobacillus_brevis_LMG_11998_mleA-mleR 
CCATACAGAGCCACCCTCAACCGTTTGCAGTCCTTCCAGCAGATTCTCAGCCTGCA
ATCGTGCGGCAATTCGTGGAAAATAATAATTTTCGATAATTGGTGGCAACCCCAGC
GTGGTTTGGCGAGCAGCTAAATGTGCTAGTTCTTGGTGGGCTAGCGTTAATTCACT
GAGGACGACTTGGGCGTGAATGGCTAGTTGCTCACCGCTCGGGGTAATTTGAATA
TCATTGTGTACCCGATCCCGGATGACTAATTTGGTTTCAAATTCGGTCTCTAATCGT
TTGATAGCCGTGGTGATCGTGGGCTGAGTCACACCGAAGAAGGTTGCTACCTTAG
AAAAATTTTTTTGTAGAATAAGCTGCTGGAAATAATCCAAATCTTTTGTATTCATATT
TTCTCCTGTCAAATCAAGGCTGATTGCTACATTATAAAATAATTTTATAACAATCGCT
ACATTTGACTATAAAATTTCCAGGGCGTTATAGTGCAATATGTAAATAAGAGCTATC
CAAATTGGAGGAAAATCATGATGACGCAAACAGCAGATACGATTTTAAATAACCCC
TTCTTAAATAAAGGAACCGCGTTCACACAAGCAGAACGGCGGGAACTTGGCTTGG
TTGGGACATTGCCAGCCCAAGTTCAAACGATTGATGAACAAGCCACTCAGGCCTAT
GCCCAATACCAAAGCAAGCCAAGTGACCTGGAAAAGCGGATTTTCTTAATGAATCT
 CTTTAACGAAAACCGGACATTATTCTTCCATTTAATGGATGAACACGTGGTTGAATT
SEQ ID NO: 7 Lactobacillus_brevis_LMG_11435_mleA-mleR 
CCATACAGAGCCGCCCTCAACAGTTTGCAGTCCTTCCAGCAGATTCTCAGCCTGCA
ATCGTGCGGCAATTCGTGGAAAATAATAATTTTCGATAATTGGTGGCAACCCCAGC
GTGGTTTGGCGAGCAGCTAAATGGGCTAGTTCTTGGTGGGCTAGCGTTAATTCACT
GAGGACGACTTGGGCGTGAATGGCTAATTGCTCACCGCTCGGGGTAATTTGAATA
TCATTGTGTACCCGATCTCGGATGACTAATTTGGTTTCAAATTCGGTCTCTAATCGT
TTGATAGCCGTGGTGATCGTGGGCTGAGTCACACCGAAGAAGGCTGCTACCTTAG
AAAAATTTTTTTGTAGAATAAGCTGCTGGAAATAATCCAAATCTTTTGTATTCATATT
TTCTCCTGTCAAATCAAGGCTGATTACTACATTATAAAATAATTTTATAACAATCGCT
ACGTTTGACTATAAAATTTTCAGGGCGTTATAGTGCAATATGTAAATAAGAGCTATC
CAAATTGGAGGAAAATCATGATGACGCAAACAGCAGATACGATTTTAAATAACCCC
TTCTTAAATAAAGGAACCGCGTTCACACAAGCAGAACGGCGGGAACTTGGCTTGG
TTGGGACATTGCCAGCCCAAGTTCAAACGATTGATGAACAAGCCACTCAGGCCTAT
GCCCAATACCAAAGCAAGCCAAGTGACCTGGAAAAGCGGATTTTCTTAATGAATCT
CTTTAACGAAAACCGGACATTATTCTTCCATTTAATGGATGAACACGTGGTTGAATT
SEQ ID NO: 8 Lactobacillus_brevis_LMG_11434_mleA-mleR 
CCATACAGAGCCACCCTCAACCGTTTGCAGTCCTTCCAGCAGATTCTCAGCCTGCA
ATCGTGCGGCAATTCGTGGAAAATAATAATTTTCGATAATTGGTGGCAACCCCAGC
GTGGTTTGGCGAGCAGCTAAATGTGCTAGTTCTTGGTGGGCTAGCGTTAATTCACT
GAGGACGACTTGGGCGTGAATGGCTAGTTGCTCACCGCTCGGGGTAATTTGAATA
TCATTGTGTACCCGATCCCGGATGACTAATTTGGTTTCAAATTCGGTCTCTAATCGT
TTGATAGCCGTGGTGATCGTGGGCTGAGTCACACCGAAGAAGGTTGCTACCTTAG
AAAAATTTTTTTGTAGAATAAGCTGCTGGAAATAATCCAAATCTTTTGTATTCATATT
TTCTCCTGTCAAATCAAGGCTGATTGCTACATTATAAAATAATTTTATAACAATCGCT
ACATTTGACTATAAAATTTCCAGGGCGTTATAGTGCAATATGTAAATAAGAGCTATC
CAAATTGGAGGAAAATCATGATGACGCAAACAGCAGATACGATTTTAAATAACCCC
TTCTTAAATAAAGGAACCGCGTTCACACAAGCAGAACGGCGGGAACTTGGCTTGG
TTGGGACATTGCCAGCCCAAGTTCAAACGATTGATGAACAAGCCACTCAGGCCTAT
GCCCAATACCAAAGCAAGCCAAGTGACCTGGAGAAGCGGATTTTCTTAATGAATCT
CTTTAACGAAAACCGGACATTATTCTTCCATTTAATGGATGAACACGTGGTTGAATT
SEQ ID NO: 9 Lactobacillus_brevis_LMG_11401_mleA-mleR 
CCATACAGAGCCGCCCTCAACAGTTTGCAGTCCTTCCAGCAGATTCTCAGCCTGCA
ATCGTGCGGCAATTCGTGGAAAATAATAGTTTTCGATAATTGGTGGCAACCCCAGC
GTGGTTTGGCGAGCAGCTAAATGGGCTAGTTCTTGGTGGGCTAGCGTTAATTCACT
AAGGACGACTTGGGCGTGAATGGCTAATTGCTCACCGCTCGGGGTAATTTGAATAT
CATTGTGTACCCGATCCCGGATGACTAATTTGGTTTCAAATTCGGTCTCTAATCGTT
TGATAGCCGTGGTGATCGTGGGCTGAGTCACACCGAAGAAGGCTGCTACCTTAGA
AAAATTTTTTTGTAGAATAAGCTGCTGGAAATAATCCAAATCTTTTGTATTCATATTT
TCTCCTGTCAAATCAAGGCTAATTGCTACATTATAAAATAATTTTATAACAATCGCTA
CATTTGACTATAAAATTTCCAGGGCGTTATAGTGCAATATGTAAATAAGAGCTATCC
AAATTGGAGGAAAATCATGATGACGCAAACAGCAGATACGATTTTAAATAACCCCTT
CTTAAATAAAGGAACCGCGTTCACGCAAGCAGAACGGCGGGAACTTGGCTTGGTT
GGGACATTGCCAGCCCAAGTTCAAACGATTGATGAACAAGCCACTCAGGCCTATG
CTCAATACCAAAGCAAGCCAAGTGACCTGGAGAAGCGGATTTTCTTAATGAATCTC
TTTAACGAAAACCGGACATTATTCTTCCATTTAATGGATGAACATGTGGTTGAATT
SEQ ID NO: 10 Lactobacilllus_brevis_LMG_6906_mleA-mleR 
CCATACAGAGCCGCCCTCAACAGTTTGCAGTCCTTCCAGCAGATTCTCAGCCTGCA
ATCGTGCGGCAATTCGTGGAAAATAATAATTTTCGATAATTGGTGGCAACCCCAGC
GTTGTTTGGCGAGCAGCTAAATGGGCTAGTTCTTGGTGGGCTAGCGTTAATTCACT
GAGGACGACTTGGGCGTGAATGGCTAATTGCTCACCGCTCGGGGTAATTTGAATA
TCATTGTGTACCCGATCCCGGATGACTAATTTGGTTTCAAATTCGGTCTCTAATCGT
TTGATAGCCGTGGTGATCGTGGGCTGAGTCACACCGAAGAAGGCTGCTACCTTAG
AAAAATTTTTTTGTAGAATAAGCTGCTGGAAATAATCCAAATCTTTTGTATTCATATT
TTCTCCTGTCAAATCAAGGCTGATTGCTACATTATAAAATAATTTTATAACAATCGCT
ACATTTGACTATAAAATTTCCAGGGCGTTATAGTGCAATATGTAAATAAGAGCTATC
CAAATTGGAGGAAAATCATGATGACGCAAACAGCAGATACGATTTTAAATAACCCC
TTCTTAAATAAAGGAACCGCGTTCACACAAGCAGAACGGCGGGAACTTGGCTTGG
TTGGGACATTGCCAGCCCAAGTTCAAACGATTGATGAACAAGCCACTCAGGCCTAT
GCCCAATACCAAAGCAAGCCAAGTGACCTGGAGAAGCGGATTTTCTTAATGAACCT
CTTTAACGAAAACCGGACATTATTCTTCCATTTAATGGATGAACATGTGGTTGAATT
SEQ ID NO: 11 Lactobacillus_brevis_LMG_12023_mleA-mleR

CATACAGAGCCGCCCTCAACAGTTTGCAGTCCTTCCAGCAGATTCTCAGCCTGCAA
TCGTGCGGCAATTCGTGGAAAATAATAATTTTCGATAATTGGTGGCAACCCCAGCG
TGGTTTGGCGAGCAGCTAAATGGGCTAGTTCTTGGTGGGCTAGCGTTAATTCACTG
AGGACGACTTGGGCGTGAATGGCTAATTGCTCACCGCTCGGGGTAATTTGAATAT
CATTGTGTACCCGATCTCGGATGACTAATTTGGTTTCAAATTCGGTCTCTAATCGTT
TGATAGCCGTGGTGATCGTGGGCTGAGTCACACCGAAGAAGGCTGCTACCTTAGA
AAAATTTTTTTGTAGAATAAGCTGCTGGAAATAATCCAAATCTTTTGTATTCATATTT
TCTCCTGTCAAATCAAGGCTGATTACTACATTATAAAATAATTTTATAACAATCGCTA
CGTTTGACTATAAAATTTTCAGGGCGTTATAGTGCAATATGTAAATAAGAGCTATCC
AAATTGGAGGAAAATCATGATGACGCAAACAGCAGATACGATTTTAAATAACCCCTT
CTTAAATAAAGGAACCGCGTTCACACAAGCAGAACGGCGGGAACTTGGCTTGGTT
GGGACATTGCCAGCCCAAGTTCAAACGATTGATGAACAAGCCACTCAGGCCTATG
CCCAATACCAAAGCAAGCCAAGTGACCTGGAAAAGCGGATTTTCTTAATGAATCTC
TTTAACGAAAACCGGACATTATTCTTCCATTTAATGGATGAACACGTGGTTGAATT

For the identification of the *Bacillus* genus, the nucB-sigK HPME marker is used.

### nucB-sigK and ycgS-ycgT-and H.P.M.E. markerfor the Bacillus genus

Bacillus subtilis subsp. subtilis str. NCIB 3610 chromosome, whole genome shotgun sequence, NCBI Accession number gi|223666305:2651588-2652664
Bacillus subtilis DNA, 283 Kb region containing skin element gi|2627063:66148-67224 Bacillus subtilis subsp. subtilis str. OH 131.1, complete genome, NCBI Accession number gb|CP007409.1|:2505351-2506295
Bacillus subtilis strain SG6, complete genome, NCBI Accession number gb|CP009796.1|:2530873-2531818
> Bacillus subtilis strain T30, complete genome, NCBI Accession number gb|CP011051.1|:453835-454784
Bacillus tequilensis KCTC 13622 contig34, whole genome shotgun sequence, NCBI Accession number gb|AYTO01000034.1|:238817-239821
Bacillus tequilensis strain FJAT-14262a Scaffold3, whole genome shotgun sequence, NCBI Accession number gb|LGRW01000003.1|:526637-527582
Bacillus vallismortis strain B4144_201601 NODE_52, whole genome shotgun sequence, NCBI Accession number gb|LQYR01000021.1|:335392-335821
Bacillus vallismortis DV1-F-3 scf7180000000938_1, whole genome shotgun sequence, NCBI Accession number gb|AFSH01000080.1|:61061-62135
Bacillus subtilis subsp. spizizenii str. W23, complete genome, NCBI Accession number gb|CP002183.1|:2508422-2509371
Bacillus subtilis subsp. spizizenii strain NRS 231, complete genome, NCBI Accession number gb|CP010434.1|1:2508413-2509362
Bacillus subtilis subsp. spizizenii TU-B-10, complete genome, NCBI Accession number gb|CP002905.1|:2633479-2634424
Bacillus subtilis subsp. inaquosorum KCTC 13429 14.BSI.1_2, whole genome shotgun sequence, NCBI Accession number gb|AMXN01000002.1|:219554-220502
Bacillus atrophaeus 1942, complete genome, NCBI Accession number gb|CP002207.1|:2171410-2171836
Bacillus atrophaeus strain NRS 1221A, complete genome, NCBI Accession number gb|CP010778.11:2096769-2097195
Bacillus atrophaeus UCMB-5137 genome, NCBI Accession number gblCP011802.11:2146753-2147182
Bacillus siamensis strain SRCM100169 contig00001, whole genome shotgun sequence, NCBI Accession number gb|LYUE01000001.1|:378573-379007
Bacillus siamensis KCTC 13613 contig41, whole genome shotgun sequence, NCBI Accession number gb|AJVF01000041.1|:425488-425922
Bacillus cereus genome assembly Bacillus JRS1, contig contig000619, whole genome shotgun sequence, NCBI Accession number gi|924105349:1135-2083
Bacillus mojavensis RRC 101 contig_72, whole genome shotgun sequence, NCBI Accession number gb|ASJT01000072.1|:62201-63276
Bacillus siamensis strain SRCM100169 contig00001, whole genome shotgun sequence, NCBI Accession number gb|LYUE01000001.1|:377399-378922
Bacillus amyloliquefaciens strain JJC33M contig_2, whole genome shotgun sequence, NCBI Accession number gb|JTJG01000002.1|1:175026-176555
Bacillus amyloliquefaciens subsp. plantarum str. FZB42, complete genome, NCBI Accession number gi|154684518:116761-117992
Bacillus licheniformis ATCC 14580, complete genome, NCBI Accession number gi|1163119169:2425104-2426659
Bacillus subtilis subsp. subtilis str. 168 chromosome, complete genome, NCBI Accession number gi|255767013:351842-353868
The tkt-hrcA target sequence is used for the identification of the *Bifidobacteria* genus.

### tkt - hrcA H.P.M.E. marker for the Bifidobacterium genus

Bifidobacterium animalis subsp. animalis ATCC 25527, NCBI Accession number gb|CP002567.1|:975534-978937
Bifidobacterium animalis subsp. lactis DSM 10140, NCBI Accession number gb|CP001606.1|:964067-967480
Bifidobacterium breve DSM 20213 = JCM 1192, NCBI Accession number gi|781874447:1141916-1145382 DNA
Bifidobacterium longum subsp. infantis ATCC 15697, NCBI Accession number gb|CP001095.1|:c1287218-1283617
Bifidobacterium longum subsp. longum JCM 1217 DN, NCBI Accession number gi|320455049:1275719-1279320 A
Bifidobacterium longum subsp. suis strain LMG 21814 Contig32, NCBI Accession number gb|JGZA01000032.1|:c3691-90
The mleA-mleR/mleS-yjcA/mae-citR target sequences are used for the identification of respectively the *Oenococcus* genus, the *Lactobacillus* genus and the *Streptococcus* genus, *Enterococcus* genus, the *Pediococcus* genus, the *Lactococcus* genus, the *Fructobacillus* genus, the *Leuconostoc* genus, and the *Weissella* genus.

### mleA-mleR and mleS-yjcA and mae-citR H.P.M.E. markers for Lactic Acid Bacteria

Oenococcus oeni mleR, mleA, mleP genes for malolactic regulator, malolactic enzyme, malate permease, complete cds, strain: ATCC 39401, NCBI Accession number gi|75755624:147-2748
Oenococcus oeni PSU-1, complete genome, NCBI Accession number gi|116490126:1481086-1483687
Oenococcus oeni strain L7 malolactic enzyme gene,partial sequence, NCBI Accession number gb|HM101476.1|:8-950
Oenococcus kitaharae DSM 17330 chromosome, whole genome shotgun sequence, NCBI Accession number gi|372325750:114550-117167
Oenococcus alcoholitolerans strain UFRJ-M7.2.18 contig00329, whole genome shotgun sequence, NCBI Accession number gb|AXCV01000329.1|:57-486
Oenococcus alcoholitolerans strain UFRJ-M7.2.18 contig00674, whole genome shotgun sequence, NCBI Accession number gb|AXCV01000674.1|:4-514
Lactobacillus plantarum strain HFC8, complete genome, NCBI Accession number gb|CP012650.1|:2763364-2764968
Lactobacillus acidophilus strain ATCC 4356 scaffold12, whole genome shotgun sequence, NCBI Accession number gb|JRUT01000012.1|:40982-42345
Lactobacillus brevis ATCC 367, complete genome, NCBI Accession number gi|116332681:2167769-2170426
Lactobacillus sakei subsp. sakei DSM 20017 = JCM 1157 DNA, contig: JCM1157.contig00016, whole genome shotgun sequence, NCBI Accession number gi|602598706:22115-23744
Lactobacillus sakei subsp. carnosus DSM 15831 NODE_99, whole genome shotgun sequence, NCBI Accession number gb|AZFG01000065.11:3788-5579
Lactobacillus curvatus JCM 1096 = DSM 20019 Scaffold76, whole genome shotgun sequence, NCBI Accession number gb|AZDL01000076.11:2749-4554
Lactobacillus reuteri ATCC 53608, WGS project CACS02000000 data, strain ATCC 53608, contig00039, whole genome shotgun sequence, NCBI Accession number gi|332795655:20606-21850
Streptococcus salivarius strain 140_SSAL 1318_7248_129091 _927_,567+, whole genome shotgun sequence, NCBI Accession number gb|JVSQ01000113.1|:2755-4031 Streptococcus infantarius subsp. infantarius ATCC BAA-102 S_infantarius-2.0.1_Cont294, whole genome shotgun sequence, NCBI Accession number gb|ABJK02000006.1|:3337-9360
Lactobacillus casei ATCC 334, complete genome, NCBI Accession number gb|CP000423.1|:736033-739024
Lactobacillus casei DSM 20011 = JCM 1134 strain DSM 20011 Scaffold4, whole genome shotgun sequence, NCBI Accession number gb|AZC001000004.1|:131693-134684 Lactobacillus paracasei subsp. paracasei ATCC 25302 contig00157, whole genome shotgun sequence, NCBI Accession number gb|ACGY01000115.1|:13771-16764
Lactobacillus paracasei subsp. tolerans DSM 20258 Scaffold70, whole genome shotgun sequence, NCBI Accession number gb|AYYJ01000070.1|:3517-6357
Lactobacillus rhamnosus DSM 20021 = JCM 1136 = NBRC 3425 strain DSM 20021 Scaffold19, whole genome shotgun sequence, NCBI Accession number gb|AZCQ01000019.1|:42815-45783
Enterococcus faecium PC4.1 contig00071, whole genome shotgun sequence, NCBI Accession number gb|ADMM01000043.1|:33742-35015
Pediococcus damnosus LMG 28219 R50501_77, whole genome shotgun sequence, NCBI Accession number gb|JANK01000077.1|:2123-3916
Streptococcus salivarius strain 918_SSAL 573_13617_118049, whole genome shotgun sequence, NCBI Accession number gb|JUNV01000175.11:10189-11803
Streptococcus infantarius subsp. infantarius CJ18, complete genome gb|CP003295.1|:1775490-1777111
Lactococcus lactis subsp. lactis II1403, complete genome, NCBI Accession number gb|AE005176.1|:919808-923388
Lactococcus lactis subsp. cremoris SK11, complete genome, NCBI Accession number gb|CP000425.1|:916691-920278
Lactococcus lactis subsp. cremoris NZ9000, complete genome, NCBI Accession number gb|CP002094.1|:1616128-1619715
Lactococcus lactis subsp. cremoris UC509.9, complete genome, NCBI Accession number gb|CP003157.1|:926472-930058
Lactococcus lactis subsp. cremoris A76, complete genome, NCBI Accession number gb|CP003132.1|:1521201-1524788
Lactococcus lactis subsp. cremoris KW2, complete genome, NCBI Accession number gb|CP004884.1|:891400-894986
Fructobacillus ficulneus DNA, contig: contig024, strain: JCM 12225, whole genome shotgun sequence, NCBI Accession number gi|850933810:24923-26399
Fructobacillus tropaeoli DNA, contig: contig019, strain: F214-1, whole genome shotgun sequence, NCBI Accession number gi|850934400:64455-65931
Fructobacillus fructosus KCTC 3544 strain DSM 20349 Scaffold5, whole genome shotgun sequence, NCBI Accession number gb|JQBH01000005.1|:29797-31268
Pediococcus damnosus strain TMW 2.1534 plasmid pL21534-4, complete sequence, NCBI Accession number gb|CP012287.11:3965-6514
Leuconostoc mesenteroides DNA, contig: LMDMO_40, strain: 213M0, whole genome shotgun sequence, NCBI Accession number gi|953966517:15883-17346
Leuconostoc mesenteroides subsp. mesenteroides ATCC 8293, complete genome, NCBI Accession number gb|CP000414.11:1004431-1005894
Weissella koreensis KACC 15510, complete genome, NCBI Accession number gb|CP002899.1|:1390746-1392204
Enterococcus faecium strain ATCC 700221, complete genome, NCBI Accession number gb|CP014449.1|:2071582-2073044
Oenococcus oeni PSU-1, complete genome, NCBI Accession number gi|116490126:402339-404605
Lactobacillus casei ATCC 334, complete genome, NCBI Accession number gb|CP000423.1|:2847899-2852959
Lactobacillus casei DSM 20011 = JCM 1134 strain DSM 20011 Scaffold40, whole genome shotgun sequence, NCBI Accession number gb|AZCO01000040.1|:6211-11271 Lactobacillus paracasei subsp. paracasei ATCC 25302 contig00006, whole genome shotgun sequence, NCBI Accession number gb|ACGY01000003.11:1-3751 Lactobacillus rhamnosus DSM 20021 = JCM 1136 = NBRC 3425 strain DSM 20021 Scaffold17, whole genome shotgun sequence, NCBI Accession number gb|AZCQ01000017.1|:1057-5965

### H.P.M.E. hrcA-grpE marker for the Bacillus genus

*Bacillus subtilis* strain NCIB 3610, NCBI Accession number NZ_CP020102.1:c2629658-2627992
*Bacillus subtilis* subsp. *spizizenii* TU-B-10, NCBI Accession number CP002905.1:2608986-2610652
*Bacillus subtilis* subsp. *inaquosorum* KCTC 13429, NCBI Accession number AMXN01000002.1:195028-196694
*Bacillus amyloliquefaciens* subsp. *plantarum* str. FZB42, NCBI Accession number CP000560.1:2497484-2499150
*Bacillus licheniformis* ATCC 14580, NCBI Accession number NC_006270.3:2636182-2637876
Bacillus megaterium NBRC 15308 = ATCC 14581 strain NBRC 15308, NCBI Accession number NZ_BCVB01000008.1:62018-63712
*Bacillus mojavensis* RO-H-1 = KCTC 3706 contig30, NCBI Accession number gi|565623731|gb|AYTL01000030.1|:510964-512631
*Bacillus vallismortis* DV1-F-3 scf7180000000938_1, NCBI Accession number gi|373958755|gb|AFSH01000080.1|:37469-39135
*Bacillus cereus* ATCC 14579 chromosome, NCBI Accession number NC_004722.1:4255895-4257592

### H.P.M.E. tkt - hrcA marker for the Bifidobacterium genus

*Bifidobacterium bifidum* ATCC 29521 = JCM 1255 = DSM 20456 strain ATCC 29521 B_bifidumBIFBIF-1.0_Cont48.4, NCBI Accession number AWSW01000046.1:33043-36754

### H.P.M.E. rsmD-ftsY marker for the Rickettsiella genus

*Rickettsiella grylli* gcontig_637, whole genome shotgun sequence, NCBI Accession number AAQJ02000001.1:605954-607684
*Rickettsiella grylli* strain TrM1 contig_822, NCBI Accession number MCRF01000441.1:2072-3802
Candidatus *Rickettsiella isopodorum* strain RCFS May 2013 383, NCBI Accession number LUKY01000027.1:36370-38097

### H.P.M.E. ftsY-rubA marker for the Rickettsiella genus

*Rickettsiella grylli* gcontig_637, NCBI Accession number AAQJ02000001.1:606695-607975
*Rickettsiella grylli* strain TrM1 contig_822, NCBI Accession number MCRF01000441.1:1872-3094
Candidatus *Rickettsiella isopodorum* strain RCFS May 2013 383, NCBI Accession number LUKY01000027.1:37144-38097
According to a one aspect of the invention, the target sequence comprising the genes coding for Malolactic Enzyme (known as *mleA* or *mleS*) and the Malolactic Enzyme Regulator, a lysR-type transcriptional regulator (*mleR*)*,* shortened to *"mleA-mleR* target", oriented on opposite direction on two DNA strands, is applied to detect, to differentiate and to identify all the bacterial species within a *genus* thanks to the sequence polymorphism.

According to yet another aspect of the invention, a nucleotide stretch comprised in the target sequence including the *mleA* (*mleS*) and *mleR* genes and conserved in at least three species of the *Oenococcus* genus, i.e. *Oenococcus oeni, Oenococcus kitaharae* and *Oenococcus alcoholitolerans,* is used to design one genus-specific oligonucleotides. More in detail two DNA sequences conserved in the genus *Oenococcus* are used to design a primer pair with degenerated nucleotides according IUPAC nomenclature for the amplification of about 1 100 bp PCR product for further Sanger sequencing using the same primers singly.

According to yet another aspect of the invention, two nucleotide stretches comprised in the *mleA-mleR* target and conserved in the three species of the *Oenococcus* genus, i.e. *Oenococcus oeni, Oenococcus kitaharae* and *Oenococcus alcoholitolerans,* is used to design genus-specific oligonucleotide pairs to be used as primers in PCR-based techniques for ecological/population studies both culture-dependent and culture-independent. In three different PCR-based protocols, the length polymorphism of the *mleA-mleR* target is used to differentiate and to identify all the three species belonging to the *Oenococcus* genus. In the fourth PCR-based protocols, the sequence polymorphism of the *mleA-mleR* target is mainly used to differentiate and to identify all the three species belonging to the *Oenococcus* genus.

More in detail, in the first protocol based on length polymorphism of the *mleA-mleR* target within the *Oenococcus* genus, the PCR products is obtained by amplifying with a primer pair, possibly with degenerate nucleotides according IUPAC nomenclature, and one of which is fluorescent marked, i.e. FAM marked forward primer. Genomic DNA purified from both pure cultures and from a mixture of strains can be used as template in the PCR reaction. Fluorescent marked PCR products are separated and detected by means of capillary electrophoresis thus allowing the identification of each pure culture and of the components of the mixture of different species.

In the second protocol based on length polymorphism of the *mleA-mleR* target within the *Oenococcus* genus, the PCR products is obtained by amplifying with a primer pair, possibly with degenerate nucleotides according IUPAC nomenclature. Genomic DNA purified from both pure cultures and from a mixture of different strains can be used as template in the PCR reaction. PCR products are digested by a specific restriction enzyme (RE), i.e. Hae III, and the resulting products are separated and detected by agarose gel electrophoresis, thus allowing the identification of each pure culture and the identification of the components in the mixture of different species thanks to the comparison with the profile of each pure culture.

In the third protocol based on length polymorphism of the *mleA-mleR* target within the *Oenococcus* genus, a combination of the previous two protocols is applied, as the PCR products obtained by amplifying with a primer pair, possibly with degenerate nucleotides according IUPAC nomenclature, and one of which is fluorescent marked are digested by a specific restriction enzyme (RE), i.e. Hae III. Fluorescent marked PCR products, digested by the RE are separated and detected by means of capillary electrophoresis thus allowing the identification of each pure culture and of the components of the mixture of different species.

In the fourth PCR-based protocol sequence polymorphisms of the *mleA-mleR* target within the *Oenococcus* genus are detected, as the obtained PCR products are further analyzed by Denaturing Gradient Gel Electrophoresis (DGEE) in a Polyacrylamide gel containing a formamide gradient which denature and differentiate the PCR products depending on their sequence. The primers applied in this protocol must not to show degenerate nucleotides according IUPAC nomenclature, otherwise artifacts are generated because also sequence polymorphisms in the primer region affect PCR product denaturation in formamide gradient. Genomic DNA purified from both pure cultures and from a mixture of strains can be used as template in the PCR reaction, thus allowing the identification of each pure culture and of the components of the mixture of different species.

According to yet another aspect of the invention, a nucleotide stretch comprised in the *mleA-mleR* target and conserved in at least three species of the *Oenococcus* genus, i.e. *Oenococcus oeni, Oenococcus kitaharae* and *Oenococcus alcoholitolerans,* is used to design one genus-specific oligonucleotides or a xeno-nucleic acid aptamers to be applied as a probe for the quantification with different technologies such as qPCR, FISH, flow-cytofluorimetry, FISH-FLOW.

According to yet another aspect of the invention, the *Oenococcus* genus-specific probe is a oligonucleotide or xeno-nucleic acid aptamer modified with a fluorescent molecule and optionally also with a quencher molecule to be applied together with species-specific primer pairs designed in variable regions, to quantify each species by separately qPCR, but using only one probe.

According to yet another aspect of the disclosure but not part of the claimed invention a genus-specific primer pair probe is coupled with species-specific oligonucleotides or xeno-nucleic acid aptamers modified with a fluorescent molecule and optionally also with a quencher molecule to be applied together to quantify each species thanks to a multiplexed qPCR.

According to yet another aspect of the invention, the length polymorphism of the *mleA-mleR* target showed to be a suitable and robust characteristic to differentiate and identify species belonging to the *Lactobacillus casei* group, i.e. *Lactobacillus casei, Lactobacillus paracasei* subsp. *paracasei, Lactobacillus paracasei* subsp. *tolerans, Lactobacillus rhhamnosus,* that are very related from a phylogenetical view-point, and for this reason the methods based on the ribosomal RNA operon sequence analysis are poorly discriminatory, especially when they are mix together. More in detail, a newly design degenerate primer pair allow to differentiate and identify *L. casei, L. paracasei* and *L*. *rhamnosus* by means of an agarose gel electrophoresis. The two subspecies of *L*. *paracasei* show the same PCR product dimension.

According to a one aspect of the invention, the target sequence comprising the genes coding for Malolactic Enzyme (known as *mleA* or *mleS*) and the Malolactic Enzyme Regulator, a lysR-type transcriptional regulator (*mleR*) is applied to detect, to differentiate and to identify bacterial species belonging to different Lactic Acid Bacteria genus such as *Lactobacillus, Lactococcus, Leuconostoc, Oenococcus, Pediococcus, Streptococcus.* According to yet another aspect of the invention, a nucleotide stretch conserved in at least two different genus, such as the nonamer "TTTGACTAT" that is comprised in the previously defined *mleA* (*mleS*) and *mleR* target sequence and it is located in the inter-genic region corresponding to the promoter of the *mleA* gene, is used to design oligonucleotide or xeno-nucleic acid aptamer, such as an LNA oligonucleotide, and applied as an universal probe together with species- specific primer pairs to detect and quantify several Lactic Acid Bacteria species.

According to yet another aspect of the invention, the target sequence including the *mleA* (*mleS*) and *mleR* genes can be Sanger sequenced, aligned and analyzed to differentiate strains belonging to the same species, such as *Oenococcus oeni* and *Lactobacillus brevis.* The target sequence including *mleA* and *mleR* genes, therefore become a DNA barcode for strain typing and/or one additional locus for MultiLocus Sequence Analysis (MLSA) and/or MultiLocus Sequence Typing (MLST).

In another aspect of the invention, the target sequence comprising the Citrate Lyase gene cluster and especially the gene coding for Oxaloacetate Decarboxylase which is related to soluble Malate Decarboxylase (known as *mae*) and transcriptional regulator (*citR*)*,* shortened to *"mae-citR* target", oriented on opposite direction on two DNA strands, is applied to detect, to differentiate and to identify bacterial species thanks both to the sequence polymorphism and length polymorphism.

According to yet another aspect of the invention the *mae-citR* target sequence is used to differentiate, to identify and to quantify *Leuconostoc mesenteroides* subsp. *cremoris* and *Leuconostoc mesenteroides* subsp. *dextranicum.*

According to yet another aspect of the invention, the *mae-citR* target and *mleA-mleR* target are used together in multiplexed protocols to differentiate, to identify and to quantify bacteria, such as cocci from dairy products, from wine-related environment, from pharmaceutical products and from dietary-suppliers belonging to the genus *Lactococcus, Leuconostoc, Oenococcus, Pediococcus Streptococcus.*

In another aspect of the invention, the target sequence comprising the genes coding for Transketolase (known as *tkt*) and the Heat-inducible Transcription Repressor (*hrcA*)*,* shortened to *"tkt-hrcA* target", oriented on opposite direction on two DNA strands, is applied to detect, to differentiate and to identify bifidobacteria thanks to both the sequence polymorphism and length polymorphism.

According to yet another aspect of the invention the *tkt-hrcA* target sequence is applied to differentiate, identify and quantify the species *Bifidobacterium animalis* subsp. *animalis, Bifidobacterium animalis* subsp. *lactis, Bifidobacterium breve, Bifidobacterium longum* subsp. *longum, Bifidobacterium longum* subsp. *infantis* and *Bifidobacterium longum* subsp. *suis.*

According to yet another aspect of the invention the *tkt-hrcA* target sequence is applied to differentiate and identify the subspecies *Bifidobacterium animalis* subsp. *animalis, Bifidobacterium animalis* subsp. *lactis,* thanks to length polymorphism, due to a difference of 12 nucleotides in the region in-between the *tkt* and *hrcA* corresponding to the gene promoter.

According to yet another aspect of the invention, a nucleotide stretch conserved in at least three different species of the *Bifidobacterium* genus, i.e. *Bifidobacterium animalis* subsp. *animalis, Bifidobacterium animalis* subsp. *lactis, Bifidobacterium breve, Bifidobacterium longum* subsp. *longum, Bifidobacterium longum* subsp. *infantis* and *Bifidobacterium longum* subsp. *suis* such as the 9-mer "ACAAGCCGG", "GAGTGCTAAT" (SEQ ID NO: 25) the 11-mer "AACACGCCAAA" (SEQ ID NO: 23) and the two 15-mer "ATTGGAAGGAAAGTA" (SEQ ID NO:24) "ATTGTATTAGCACTC" (SEQ ID NO: 26) that is comprised in the previously defined *tkt-hrcA* target sequence and it is located in the inter-genic region corresponding to the promoter of the *tkt* gene, is used to design oligonucleotide or xeno-nucleic acid aptamer, such as an LNA oligonucleotide, and it is applied as an *Bifidobacterium-specific* probe together with species- specific primer pairs to detect and quantify bifidobacteria.

In another aspect of the invention, the target sequence comprising the genes coding for the Extracellular Nuclease involved in Sporulation and Transcriptional Sigma Factor 70, shortened to *"ens-sf70* target", oriented on opposite direction on two DNA strands, is applied to detect, to differentiate and to identify bacterial species within the *Bacillus genus* thanks to the sequence polymorphism and length polymorphism.

In another aspect of the invention, the target sequence comprising the genes coding for the Primase and NAD(P) Tranhydrogenase subunit Alpha oriented on opposite direction on two DNA strands, is applied to detect, to differentiate and to identify bacterial species within the Acetic Acid Bacteria and especially those belonging to the genus *Gluconobacter* thanks to the sequence polymorphism and length polymorphism. According to yet another aspect of the invention the above cited target sequences are applied together in multiplexed protocols and applications in order to enhance the capacity to differentiate to identify and the quantify different Taxa from different sources, such as environmental samples, water samples, soil samples, plant-related samples, dairy products, wine-related environment, dough, sourdough, fermented food, fermented beverage, fecal samples, pharmaceutical products and dietary-suppliers.

The characteristics of the invention is dramatically distinct from known markers applied for the differentiation, identification and quantification of microorganisms and viruses. The method to use genetic markers invented here guarantee indeed both specificity and also robustness. Two levels of specificity allow to use the molecular markers gene for identification and quantification of related species also in mixture, avoiding mis-identifications and false-positive results, even using primers and probes designed on conserved sequences in phylogenetically far related microorganisms such as those belonging to *Lactococcus* and *Oenococcus* genus .

Various embodiments and aspects of the present invention as delineated hereinabove and as claimed in the claims section below find experimental support in the following examples.

### EXAMPLES

Reference is now made to the following examples, which together with the above descriptions illustrate some embodiments of the invention. Example 9 is not an Example of the invention

### Example 1.

### Example 1: Sanger sequencing of the H.P.M.E. marker, preferably represented in the Oenococcus genus by the H.P.M.E. mleA-mleR marker, followed by the alignment and the comparison with the homologous H.P.M.E. mleA-mleR marker of Oenococcus oeni Oenococcus kitaharae and Oenococcus alcoholitolerans.

One preferred form of the H.P.M.E. marker is represented in the *Oenococcus* genus by a target sequence comprising: a) the *mleA* gene coding for Malolactic Enzyme; b) the *mleR* gene coding for the Malolactic Enzyme Regulator, i.e. a lysR family transcriptional regulator, oriented in opposite direction from the *mleA* gene on the complementary strand of the DNA; c) the region corresponding to the gene trascriptional promoter that is between the *mleA* gene and the *mleR* gene previously described.

The H.P.M.E. *mleA-mleR* marker for the species *Oenococcus oeni* is represented in the preferred form by the sequence of the strain *Oenococcus oeni* PSU-1 (gi|116490126:1481086-1483687) and for the species *Oenocococcus kitaharae* is represented in the preferred form by *Oenococcus kitaharae* DSM 17330 (gi|372325750:114550-117167).

The H.P.M.E. *mleA-mleR* marker for the species *O. alcoholitolerans* was obtained by Sanger sequencing with one of the four primers applied in two possible PCRs, one of which is characterized by the use of primers with degenerated nucleotide sites, according the IUPAC nomenclature.

The first PCR is applied with the primer OEX_mleR_F1_seq, which sequence is AAATAATAATTACCAATGATTTGCGG (SEQ ID NO: 12) and OEX_mle-R1_seq, which sequence is ATACCAGTTCCCTGAATATCATC (SEQ ID NO: 13);

The second PCR is characterized by the use of primers with degenerated nucleotide sites according the IUPAC nomenclature: OEX_mleR_FN_seq, which sequence is AARTAAWAATTWCCRATDATYTGCGG (SEQ ID NO: 14); and OEX_mle-RN_seq, which sequence is ATWCCDGTDCCYTGRATATCATC (SEQ ID NO: 15).

The conditions were same for both the PCRs: the reaction was performed in 20µl volume using 1 U of DNA polymerase GoTaq (Promega) in 1x buffer with addition of magnesium chloride (MgCl₂) at the final concentration of 1.5 mM and tri-phosphate nucleotides (dNTPs) at the final concentration of 0.2 mM each. The concentration of the primer was 1 µM each. Amplification was in the Nexus Mastercycler Gradient instrument (Eppendorf) with a thermal program consisting of an initial denaturation step at 94°C for 5 minutes followed by one cycle at 94°C for 30 seconds, 54°C for 30 seconds and 72°C for 60 seconds, repeated 35 times, followed by a final extension step at 72°C for 10 minutes. About 20 ng of purified genomic DNA from the strain *Oenococcus alcoholitolerans* LMG 27599 was applied as template DNA in the PCR. The PCR product was purified and sequenced according Sanger reaction protocol.

The resulting sequence of the H.P.M.E. *mleA-mleR* marker for the species *O*. *alcoholitolerans* was aligned and compared with the homologous region of the H.P.M.E. *mleA-mleR* marker of *O*. *oeni* and *O. kitaharae* (FIGURE 2).

The sequence similarity between *O*. *oeni* and *O. kitaharae* is 72,04%, the sequence similarity between *O*. *oeni* and *O. alcoholitolerans* is 71,04% and the sequence similarity between *O. kitaharae* and *O. alcoholitolerans* is 71,69%.

The analysis of the alignment (Figure 2) clearly show that the region of the *O*. *alcoholitolerans* sequenced by Sanger reaction, compared with *O. oeni* and *O. kitaharae* is characterized by all the characteristics of a H.P.M.E. marker. The three sequences are indeed highly polymorphic because of considering the low percentage similarity and the presence insertion/deletion, i.e. length polymorphisms. Despite the highly polymorphic sequence previously described, conserved domains and in general, conserved DNA stretches, are available. This is clearly demonstrated by the possibility to design oligonucleotides (Figure 2) to be applied successfully as robust primers/probes (OeX_mleR_68-F1, OeX_mleA_01-R1, OeX_mleA_244-RN) in the following examples.

### Example 2: differentiation and identification of the species within the Oenococcus genus by capillary electrophoresis of labelled PCR products, obtained with primers designed on the H.P.M.E. mleA-mleR marker, comprising insertions/deletions in the promoter region.

The DNA extracted from the pure cultures of *O*. *oeni, O. kitaharae* and *O. alcoholitolerans* was amplified with two different PCR protocols.

The PCR was carried out with the primer OeX_mleR_68-F2, which sequence is GTCGGTTGGCTGACATTGAAAAAA (SEQ ID NO:16) and OeX_mleA_01-R1, which sequence is TTTAAAATACCTACTGGATCTGTCAT (SEQ ID NO:17) and it was modified with the addition of FAM fluorescent dye.

The reaction was performed in 20µl volume using 1 U of DNA polymerase GoTaq (Promega) in 1x buffer with addition of magnesium chloride (MgCl₂) at the final concentration of 1.5 mM and tri-phosphate nucleotides (dNTPs) at the final concentration of 0.2 mM each. The concentration of the primer was 1 µM each. Amplification was in the Nexus Mastercycler Gradient instrument (Eppendorf) with a thermal program consisting of an initial denaturation step at 94°C for 5 minutes followed by one cycle at 94°C for 30 seconds, 59°C for 30 seconds and 72°C for 45 seconds, repeated 35 times, followed by a final extension step at 72°C for 10 minutes.

The same protocol was repeated also with the DNA purified from mixed culture of *O. oeni* and *O. kitaharae, O. oeni* and *O. alcoholitolerans, O. kitaharae* and *O. alcoholitolerans,* and finally the mixed culture of all the three species belonging to the genus *Oenococcus.* The amplification was followed by separation and detection of the PCR products by capillary electrophoresis using the filter D: and ROX as an internal reference. The profile analysis in the capillary was performed with the Peak Scanner 2.0 software with the following parameters: "size standard: GS500 (-250)" and "analysis method: default sizing
- NPP". The results of the detection are reported in Table 1 and clearly show that the three species are characterized by different size of the amplified region, as expected by in-silico analysis (Figure 2). More in details the difference between *O*. *oeni* and *O*. *kitaharae* is 1 nucleotide, the difference between *O*. *oeni* and *O. alcoholitolerans* is 5 nucleotides and the difference between *O. kitaharae* and *O. alcoholitolerans* is 4 nucleotides.

The three peaks specific for *O*. *oeni O. kitaharae* and *O. alcoholitolerans* were clearly detected also in the mixed culture of all the three species together (Figure 3).

This example show that the stretches of conserved sequences found in the H.P.M.E. *mleA-mleR* marker can be used to design primers that successfully amplify all the target samples. Moreover the insertion or the deletion observed in the region of the H.P.M.E. *mleA-mleR* marker corresponding to the gene promoter can be successfully applied in a detection method to differentiate and identify *Oenococcus* spp. strains both in pure and in mixed cultures on the basis of length polymorphism.

**Table 1. Results obtained by capillary electrophoresis for the labelled PCR products from pure cultures and mixed cultures of species belonging to Oenococcus genus.**

| Sample | PCR1 product size (bp) |
|---|---|
| *O. oeni* DSM 20252 | 199 |
| *O. kitaharae* DSM 17330 | 200 |
| *O. alcoholitolerans LMG* 27599 | 204 |
| *O. oeni* DSM 20252 and *O. kitaharae* DSM 17330 | 199, 200 |
| *O. oeni* DSM 20252 and *O. alcoholitolerans* LMG 27599 | 199, 204 |
| *O. kitaharae* DSM 17330 and *O. alcoholitolerans* LMG 27599 | 200, 204 |
| *O. oeni* DSM 20252, *O. kitaharae* DSM 17330 and *O*. *alcoholitolerans* LMG 27599 | 199, 200, 204 |

### Example 3: differentiation and identification of the species within the Oenococcus genus by PCR-RFLP by agarose gel electrophoresis, obtained with primers designed on the H.P.M.E. mleA-mleR marker.

PCR-RFLP is a molecular biology technique developed to differentiate and to compare different samples on the basis of the differences in nucleotide sequence of a specific and variable genetic target, which is amplified by PCR. The differences in the nucleotide sequence between the samples are detected indirectly thanks to a digestion with a Restriction Enzyme that recognize and cut the PCR product in specific short palindromic sequences. The target sequence of the Restriction Enzyme Hae III, used in this example, is "GGCC". PCR products characterized by different sequences and a different number of target sequence, show different profiles after the digestion with the same enzyme

The DNA extracted from the pure cultures of *O*. *oeni, O. kitaharae* and *O. alcoholitolerans* was amplified with two different PCR protocols.

The PCR was carried out with the primer OeX_mleR_68-F2, which sequence is GTCGGTTGGCTGACATTGAAAAAA (SEQ ID NO:16) and OeX_mleA_244-RN, which sequence is CRATYGGCATRAATTCAACMACRTG (SEQ ID NO:18) and it is characterized by the presence of degenerated nucleotides according the IUPAC nomenclature.

The reaction was performed in 20µl volume using 1 U of DNA polymerase GoTaq (Promega) in 1x buffer with addition of magnesium chloride (MgCl₂) at the final concentration of 1.5 mM and tri-phosphate nucleotides (dNTPs) at the final concentration of 0.2 mM each. The concentration of the primer was 1 µM each. Amplification was in the Nexus Mastercycler Gradient instrument (Eppendorf) with a thermal program consisting of an initial denaturation step at 94°C for 5 minutes followed by one cycle at 94°C for 30 seconds, 59°C for 45 seconds and 72°C for 60 seconds, repeated 30 times, followed by a final extension step at 72°C for 10 minutes.

The same protocol was repeated also with the DNA purified from mixed culture of *O. oeni* and *O. kitaharae, O. oeni* and *O. alcoholitolerans, O. kitaharae* and *O. alcoholitolerans,* and finally the mixed culture of all the three species belonging to the genus *Oenococcus.* The amplification was followed by digestion with the Restriction Enzyme Hae III, according the instruction of the producer. The digested PCR products were analysed by electrophoresis on a 3% agarose gel. The result (Figure 4) clearly show that the three species belonging to *Oenococcus* genus are characterized, as expected, by different RFLP profiles and their presence is detectable also when they are present in a mixed culture at the same concentration.

This example shows that nucleotide stretches in the H.P.M.E. *mleA-mleR* marker, that are characterized by low variability in the three species of the *Oenococcus* genus, can be used to design new primers with degenerate nucleotide according IUPAC nomenclature. In this specific case the new primer is OeX_mleA_244-RN which was combined with the previously tested OeX_mleR_68-F2 to successfully amplify all the target samples. Moreover the high sequence variability shown by H.P.M.E. *mleA-mleR* marker (Example 1) was confirmed indirectly thanks to the digestion with the Restriction Enzyme and it was successfully applied to differentiate and identify *Oenococcus* spp. strains both in pure and in mixed cultures on the basis of sequence polymorphism.

### Example 4: differentiation of all the species of the Oenococcus genus present in a mixture by PCR-DGGE, a metagenomics technique for culture-independent ecological studies, using a primer pair designed on the H.P.M.E. mleA-mleR marker, adapted for this purpose.

Denaturing Gradient Gel Electrophoresis, known also as PCR-DGGE, is a molecular biology technique developed to differentiate bacterial species in a mixture on the basis of the nucleotide sequence of a specific target amplified by PCR, thanks to an electrophoresis migration in a denaturing gel containing a variable Formamide concentration. One primer is modified with addition of a GC clamp, i.e. a strand of DNA rich in Guanine and Cytosine that is long about 40 bp, which is not denatured by the working concentration of Formamide. PCR products loaded on the electrophoresis gel are denatured by Formamide, depending on their nucleotide composition. The GC-clamp blocks the migration of the PCR products whenever they have been denatured by Formamide, generating a profile of DNA bands that display the number of the microbial components of the initial sample that are amplified by PCR (usually the microbial components represented by less than 1% of the total microbial population are not detected).

The PCR has been carried out with the forward primer OeX_mleR_68-F2-GC, which sequence is GTCGGTTGGCTGACATTGAAAAAA (SEQ ID NO:16) and it was modified with the addition of a GC clamp rich in Guanine and Cytosine according Walter *et al.* (2000). The primer reverse was OeX_mleA_01-R, that is TTTAAAATACCTACTGGATCTGTCAT (SEQ ID NO:17).

The reaction was performed in 20µl volume using 1U of DNA polymerase GoTaq (Promega) in 1x buffer with addition of magnesium chloride (MgCl₂) at the final concentration of 1.5 mM and tri-phosphate nucleotides (dNTPs) at the final concentration of 0.2 mM each. The concentration of the primer was 1 µM each.

Amplification was in the Nexus Mastercycler Gradient instrument (Eppendorf) with a thermal program consisting of an initial denaturation step at 94°C for 5 minutes followed by one cycle at 94°C for 30 seconds, 54°C for 30 seconds and 72°C for 45 seconds, repeated 30 times, followed by a final extension step at 72°C for 5 minutes.

The Formamide concentration in the denaturing polyacrylamide gel was ranging from 20% to 40%, starting from stock solutions prepared according Walter *et al.* (2000). The electrophoretic run was carried out in a DCode system (BioRad) with a warm-up step at 90V for 10 minutes, followed by a run at 50V for 16 hours.

A the end of the run, DNA bands were stained with a UV-fluorescent DNA double helix intercalating molecule.

The gel picture in Figure 5 clearly show that the three species belonging to the genus *Oenococcus* give a unique profile, each. his is in agreement with the in-silico analysis and the observation of the H.P.M.E. *mleA-mleR* marker alignment for the species *O*. *oeni, O. kitaharae* and *O. alcoholitolerans. O. kitaharae* is characterized by an higher band mobility, followed by *O. alcoholitolerans* and then by *O. oeni.*

Thanks to the specific mobility pattern, the three species are clearly differentiated and identified also when they are present together in a mixture.

This Example clearly shows that the H.P.M.E. *mleA-mleR* marker is a flexible marker that can be adapted to different technological platform and protocols. The primer pair applied for the PCR-DGGE are, indeed, the same applied in Example 2 for capillary electrophoresis, but with a different modification: the addition of the GC-clamp, instead of the FAM labelling.

Moreover Example 4 demonstrate that H.P.M.E. *mleA-mleR* marker is suitable and robust to be applied successfully in a case-study regarding a culture-independent ecological analysis, and analogously it would be adapted to support Next Generation Sequencing based Metagenomics.

### Example 5: improvement of a PCR-based protocol for the identification of Lactobacillus casei, Lactobacillus paracasei and Lactobacillus rhamnosus, thanks to a primer pair designed on the sequences of the H.P.M.E. mleA-mleR marker

The species of Lactic Acid Bacteria *Lactobacillus casei, Lactobacillus paracasei* subsp. paracasei, *Lactobacillus paracasei* subsp. *tolerans* and *Lactobacillus rhamnosus* are phylogenetically very related and belong to the *Lactobacillus casei* group.

One of the most applied and cited method to identify these four species is a PCR-based protocol developed by Ward and Timmins in 1999, which is characterized by three different PCR. One primer, namely primer Y2, is common for the three PCR, while specific primers, namely CASEI, PARA and RHAMN were specific respectively for the species *Lactobacillus casei, Lactobacillus paracasei* and *Lactobacillus rhamnosus.* The size of the three PCR products is similar, as they were designed on the homologous nucleotide positions *Lactobacillus casei, Lactobacillus paracasei* and *Lactobacillus rhamnosus,* within the variable region V1 of the 16S rRNA. For this reason they cannot be applied together to identify these species in a mixed culture, but in three distinct PCRs (Figure 6A, 6B and 6C).

The new PCR developed here thanks to a thanks to a primer pair designed the homologous nucleotide positions within the sequences of the H.P.M.E. *mleA-mleR* marker for the species *Lactobacillus casei, Lactobacillus paracasei* and *Lactobacillus rhamnosus* allow to differentiate them on the basis of length polymorphism (Figure 6D). The PCR has been carried out with the forward primer mleR_cas-G-F1, which sequence is TCCCAYGCAATKTCYTGYTKSGC (SEQ ID NO:19) and with primer reverse mleA_cas-G-R1, which sequence is GCNGTNCCNTTNTTTAAAAANGG (SEQ ID NO:20).

This example shows that the H.P.M.E. *mleA-mleR* marker can be used to design primers that successfully amplify the target samples also in the case of *Lactobacillus* genus. Moreover the insertion/deletion observed in the region of the H.P.M.E. *mleA-mleR* marker corresponding to the gene promoter can be successfully applied in a detection method to differentiate lactobacilli belonging to phylogenetically related species such as *Lactobacillus casei, Lactobacillus paracasei* and *Lactobacillus rhamnosus* improving the state of the art.

### Example 6: primers designed on the H.P.M.E. mleA-mleR marker to sequence phyloaenetically unrelated lactobacilli have been successfully applied also for typing different L. brevis strains and analysing the intraspecific variability.

*Lactobacillus brevis* and *Lactobacillus plantarum* are widespread species involved also in many technological processes, ranging from agri-food to medical applications. Despite this common features and applications, they are phylogenetically unrelated species according Hammes and Vogel (1995), as *L. brevis* belongs to the Group C of obligately heterofermentative lactobacilli, whereas *L. plantarum* belongs to the Group B of facultative heterofermentative lactobacilli.

As they are fundamental lactobacilli from both and ecological and a technological view-point, a primer pair for amplification by PCR, sequencing and identification at the species level was developed on the H.P.M.E. *mleA-mleR* marker. More in detail, the sequences of the H.P.M.E. *mleA-mleR* marker already available in public databases for the strain *L*. *brevis* ATCC 367 (gi|116332681:2167769-2170426) and *L. plantarum* WCSF1 (gi|342240345:1014055-1016775) were aligned and analysed.

Despite the low percentage of similarity, i.e. 70,41 % in pairwise alignment, and a deletion of 35 bp in the promoter region of *L. brevis,* two conserved DNA stretches have been found within the *mleA* and in *mleR* genes, as well as the conserved 9-mer "TTTGACTAT" in the extragenic sequence corresponding to the promoter region. The conserved DNA stretches on *mleA* and *mleR* genes have been used to design the primer Lb_br-pl_mleA-R1_seq, of which the sequence is TCATAAACGATTGGCATAAATTC (SEQ ID NO:21) and the primer Lb_br-pl_mleR-F1_seq, of which the sequence is characterized by some degenerated nucleotides according IUPAC nomenclature: CGRTCAAATTCTTCRGCAATCA (SEQ ID NO:22).

The PCR was successfully applied also to amplify ten different strains belonging to the species *L. brevis,* namely *L. brevis* strains LMG 6906; *L. brevis* JCM 17312; *L. brevis* 11401; *L. brevis* 11989; *L. brevis* 11435; *L. brevis* 12023; *L. brevis* 25561; *L. brevis* 11434; *L. brevis* LMG 11998; *L. brevis* LMG 18022.

More in detail, the reaction was performed in 20µl volume using 1U of DNA polymerase GoTaq (Promega) in 1x buffer with addition of magnesium chloride (MgCl₂) at the final concentration of 1.5 mM and tri-phosphate nucleotides (dNTPs) at the final concentration of 0.2 mM each. The concentration of the primer was 1 µM each. Amplification was in the Nexus Mastercycler Gradient instrument (Eppendorf) with a thermal program consisting of an initial denaturation step at 94°C for 5 minutes followed by one cycle at 94°C for 30 seconds, 60°C for 30 seconds and 72°C for 60 seconds, repeated 30 times, followed by a final extension step at 72°C for 5 minutes.

The PCR products obtained for the ten strains were purified and sequences according the Sanger's method. The ten sequences of the H.P.M.E. *mleA-mleR* marker were aligned and analysed to find the relationship between the ten strains. The dendrogram (Figure 7A) obtained by analysing the RAPD-PCR profile with the primer 1281 according Akopyanz et al. (1991) was compared with the phylogenetic tree (Figure 7B) resulting from the ten sequences of the H.P.M.E. *mleA-mleR* marker.

This Example clearly show that the the H.P.M.E. *mleA-mleR* is a flexible marker to be used both for characterize phylogenetically unrelated species of the *Lactobacillus* genus, as well as for the typing of different strains belonging to the same species. This final application suggests that the H.P.M.E. *mleA-mleR* marker could be included in a MLST scheme for strain typing.

### Example 7: improvement of a PCR-based protocol for the identification of species belonging to Bifidobacterium genus, thanks to the H.P.M.E. tkt-hrcA marker constituted by the partial sequence of the tkt gene and the hrcA gene and the complete sequence of the gene promoter

The species *Bifidobacterium* breve, *Bifidobacterium animalis* subsp. *lactis* and *Bifidobacterium longum* have been applied in many nutraceutical and medical products. *Bifidobacterium animalis* subsp. *lactis* is phylogenetically related with the subspecies *Bifidobacterium animalis* subsp. *animalis,* while the species Bifidobacterium longum is constituted by three subspecies: *Bifidobacterium longum* subsp. *longum, Bifidobacterium longum* subsp. *infantis* and *Bifidobacterium longum* subsp. *suis.*

One of the most applied and cited method to identify *Bifidobacterium breve* is a PCR-based protocol developed by Matsuki *et al.* (1999), which target is 16S rRNA. Unfortunately the PCR protocol can give false positive results also for *Bifidobacterium animalis* subsp. *animalis* and *Bifidobacterium animalis* subsp. *lactis* (Figure 8) probably due to the great efficacy of the new enzyme and reagents compared to those used when the PCR was developed.

The H.P.M.E. *tkt-hrcA* marker, constituted by the partial sequence of the *tkt* gene coding for Transketolase and the *hrcA* gene coding for the Heat-inducible Transcription Repressor, i.e. a gene regulator protein according PFAM nomenclature, and the complete sequence of the gene promoter is a suitable and reliable genetic marker for the differentiation, the identification and the quantification of *Bifidobacterium breve, Bifidobacterium animalis* subsp. *animalis, Bifidobacterium animalis* subsp. *lactis, Bifidobacterium longum* and *Bifidobacterium bifidum* (Figure 9; 10A and 10B)

More in detail, the extragenic region where also promoter is located, is characterized by insertion/deletion events which allow to differentiate the three species by length polymorphism. Moreover length polymorphism due to insertion/deletion differentiates also *Bifidobacterium animalis* subsp. *animalis* and *Bifidobacterium animalis* subsp. *lactis* (Figure 9; 10A and 10B). The preferred regions were used to design oligonucleotides to be used as primers, such as BfX_hrc_46-FN2 TARTCYTCYACVAYRGCSCGMAG (SEQ ID NO: 65) and BfX_tkt_170-RN2

TCRTTCGGATCRTGCTTGATG (SEQ ID NO: 66) and probes based on BfX_tkt-hrcA_Stretch_1 ACAAGCCGG, BfX_tkt-hrcA_Stretch_2 GAGTGCTAAT (SEQ ID NO: 25), BfX_tkt-hrcA_Stretch_3 AACACGCCAAA (SEQ ID NO: 23), BfX_tkt-hrcA_Stretch_4 ATTGGAAGGAAAGTA (SEQ ID NO:24), BfX_tkt-hrcA_Stretch_5 ATTGTATTAGCACTC (SEQ ID NO: 26) for different applications (Figure 9; 10A and 10B).

The PCR was carried out with the primer BfX_hrc_46-FN2 TARTCYTCYACVAYRGCSCGMAG (SEQ ID NO: 65) and BfX_tkt_170-RN2 TCRTTCGGATCRTGCTTGATG (SEQ ID NO: 66) characterized by the presence of degenerated nucleotides according the IUPAC nomenclature.

The reaction was performed in 20µl volume using 1 U of DNA polymerase GoTaq (Promega) in 1x buffer with addition of magnesium chloride (MgCl₂) at the final concentration of 1.5 mM and tri-phosphate nucleotides (dNTPs) at the final concentration of 0.2 mM each. The concentration of the primer was 2 µM each. Amplification was in the Nexus Mastercycler Gradient instrument (Eppendorf) with a thermal program consisting of an initial denaturation step at 94°C for 5 minutes followed by one cycle at 94°C for 30 seconds, 65°C for 45 seconds and 72°C for 60 seconds, repeated 30 times, followed by a final extension step at 72°C for 10 minutes.

This example clearly show that the H.P.M.E. *tkt-hrcA* marker is the solution to improve the state of the art regarding the differentiation and the identification of *Bifidobacterium breve, Bifidobatrium animalis Bifidobacterium longum* and *Bifidobacterium bifidum.*

Moreover this example represents the confirmation that the H.P.M.E. markers such as *tkt-hrcA* for bifidobacteria and *mleA-mleR* for Lactic Acid Bacteria is a very useful class of genetic markers to develop new protocols and applications for the differentiation and the identification of microorganisms.

### Example 8: technical advantages in the application of H.P.M.E. mleA-mleR marker compared with the exploitation of the mleA gene as a target in PCR-based protocols and as a marker within a Multilocus Sequence Typing (MLST) scheme to differentiate and characterize the three species belonging to the Oenococcus genus and different strains within the Onococcus oeni species.

The genus *Oenococcus* is constituted by three species *Oenococcus oeni, Oenococcus kitaharae* and *Oenococcus alcoholitolerans. O. oeni* is undoubtedly the most important and the most studied mainly because it was isolated more frequently than the other two species and also because it has a fundamental role in leading the malolactic fermentation in red wine and cider. Nothing is known about the possible commercial application of *O*. *kitaharae* and *O. alcoholitolerans.*

Due to its ecological and also commercial significance, many *O*. *oeni* strains have been collected and characterized in the framework of by different private and public research projects.

The most applied and cited method to identify, quantify and to characterize different *O*. *oeni* strains are PCR/qPCR-based protocols developed by different authors such as Zapparoli *et al.* (1998), Groisillier *et al.,* (1999); Divol *et al.* (2003), de las Rivas *et al.,* (2004), Beltramo *et al.,* (2006). The genetic marker targeted by all the above cited methods was the *mleA* (*mleS*) gene coding for Malolactic Enzyme.

One preferred form of the H.P.M.E. marker is represented in the *Oenococcus* genus by a target sequence comprising: a) the *mleA* gene coding for Malolactic Enzyme; b) the *mleR* gene coding for the Malolactic Enzyme Regulator. i.e. a lysR family transcriptional regulator according PFAM nomenclature, oriented in opposite direction from the *mleA* gene on the complementary strand of the DNA; c) the region corresponding to the gene trascriptional promoter that is between the *mleA* gene and the *mleR* gene previously described.

The H.P.M.E. *mleA-mleR* marker for the species *Oenococcus oeni,* described above, is represented in the preferred form by the sequence of the strain *Oenococcus oeni* PSU-1 (gi|116490126:1481086-1483687); for the species *Oenocococcus kitaharae* is represented in the preferred form by *Oenococcus kitaharae* DSM 17330 (gi|372325750:114550-117167) and for the specie Oenococcus alcoholitolerans by the sequence of the strain *O. alcoholitolerans* LMG 27599.

Besides *O*. *oeni* PSU-1 and the Type Strain of the species *O. kitaharae* DSM 17330 and *O. alcoholitolerans* LMG 27599, three *O*. *oeni* strains, which have been reported to belong to different clusters within the *O*. *oeni species,* based on their own genotype, have been included in the dataset. More in detail Bilhere *et al.* (2009) thanks to Multilocus sequence typing of *Oenococcus oeni* discovered the presence of two subpopulations (Group A and B) shaped by intergenic recombination, and clustered in two different subgroups each (Group A1, A2 and Group B1, B2). Moreover, Dimopoulou *at al.* (2014) characterized a well-defined set of *O*. *oeni* strains which genomes were publicly available exploring their potential for exopolysaccharide (EPS) synthesis both by genome comparison and by analysing their phenotype. Dimopoulou *at al.* (2014) found two main clusters of strains within the species *O*. *oeni:* cluster A (EPS-A) and cluster B (EPS-B). The four *O*. *oeni* strains included in the dataset besides *O. kitaharae* DSM 17330 and *O*. *alcoholitolerans* LMG27599 have been reported to show the following characteristics:
- *O. oeni* PSU-1, a commercial strain from red wine, belongs to the group A1 and EPS-A;
- *O. oeni* IOEB 9803 (gi|692709670|gb|AZKF01000023.1:189036-191637), from red wine, belongs to the group A2 and EPS-B;
- *O. oeni* ATCC BAA-1163 (gi|118432235|gb|AAUV01000058.1|:96988-99589), from red wine, belong to the grou B2 and EPS-B;
- *O. oeni* IOEB 9304 (gi|692713155|gb|AZKl01000016.1:53285-55886), from cider, belongs to the group B2 and EPS-B;

Contrarily to the protocols known up to the date, the H.P.M.E. *mleA-mleR* marker is not constituted only by *mleA* gene but also by the partial sequence of the *mleR* gene and the complete sequence of the gene promoter comprised in between the *mleA* and *mleR* genetic loci, according the Configuration A shown in Figure 1A. This feature confers to the H.P.M.E. *mleA-mleR* marker an increased versatility as it can be applied successfully to different taxonomic units such as genus and species, while the known protocols up to the date failed when they were applied at least to one of the two taxonomic units. Thanks to H.P.M.E. *mleA-mleR marker* it was possible, indeed to differentiate both:
- different species within the same genus, such as *O. oeni, O. kitaharae* and *O*. *alcoholitolerans* within the *Oenococcus* genus, while any other known protocol failed in targeting the only *mleA locus* in at least one of the species *O. kitaharae* and *O*. *alcoholitolerans* (Table 2).
- different strains within the same species, such as *O. oeni* PSU-1, *O. oeni* ATCC BAA-1163, *O. oeni* IOEB 9304, *O. oeni* 9803 within the *Oenococcus oeni* species (Table 3), while the partial sequence of the *mleA* gene amplified with the primer OO1 and 002 according the MLST scheme reported by de las Rivas et al. (2004) was not able to differentiate the same strains (Table 4).

This example represents the confirmation that the H.P.M.E. markers, such as *mleA-mleR* for Lactic Acid Bacteria and *tkt-hrcA* for bifidobacteria, give a remarkable advantage compared to the known protocols up to the date in the differentiation and the identification of microorganisms by combining both highly variable sequence polymorphisms (SNPs) and length polymorphisms (IN/DEL).

**Table 2. Summary of the results of both the in-silico analysis and the corresponding PCR assays (Figures 4, 5, 14A, 14B, 14C, 14D and 14E) regarding the technical advantages of the application of the primers designed on the H.P.M.E. mleA-mleR marker compared with the primers designed to amplify partially the mleA (mleS) gene, coding for Malolactic Enzyme.**

| | Targeted to *Oenococcus* spp. a | Single Nucleotide Polymorphisms (SNPs) ^{b} | Insertions / Deletions (IN/DEL) ^{c} |
|---|---|---|---|
| Primer pair:On1/On2; On3/On4 (Zapparoli *et al.,* 1998) | NO | YES | NO |
| Primer pair: mleS sense / mleS antisense. (Groisillier *et al.,* 1999) | NO | YES | NO |
| Primer pair: OO1 / OO2 (Divol et al. 2003, de las Rivas *et al.,* 2004) | NO | YES | NO |
| Primer pair: mleA forward primer/ mleA reverse primer (Beltramo *et al.,* 2006) | NO | YES | NO |
| Primer pair: Oex_mleR_68-F1 / Oex_mleA_01-R1 (H.P.M.E. *mleA-mleR* marker) | YES | YES | YES |
| Primer pair: Oex_mleR_68-F1 / Oex_mleA_244-RN (H.P.M.E. *mleA-mleR* marker) | YES | YES | YES |

| | | | |
|---|---|---|---|
| Notes ^{a} : the primer pair is designed to anneal and amplify all the three species belonging to the *Oenococcus* genus, i.e. *Oenococcus oeni, Oenococcus kitaharae; Oenococcus alcoholitolerans.* ^{b} : different allelic forms, characterized by specific mutations in define nucleotide sites compared to homologous genetic loci, i.e. Single Nucleotide Polymorphisms (SNPs) have been reported and applied to differentiate strains belonging to the same species (e.g. *Oenococcus oeni*) and or different species (*O*. *oeni, O. kitaharae; O. alcoholitolerans*); ^{c} : length polymorphisms represented by insertion and/or deletion of, at least, one nucleotide site compared to homologous genetic loci have been detected and applied to differentiate species belonging to the same genus (e.g. *O. oeni, O. kitaharae; O. alcoholitolerans*)*..* | | | |

**Table 3. Similarity percentage defined on the basis of the pairwise alignment of the H.P.M.E. mleA-mleR marker sequences in the region comprised within the primers Oex_mleR_68-F1 and Oex_mleA_244-RN.**

| | PSU-1 | ATCC BAA-1163 | IOEB 9304 | IOEB 9803 | DSM 17330 | LMG 27599 |
|---|---|---|---|---|---|---|
| *O. oeni* PSU-1 | 100,00% | 98,72% | 98,47% | 98,47% | 72,08% | 67,83% |
| *O. oeni* ATCC BAA-1163 | 98,72% | 100,00% | 98,98% | 98,72% | 72,33% | 68,09% |
| *O. oeni* IOEB 9304 | 98,47% | 98,98% | 100,00% | 99,49% | 72,08% | 68,34% |
| *O. oeni* IOEB 9803 | 98,47% | 98,72% | 99,49% | 100,00% | 72,58% | 68,09% |
| *O. kitaharae* DSM 17330 | 72,08% | 72,33% | 72,08% | 72,58% | 100,00% | 68,59% |
| *O*. *alcoholitolerans* LMG 27599 | 67,83% | 68,09% | 68,34% | 68,09% | 68,59% | 100,00% |

**Table 4. Similarity percentage defined on the basis of the pairwise alignment of the partial mleA gene sequences in the region comprised within the primers OO1 and 002, according de las Rivas et al. (2004).**

| | PSU-1 | ATCC BAA-1163 | IOEB 9304 | IOEB 9803 | DSM 17330 | LMG 27599 |
|---|---|---|---|---|---|---|
| *O. oeni* PSU-1 | 100,00% | 100,00% | 100,00% | 100,00% | not amplified | not amplified |
| *O. oeni* ATCC BAA-1163 | 100,00% | 100,00% | 100,00% | 100,00% | not amplified | not amplified |
| *O. oeni* IOEB 9304 | 100,00% | 100,00% | 100,00% | 100,00% | not amplified | not amplified |
| *O. oeni* IOEB 9803 | 100,00% | 100,00% | 100,00% | 100,00% | not amplified | not amplified |
| *O. kitaharae* DSM 17330 | not amplified | not amplified | not amplified | not amplified | not amplified | not amplified |
| *O*. *alcoholitolerans* LMG 27599 | not amplified | not amplified | not amplified | not amplified | not amplified | not amplified |

### The following Example 9 is not an example of the invention

### Example 9: technical advantages in the application of both H.P.M.E. rsmD-ftsY marker and H.P.M.E. ftsY-rubA marker compared with the exploitation of the only ftsY genetic locus coding for the protein FtsY as a marker within a Multilocus Sequence Typing (MLST) scheme to differentiate and characterize entomopathogenic bacteria belonging to the Rickettsiella genus and different pathovars within the Rickettsiella grylli species.

Bacteria belonging to the genus *Rickettsiella* are obligate intracellular pathogens of a wide range of arthropods. This genus is currently constituted by three recognized species, *Rickettsiella popilliae, Rickettsiella grylli,* and *Rickettsiella chironomi,* together with numerous further pathotypes or "subjective synonyms". Moreover it is phylogenetically close to vertebrate pathogenic bacteria of the *Legionella* genus (Leclerque, 2008).

As there is no laboratory strain currently available in axenic media or cell culture, the characterization of the *Rickettsiella* obligate intracellular pathogens is mainly based on light and electron microscopic observations. These bacteria were initially classified mainly on the basis of morphologic criteria, including their intracellular location, their oval or rod-like to pleomorphic forms, the occurrence of a complex intravacuolar cycle and the occurrence of crystalline-like structures. The genome of *Rickettsiella grylli* is currently available in GenBank (Accession Number AAQJ00000000), though annotation was generated automatically without manual curation (Mediannikov *et al.,* 2010). Based on the analysis of several genes, a separate taxonomic position was proposed for rickettsiellae (Leclerque, 2008).

In order to improve the characterization of these important entomopathogenic bacteria nine selected genes, i.e. the 16S and 23S rRNA genes and the protein-encoding genes *dnaG, ftsY, gidA ksgA, rpoB, rpsA,* and *sucB* - were evaluated for their potential as markers for the generic and infra-generic taxonomic classification of *Rickettsiella-like* bacteria and the development of a MLST scheme (Leclerque *et al.,* 2011).

Among these genetic markers, the most interesting genetic marker besides its role in the MLST scheme is surely the *ftsY* gene, which encodes the bacterial homolog of the eukaryotic signal recognition particle receptor subunit alpha involved in protein translocation and has previously been identified as the most appropriate single gene marker for the estimation of the G+C content in prokaryotic genomes (Fournier *et al.,* 2006), has recently been introduced as a phylogenetic marker for the characterization of *Rickettsiella-like* bacteria (Mediannikov *et al.,* 2010).

As usual for the Multilocus Sequence Typing approach, a partial sequence constituted by 684 bp of the ftsYgene and its corresponding peptide sequence automatically translated by *in-silico* analysis (228 aminoacids) was considered for strains typing (Leclerque *et al.,* 2011 -Table S2).

Remarkably the *ftsY* gene, when considered together with its flanking genes in two different H.P.M.E. markers represents a great improvement of the prior art, i.e. the MLST scheme (Leclerque *et al.,* 2011).

The first case is represented by H.P.M.E. *rsmD-ftsY* marker, constituted by the partial sequence of the *rsmD* gene coding for 16S rRNA Methyltransferase, oriented in the opposite direction of *ftsY,* which is the gene coding for the signal recognition particle receptor subunit alpha involved in protein translocation. The H.P.M.E. *rsmD-ftsY* marker is characterized by the Configuration A according the definition in Figure 1. The second case is represented by the H.P.M.E *ftsY-rubA* marker, constituted by the gene *ftsY* and the gene coding for Rubredoxin that is identified both with the name *rubA* and *ygaK* and it is characterized by the Configuration B, according the definition in Figure 1.

These two H.P.M.E. markers allow to differentiate *Rickettsiella grylli* and *Candidatus Rickettsiella isopodorum* on the basis of the *in-silico* comparative analysis of the three available genome sequences for this genus.

More in detail, it is possible to differentiate the two species thanks to the H.P.M.E. *rsmD-ftsY* marker by analyzing the region comprised within the primer Rck_rsmD-33-FN GTTTTCTWCCKCGCCATTG (SEQ ID NO: 79) and Rck_ftsY-01-R1 GAGATTCTTTGCGTTTTAAAAATTTAAAC (SEQ ID NO: 74) or within the primer Rck_rsmD-33-FN GTTTTCTWCCKCGCCATTG (SEQ ID NO: 79) and Rck_ftsY-85-RN GTTTTYWGTAAACTATTTTTTATTCG (SEQ ID NO: 75). The regions defined by these two primer pairs are indeed variable both considering the nucleotide sequence and the size, because of the presence of insertion/deletion nucleotide sites (Table 5).

Analogously it is possible to differentiate *Rickettsiella grylli* and *Candidatus Rickettsiella isopodorum* on the basis of both the sequence and the size, thanks to the H.P.M.E. *ftsY-rubA* marker by analyzing the region comprised within the primer pair comprising one of the following three oligonucleotides as the forward primer: Rck_ftsY-834-F1 GCTGACTAAATTAGATGGGACTGCC (SEQ ID NO: 76); Rck_ftsY-942-FN TACABGTTTTYTCWGCAMAGGAATTTG (SEQ ID NO: 77) or Rck_ftsY-947-FN GTTTTYTCWGCAMAGGAATTTG (SEQ ID NO: 78) and one of the two following two oligonucleotides as the reverse primer: Rck_rubA_145-RN CAAAATCTTCTTTCATKGCACC (SEQ ID NO: 80); Rck_rubA-23-RN CACAMAGYARGCACATATATTTTC (SEQ ID NO: 81). It is not possible to predict the size of the PCR product given by the primer pair ftsY fwd / ftsY rev according Leclerque *et al.,* (2011) by *in-silico* analysis.

**Table 5. Summary of the results of the in-silico analysis and virtually corresponding PCR assays (Figure 15) regarding the technical advantages of the application of the primers designed on the H.P.M.E. rsmD-ftsY marker and H.P.M.E. ftsY-rubA marker**

| | Targeted to *Rickettsiella* spp. ^{a} | Single Nucleotide Polymorphisms (SNPs) ^{b} | Insertions / Deletions (IN/DEL) ^{c} |
|---|---|---|---|
| H.P.M.E. *rsmD-ftsY* Primer pair: RcK_rsmD-33-F1/Rck_ftsY-01-R1 | YES | YES | YES |
| H.P.M.E. *rsmD-ftsY* Primer pair: RcK_rsmD-33-F1/ Rck_ftsY-85-RN | YES | YES | YES |
| H.P.M.E. *ftsY-rubA* Primer pair: Rck_ftsY-834-F1 / Rck_rubA-145-RN | YES | YES | YES |
| H.P.M.E. *ftsY-rubA* Primer pair: Rck_ftsY-942-FN / Rck_rubA-23-RN | YES | YES | YES |

### Example 10: technical advantages in the application of H.P.M.E.hrcA-orpE marker to differentiate and characterize species bacteria belonging to the Bacillus genus.

H.P.M.E. *hrcA-grpE* marker, constituted by the partial sequence of the *hrcA* gene and the *grpE* gene and the complete sequence of the gene promoter from *Bacillus subtilis* subsp. *subtilis* NCIB 3610; *Bacillus subtilis* subsp. *spizizenii* TU-B-10; *Bacillus subtilis* subsp. *inaquosorum* KCTC 13429; *Bacillus velezensis* FZB42 and *Bacillus licheniformis* ATCC 14580.

The genes *hrcA* and *grpE* are oriented in the same direction. In the promoter region the conserved sequencesStretch_1 and Stretch_2, corresponding respectively to BcX_hrcA-grpE_1095-F1 GAGGGAGGTGAACACAATGTC (SEQ ID NO: 72) and BcX_hrcA-grpE_1120-R1 GACATTGTGTTCACCTCCCTC (SEQ ID NO: 73) are comprised in the PCR product obtained with the primer pair constituted by BcX_hrcA_991-FN2 TCDGACTTGTCAAAAGCRYTVACAA (SEQ ID NO: 69) or BcX_hrcA_991-FN1 TCDGAYWTGTCWMAAGYRYTVACAA (SEQ ID NO: 68), as forward primer, and BcX_hrpE_1311-RN1 TTWTCRAARTCHGCYTGWASACG (SEQ ID NO: 70) or BcX_hrpE_1311-RN2 TTTTCAAAGTCYGCYTGAACACG (SEQ ID NO: 71), as reverse primer (Figure 16 and 17).

In both the cases species belonging to the *Bacillus* genus can be differentiated both by sequence polymorphisms (SNPs) and by length polymorphisms (insertion/deletion) as reported in Figure 17.

### Example 11: detection and quantification of microorganisms by quantitative real-time PCR (qPCR) combining group-specific TaqMan probes or group-specific short, Locked Nucleic Acid (LNA) TaqMan probes and species-specific primers designed on the H.P.M.E. mleA-mleR marker and H.P.M.E. tkt-hrcA marker and H.P.M.E. hrcA-grpE marker.

Quantitative real-time qPCR methods have been increasingly used as a rapid and sensitive technique for the detection of microorganisms. With the use of TaqMan^{®} probes, real-time PCR offers an advantage of rapid, sensitive and specific detection of the target microorganisms, while avoiding cross-contamination from other closely related bacteria.

The TaqMan method depends on a DNA-based probe with a fluorescent reporter at one end and a quencher of fluorescence at opposite end of the probe. The close proximity of the reporter to the quencher prevents emission of its fluorescence, hydrolyzation of the probe by the 5' to 3' exonuclease activity of the Taq polymerase releases the reporter and thus allows emission of fluorescence. Therefore an increase of the product targeted by the reporter probe at each PCR cycle causes a proportional increase of fluorescence. Fluorescence is detected and measured in a real-time PCR machine.

An increasing number of alternative probe chemistries for quantitative real time PCR (qPCR) are being marketed besides TaqMan@ probes qPCR, such as minor groove binder (MGB), Molecular Beacon, Scorpion, locked nucleic acid (LNA) and Light Upon extension (LUX). The alternative probe technologies are based on different chemistries and claim to have some advantages compared with the conventional TaqMan^{®} DNA probes (Josefsen *et al.,* 2009).

Incorporation of locked nucleic acids (LNA) molecules in the probe has further helped to increase the sensitivity of these assays. LNA are nucleic acid analogs containing a locked bicyclic furanose unit in an RNA-mimicking sugar conformation. These molecules allow for better base stacking and, therefore, show a higher stability and affinity towards LNA, DNA and RNA targets.

LNA TaqMan@ probes have certain nucleic bases substituted by LNA monomers, i.e. nucleic acid analogs containing a 20-O,40-C methylenebridge, restricting the flexibility of the ribofuranose ring and rendering the monomer in a rigid bicyclic formation. This enhances the hybridization performance of LNA containing probes compared to classical TaqMan@ probes, and allows shorter probe designs. The incorporation of LNA monomers will increase the thermal stability of a duplex complementary DNA significantly, i.e. up to 8° C per substitution (Josefsen *et al.,* 2009).

The application of qPCR protocols based on the LNA TaqMan@ probe technology, combining group-specific short LNA TaqMan probes and species-specific primers designed on the H.P.M.E. *mleA-mleR* marker and H.P.M.E. *tkt-hrcA* marker and H.P.M.E. *hrcA-grpE* marker gives several advantages such as the possibility to use the same probe to quantify closely related species in distinct reactions

**Table 6. qPCR protocols based on the LNA TaqMan@ probe technology, combining short LNA TaqMan probe targeting microorganisms of the Lactobacillales order and species-specific primers designed on the H.P.M.E. mleA-mleR marker.**

| Species-specific Forward primer | Group specific LNA TaqMan@ probe | Species-specific Reverse primer |
|---|---|---|
| Target species: *Oenococcus oeni* | | |
| Oligonucleotide designed on the *O*. *oeni mleR* sequence | TTTGACTAT | Oligonucleotide designed on the *O. oeni mleA* sequence |

| Target species: *Lactobacillus plantarum* | | |
|---|---|---|
| Oligonucleotide designed on the *L. plantarum mleR* sequence | TTTGACTAT | Oligonucleotide designed on the *L. plantarum mleA* sequence |

| Target species: *Lactobacillus pentosus* | | |
|---|---|---|
| Oligonucleotide designed on the *L. pentosus mleR* sequence | TTTGACTAT | Oligonucleotide designed on the *L. pentosus mleA* sequence |

| Target species: *Lactobacillus casei* | | |
|---|---|---|
| Oligonucleotide designed on the *L. casei mleR* sequence | TTTGACTAT | Oligonucleotide designed on the *L. casei mleA* sequence |

| Target species: *Lactobacillus paracasei* | | |
|---|---|---|
| Oligonucleotide designed on the *L. paracasei mleR* sequence | TTTGACTAT | Oligonucleotide designed on the *L. paracasei mleA* sequence |

| Target species: *Lactobacillus rhamnosus* | | |
|---|---|---|
| Oligonucleotide designed on the *L rhamnosus mleR* sequence | TTTGACTAT | Oligonucleotide designed on the *L rhamnosus mleA* sequence |

| Target species: *Lactobacillus acidophilus* | | |
|---|---|---|
| Oligonucleotide designed on the *L. acidophilus mleR* | TTTGACTAT | Oligonucleotide designed on the *L. acidophilus mleA* |
| sequence | | sequence |
| | TTTGACTAT | |

| Target species: *Lactobacillus brevis* | | |
|---|---|---|
| Oligonucleotide designed on the *L. brevis mleR* sequence | TTTGACTAT | Oligonucleotide designed on the *L. brevis mleA* sequence |

| Target species: *Lactobacillus gasseri* | | |
|---|---|---|
| Oligonucleotide designed on the *L gasseri mleR* sequence | TTTGACTAT | Oligonucleotide designed on the *L. gasseri mleA* sequence |

| Target species: *Lactobacillus reuteri* | | |
|---|---|---|
| Oligonucleotide designed on the *L. reuteri mleR* sequence | TTTGACTAT | Oligonucleotide designed on the *L. reuteri mleA* sequence |

| Target species: *Lactobacillus sakei* | | |
|---|---|---|
| Oligonucleotide designed on the *L. sakei mleR* sequence | TTTGACTAT | Oligonucleotide designed on the *L. sakei mleA* sequence |

| Target species: *Lactobacillus curvatus* | | |
|---|---|---|
| Oligonucleotide designed on the *L. curvatus mleR* sequence | TTTGACTAT | Oligonucleotide designed on the *L. curvatus mleA* sequence |

| Target species: *Streptococcus salivarius* subsp. *thermophilus* | | |
|---|---|---|
| Oligonucleotide designed on the *S*. *salivarius* subsp. *thermophilus mleR* sequence | TTTGACTAT | Oligonucleotide designed on the *S*. *salivarius* subsp. *thermophilus mleA* sequence |

| Target species: *Leuconostoc mesenteroides* subsp. *mesenteroides* | | |
|---|---|---|
| Oligonucleotide designed on the *L. mesenteroides* | TTTGACTAT | Oligonucleotide designed on the . *mesenteroides* |
| subsp. *mesenteroides mleR* sequence | | subsp. *mleR* sequence |

| Target species: *Pediococcus damnosus* | | |
|---|---|---|
| Oligonucleotide designed on the *P. damnosus mleR* sequence | TTTGACTAT | Oligonucleotide designed on the *P. damnosus mleA* sequence |

**Table 7. qPCR protocols based on the LNA TaqMan@ probe technology, combining short LNA TaqMan probe targeting microorganisms of the Bifidobacterium genus and species-specific primers designed on the H.P.M.E. tkt-hrcA marker.**

| Species-specific Forward primer | Group specific LNA TaqMan@ probe | Species-specific Reverse primer |
|---|---|---|
| *Bifidobacterium longum subsp. longum* | | |
| Oligonucleotide designed on the *B. longum subsp. longum* sequence | ACAAGCCGG GAGTGCTAAT (SEQ ID NO: 25) | Oligonucleotide designed on the *B. longum subsp. longum* sequence |
| | AACACGCCAAA (SEQ ID NO: 23) | |
| | ATTGGAAGGAAAGTA (SEQ ID NO:24) | |
| | ATTGTATTAGCACTC (SEQ ID NO: 26) | |

**Table 8. qPCR protocols based on the LNA TaqMan@ probe technology, combining short LNA TaqMan probe targeting microorganisms of the Bacillus genus and species-specific primers designed on the H.P.M.E. hrcA- marker.**

| Species-specific Forward primer | Group specific LNA TaqMan@ probe | Species-specific Reverse primer |
|---|---|---|
| Target species: *Bacillus subtilis* subsp. *subtilis* | | |
| Oligonucleotide designed on the *B. subtilis* subsp. *subtilis* sequence | TTTGTTTTTCTTCT TGTGTTCACCTCCCT | Oligonucleotide designed on the *B. subtilis* subsp. *subtilis* sequence |

The present invention therefore resolves the above-lamented problem with reference to the mentioned prior art, offering at the same time numerous other advantages, including making possible the development of methods capable of identifying and quantifying prokaryotic microroganisms so to refine not only the diagnostics but above all direct the best therapeutic choice.

### References

Akopyanz N., Bukanov N.O., Westblom T.U., Kresovich S., Berg D.E. (1992) DNA diversity among clinical isolates of Helicobacter pylori detected by PCR-based RAPD fingerprinting. Nucleic Acids Research. 20: 5137-5142.
Esteve-Zarzoso B., Belloch C., Uruburu F., Querol A. (1999). Identification of yeasts by RFLP analysis of the 5.8S rRNA gene and the two ribosomal internal transcribed spacers. Int J Syst Bacteriology. 49: 329-337.
Ferrario C., Milani C., Mancabelli L., Lugli G.A., Turroni F., Duranti S., Mangifesta M., Viappiani A., Sinderen D.v., Ventura M. (2015). A genome-based identification approach for members of the genus Bifidobacterium. FEMS Microbiol Ecol. 91: 3.
Glaeser S.P., Kämpfer P. (2015). Multilocus sequence analysis (MLSA) in prokaryotic taxonomy. Syst Appl Microbiol 38: 237-245.
Hammes W.P. & Vogel R.F. (1995). The genus Lactobacillus. Chapter 3 in "The genera of Lactic acid Bacteria", Ed. by Wood and Holzapfel, pages: 19-54.
Gil-Lamaignere C., Roilides E., Hacker J., Müller F.M. (2003). Molecular typing for fungi--a critical review of the possibilities and limitations of currently and future methods. Clin Microbiol Infect. 9: 172-185.
Matsuki T., Watanabe K., Tanaka R., Fukuda M., Oyaizu H. (1999). Distribution of bifidobacterial species in human intestinal microflora examined with 16S rRNA-gene-targeted species-specific primers. Appl Environ Microbiol. 65: 4506-4512.
Moore E.R., Mihaylova S.A., Vandamme P., Krichevsky M.I., Dijkshoorn L. (2010). Microbial systematics and taxonomy: relevance for a microbial commons. Res Microbiol. 161: 430-438.
Shariat N., Kirchner M.K., Sandt C.H., Trees E., Barrangou R., Dudley E.G. (2013). Subtyping of Salmonella enterica serovar Newport outbreak isolates by CRISPR-MVLST and determination of the relationship between CRISPR-MVLST and PFGE results. J. Clin. Microbiol. 51: 2328-2336.
Shaw G. (2013). Polymorphism and single nucleotide polymorphisms (SNPs). BJU Int. 112: 664-665.
Thanh H.D., Park H.K., Kim W., Shin H.S. (2013). Development of a 16S-23S rRNA intergenic spacer-based quantitative PCR assay for improved detection and enumeration of Lactococcus garvieae. FEMS Microbiol Lett 339: 10-16.
Walter J., Tannock G.W., Tilsala-Timisjarvi A., Rodtong S., Loach D.M., Munro K., Alatossava T. (2000) Detection and identification of gastrointestinal Lactobacillus species by using denaturing gradient gel electrophoresis and species-specific PCR primers. Appl Environ Microbiol. 66: 297-303.
Ward L.J., Timmins M.J. (1999). Differentiation of Lactobacillus casei, Lactobacillus paracasei and Lactobacillus rhamnosus by polymerase chain reaction. Lett Appl. Microbiol. 29: 90-92.
Beltramo C, Desroche N, Tourdot-Marechal R, Grandvalet C, Guzzo J. (2006). Real-time PCR for characterizing the stress response of Oenococcus oeni in a wine-like medium. Res Microbiol. 157(3):267-274.
Bilhere E., Lucas P.M., Claisse O., Lonvaud-Funel A. (2009). Multilocus sequence typing of Oenococcus oeni: detection of two subpopulations shaped by intergenic recombination. Appl Environ Microbiol. 2009 Mar;75(5):1291-300.
de Las Rivas B., Marcobal A., Muñoz R. (2004). Allelic diversity and population structure in Oenococcus oeni as determined from sequence analysis of housekeeping genes. Appl Environ Microbiol. 70(12):7210-7219.
Dimopoulou M., Vuillemin M., Campbell-Sills H., Lucas P.M., Ballestra P., Miot-Sertier C., Favier M., Coulon J., Moine V., Doco T., Roques M., Williams P., Petrel M., Gontier E., Moulis C., Remaud-Simeon M., Dols-Lafargue M. (2014). Exopolysaccharide (EPS) synthesis by Oenococcus oeni: from genes to phenotypes. PLoS One. 2014 Jun 5;9(6):e98898.
Divol, B., T. Tonon, S. Morichon, E. Gindreau, and A. Lonvaud-Funel. 2003. Molecular characterization of Oenococcus oeni genes encoding proteins involved in arginine transport. J. Appl. Microbiol. 94:738-746.
Groisillier A., Lonvaud-Funel A. (1999). Comparison of partial malolactic enzyme gene sequences for phylogenetic analysis of some lactic acid bacteria species and relationships with the malic enzyme. Int J Syst Bacteriol. 49:1417-1428.
Zapparoli G., Torriani S., Pesente P., Dellaglio F. (1998). Design and evaluation of malolactic enzyme gene targeted primers for rapid identification and detection of Oenococcus oeni in wine. Lett Appl Microbiol. 27(5): 243-246.
de Las Rivas B1, Marcobal A, Muñoz R. (2004) Allelic diversity and population structure in Oenococcus oeni as determined from sequence analysis of housekeeping genes Appl Environ Microbiol. Dec;70(12):7210-9
Leclerque A, Hartelt K, Schuster C, Jung K, Kleespies RG. (2011) Multilocus sequence typing (MLST) for the infra-generic taxonomic classification of entomopathogenic Rickettsiella bacteria FEMS Microbiol Lett. Nov;324(2):125-34.
Fournier P.E., Suhre K., Fournous G., Raoult D. (2006). Estimation of prokaryote genomic DNA G+C content by sequencing universally conserved genes. Int J Syst Evol Microbiol 56: 1025-1029.
Leclerque A. (2008). Whole genome-based assessment of the taxonomic position of the arthropod pathogenic bacterium Rickettsiella grylli. FEMS Microbiol 283:117-127. Mediannikov O., Sekeyova Z., Birg M.L., Raoult D. (2010). A novel obligate intracellular gamma-proteobacterium associated with ixodid ticks, Diplorickettsia massiliensis, Gen. Nov., Sp. Nov. PLoS One. 5(7):e11478.
Assis L.C.S. & Santos, L.M. (2014). Phylogenetics is not phylogenomics. Cladistics 30:8-9.
Bustin S.A., Benes V., Garson J.A., Hellemans J., Huggett J., Kubista M., Mueller R., Nolan T., Pfaffl M.W., Shipley G.L., Vandesompele J., Wittwer C.T. (2009). The MIQE guidelines: minimum information for publication of quantitative real-time PCR experiments. Clin Chem. 55(4): 611-622.
Capella-Gutierrez S, Kauff F, Gabaldon T. (2014). A phylogenomics approach for selecting robust sets of phylogenetic markers. Nucleic Acids Res. 42(7):e54.
Josefsen MH, Löfström C, Sommer HM, Hoorfar J. (2009). Diagnostic PCR: comparative sensitivity of four probe chemistries. Mol Cell Probes. 23:201-203.
Jun S.R., Nookaew I., Hauser L., Gorin A. (2017). Assessment of genome annotation using gene function similarity within the gene neighborhood. BMC Bioinformatics. 18(1):345.
Poptsova M.S., Gogarten J.P. (2010). Using comparative genome analysis to identify problems in annotated microbial genomes. Microbiology. 156:1909-1917.
Rong & Huang (2014) Multilocus Sequence Analysis: Taking Prokaryotic Systematics to the Next Level. Chapter 11 in Methods in Microbiology 41:221-251.
Schumann W. (2003). The Bacillus subtilis heat shock stimulon. Cell Stress Chaperones. Fall 8:207-217.
Schaeffer R.D., Liao Y., Cheng H., Grishin N.V. (2017). ECOD: new developments in the evolutionary classification of domains. Nucleic Acid Research. 45: 296-302.
Bateman A. & Finn R.D. (2007) SCOOP: simple method for identification of novel protein superfamily relationships. Bioinformatics. 2007;23(7):809-814
Mistry J., Bateman A., Finn R.D. (2007). Predicting active site residue annotations in the Pfam database. BMC Bioinformatics. 8:298.
Maddocks S.E., Oyston P.C. (2008). Structure and function of the LysR-type transcriptional regulator (LTTR) family proteins. Microbiology. 154:3609-3623.

## Claims

1. A Highly Polymorphic and Modular Extragenic (H.P.M.E.) marker, said H.P.M.E. being a microbial genome nucleotide sequence having a length of less than or equal to 2000bp, and said HPME comprising two genetic elements, at least one of which is a predicted Open Reading Frame coding for a protein, a hypothetical protein, or a pseudogene, wherein said two genetic elements are divided by a noncoding nucleotide sequence corresponding to a promoter region, wherein said H.P.M.E. marker allows the identification and quantification of microorganisms and wherein said two genetic elements are respectively:
- the gene mleA coding for the Malolactic enzyme and the gene mleR coding for the mleA transcriptional regulator, belonging to the LysR-type transcriptional regulator family, namely H.P.M.E. mleA-mleR marker, for the identification and quantification of microorganisms within the Lactobacillales order;
- the gene tkt coding for the Transketolase enzyme and the gene hrcA coding for the HrcA transcriptional regulator, namely H.P.M.E. tkt-hrcA marker, for the identification and quantification of microorganisms within the Bifidobacterium genus;
- the gene grpE coding for the Heat Shock Protein GrpE and the gene hrcA coding for the HrcA transcriptional regulator, namely H.P.M.E. hrcA-grpE marker, for the identification and quantification of microorganisms within the Bacillus genus.

2. The marker according to claim 1, wherein said microbial genome nucleotide sequence is a prokaryotic sequence, and wherein said prokaryotic sequence belongs to a microorganism of the genus chosen from the group consisting of *Oenococcus, Lactobacillus, Lactococcus, Leuconostoc, Pediococcus, Streptococcus, Fructobacillus, Weisella, Enterococcus, Bifidobacterium, Bacillus,*

3. The marker according to claim 1, wherein said microbial genome nucleotide sequence is a bacterial sequence, and wherein bacterial sequence belongs to a bacterium of the genus chosen from the group consisting of *Lactobacillus, Leuconostoc* and *Pediococcus Streptococcus, Enterococcus, Bifidobacterium* and *Bacillus*

4. The marker according to anyone of claims 1 to 3 wherein said two genetic elements are:
- oriented in the same direction on the same strand of DNA;
- oriented in the opposite direction on the two complementary strands of DNA.

5. The use of the Highly Polymorphic and Modular Extragenic (HPME) marker, according to anyone of claims 1 to 4 for the identification of the genus and/or the species and/or subspecies or any of the Operational Taxonomic Unit (OTU) of a microorganism.

6. The use of the Highly Polymorphic and Modular Extragenic (HPME) marker, according to anyone of claims 1 to 4 for the identification and quantification of a target microorganisms.

7. The use of the Highly Polymorphic and Modular Extragenic (HPME) marker, according to claim 6 wherein said identification and quantification of microorganisms is carried out with HPME marker specific primers and probes

8. The use according to claim 7 wherein said primers and probes are for PCR, rtPCR, qPCR, LAMP-PCR, Next Generation Sequencing, molecular applications mediated by hybridization, preferably qPCR, cytofluorimetry, and cell sorting, Fluorescent In Situ Hybridization (FISH), FISH-FLOW.

9. The use according to claim 6 wherein said identification and quantification of microorganisms is carried out with:
- the target markers consisting of the sequences ACAAGCCGG, GAGTGCTAAT (SEQ ID NO: 25), AACACGCCAAA (SEQ ID NO: 23), ATTGGAAGGAAAGTA (SEQ ID NO:24), ATTGTATTAGCACTC (SEQ ID NO: 26), within the H.P.M.E. *tkt-hrcA* marker, for the *Bifidobacterium* genus;
- the target markers consisting of the sequence TTTGTTTTTCTTCT, TGTGTTCACCTCCCT within the H.P.M.E. *hrcA-grpE* marker; for the *Bacillus* genus.

10. The use according to claim 9, wherein the microorganism is a bacterium and is chosen from the group consisting of the genera *Bifidobacterium* and *Bacillus.*

## Patentansprüche

1. Hochpolymorpher und modularer extragenischer (H.P.M.E.)-Marker, wobei der H.P.M.E. eine mikrobielle Genom-Nukleotidsequenz mit einer Länge von weniger als oder gleich 2000 bp ist und der HPME zwei genetische Elemente umfasst, von denen mindestens eines ein vorhergesagter offener Leserahmen ist, der für ein Protein, ein hypothetisches Protein oder ein Pseudogen kodiert, wobei die beiden genetischen Elemente durch eine nichtkodierende Nukleotidsequenz geteilt sind, die einer Promotorregion entspricht, wobei der H.P.M.E.-Marker die Identifizierung und Quantifizierung von Mikroorganismen ermöglicht und wobei die beiden genetischen Elemente jeweils Folgendes sind:
- das Gen mleA, das für das malolaktische Enzym kodiert, und das Gen mleR, das für den mleA-Transkriptionsregulator kodiert, der zur Familie der Transkriptionsregulatoren vom LysR-Typ gehört, nämlich H.P.M.E.-mleA-mleR-Marker, zur Identifizierung und Quantifizierung von Mikroorganismen innerhalb der Ordnung der Lactobacillales;
- das Gen tkt, das für das Enzym Transketolase kodiert, und das Gen hrcA, das für den HrcA-Transkriptionsregulator kodiert, nämlich H.P.M.E.-tkt-hrcA-Marker, zur Identifizierung und Quantifizierung von Mikroorganismen innerhalb der Gattung Bifidobacterium;
- das Gen grpE, das für das Hitzeschockprotein GrpE kodiert, und das Gen hrcA, das für den HrcA-Transkriptionsregulator kodiert, nämlich den H.P.M.E.-HRcA-grpE-Marker, zur Identifizierung und Quantifizierung von Mikroorganismen innerhalb der Gattung Bacillus.

2. Marker nach Anspruch 1, wobei die mikrobielle Genom-Nukleotidsequenz eine prokaryotische Sequenz ist und wobei die prokaryotische Sequenz zu einem Mikroorganismus der Gattung gehört, ausgewählt aus der Gruppe bestehend aus *Oenococcus, Lactobacillus, Lactococcus, Leuconostoc, Pediococcus, Streptococcus, Fructobacillus, Weisella, Enterococcus, Bifidobacterium, Bacillus.*

3. Marker nach Anspruch 1, wobei die Nukleotidsequenz des mikrobiellen Genoms eine bakterielle Sequenz ist und wobei die bakterielle Sequenz zu einem Bakterium der Gattung gehört, ausgewählt aus der Gruppe bestehend aus *Lactobacillus, Leuconostoc* und *Pediococcus Streptococcus, Enterococcus, Bifidobacterium* und *Bacillus.*

4. Marker nach einem der Ansprüche 1 bis 3, wobei die beiden genetischen Elemente:
- auf demselben DNA-Strang in derselben Richtung ausgerichtet sind;
- auf den beiden komplementären DNA-Strängen in entgegengesetzter Richtung ausgerichtet sind.

5. Verwendung des hochpolymorphen und modularen extragenen (HPME)-Markers nach einem der Ansprüche 1 bis 4 zur Identifizierung der Gattung und/oder der Spezies und/oder der Subspezies oder einer der Operational Taxonomic Units (OTU) eines Mikroorganismus.

6. Verwendung des hochpolymorphen und modularen extragenen (HPME)-Markers nach einem der Ansprüche 1 bis 4 zur Identifizierung und Quantifizierung eines Zielmikroorganismus.

7. Verwendung des hochpolymorphen und modularen extragenen (HPME)-Markers nach Anspruch 6, wobei die Identifizierung und Quantifizierung von Mikroorganismen mit HPME-Marker-spezifischen Primern und Sonden durchgeführt wird.

8. Verwendung nach Anspruch 7, wobei die Primer und Sonden für PCR, rtPCR, qPCR, LAMP-PCR, Next-Generation-Sequencing, molekulare Anwendungen durch Hybridisierung, vorzugsweise qPCR, Zytofluorimetrie und Zellsortierung, Fluoreszenz-In-Situ-Hybridisierung (FISH), FISH-FLOW sind.

9. Verwendung nach Anspruch 6, wobei die Identifizierung und Quantifizierung von Mikroorganismen durchgeführt wird mit:
- den Zielmarkern bestehend aus den Sequenzen ACAAGCCGG, GAGTGCTAAT (SEQ ID NO: 25), AACACGCCAAA (SEQ ID NO: 23), ATTGGAAGGAAAGTA (SEQ ID NO:24), ATTGTATTAGCACTC (SEQ ID NO: 26) innerhalb des H.P.M.E.-tkt-hrcA-Markers für die Gattung *Bifidobacterium,*
- den Zielmarkern, bestehend aus der Sequenz TTTGTTTTTTCTTCT, TGTGTTCACCTCCCT innerhalb des H.P.M.E.-*hrcA*-*grpE*-Markers für die Gattung *Bacillus.*

10. Verwendung nach Anspruch 9, wobei der Mikroorganismus ein Bakterium ist und ausgewählt ist aus der Gruppe bestehend aus den Gattungen *Bifidobacterium* und *Bacillus.*

## Revendications

1. Marqueur hautement polymorphe et modulaire extragénique (H.P.M.E.), ledit H.P.M.E. étant une séquence nucléotidique du génome microbien d'une longueur inférieure ou égale à 2000 pb, et ledit HPME comprenant deux éléments génétiques, dont au moins un est un cadre de lecture ouvert prédit codant pour une protéine, une protéine hypothétique ou un pseudogène, dans lequel lesdits deux éléments génétiques sont divisés par une séquence nucléotidique non codante correspondant à une région promotrice, dans lequel ledit marqueur H.P.M.E. permet l'identification et la quantification des micro-organismes et dans lequel lesdits deux éléments génétiques sont respectivement :
- le gène mleA codant pour l'enzyme malolactique et le gène mleR codant pour le régulateur transcriptionnel mleA, appartenant à la famille des régulateurs transcriptionnels de type LysR, à savoir le marqueur H.P.M.E. mleA-mleR, pour l'identification et la quantification des micro-organismes de l'ordre des Lactobacillales ;
- le gène tkt codant pour l'enzyme transcétolase et le gène hrcA codant pour le régulateur transcriptionnel HrcA, à savoir le marqueur H.P.M.E. tkt-hrcA, pour l'identification et la quantification des micro-organismes du genre Bifidobacterium ;
- le gène grpE codant pour la protéine de choc thermique GrpE et le gène hrcA codant pour le régulateur transcriptionnel HrcA, à savoir le marqueur H.P.M.E. hrcA-grpE, pour l'identification et la quantification des micro-organismes du genre Bacillus.

2. Marqueur selon la revendication 1, dans lequel ladite séquence nucléotidique du génome microbien est une séquence procaryote, et dans lequel ladite séquence procaryote appartient à un micro-organisme du genre choisi dans le groupe constitué de *Oenococcus, Lactobacillus, Lactococcus, Leuconostoc, Pediococcus, Streptococcus, Fructobacillus, Weisella, Enterococcus, Bifidobacterium, Bacillus,*

3. Marqueur selon la revendication 1, dans lequel ladite séquence nucléotidique du génome microbien est une séquence bactérienne, et dans lequel la séquence bactérienne appartient à une bactérie du genre choisi dans le groupe constitué de *Lactobacillus, Leuconostoc* et *Pediococcus Streptococcus, Enterococcus, Bifidobacterium* et *Bacillus.*

4. Marqueur selon l'une quelconque des revendications 1 à 3, dans lequel lesdits deux éléments génétiques sont :
- orientés dans la même direction sur le même brin d'ADN ;
- orientés dans la direction opposée sur les deux brins complémentaires d'ADN.

5. Utilisation du marqueur hautement polymorphe et modulaire extragénique (HPME) selon l'une quelconque des revendications 1 à 4 pour l'identification du genre et/ou de l'espèce et/ou de la sous-espèce ou de l'unité taxonomique opérationnelle (OTU) d'un micro-organisme.

6. Utilisation du marqueur hautement polymorphe et modulaire extragénique (HPME), selon l'une quelconque des revendications 1 à 4, pour l'identification et la quantification d'un micro-organisme cible.

7. Utilisation du marqueur hautement polymorphe et modulaire extragénique (HPME) selon la revendication 6, dans laquelle l'identification et la quantification des micro-organismes sont effectuées à l'aide d'amorces et de sondes spécifiques du marqueur HPME.

8. Utilisation selon la revendication 7, dans laquelle lesdites amorces et lesdites sondes sont destinées à la PCR, à la rtPCR, à la qPCR, à la LAMP-PCR, au séquençage de nouvelle génération, aux applications moléculaires médiées par hybridation, de préférence à la qPCR, à la cytofluorimétrie et au tri cellulaire, à l'hybridation fluorescente in situ (FISH), à la FISH-FLOW.

9. Utilisation selon la revendication 6, dans laquelle l'identification et la quantification des micro-organismes sont effectuées avec :
- les marqueurs cibles constitués des séquences ACAAGCCGG, GAGTGCTAAT (SEQ ID NO: 25), AACACGCCAAA (SEQ ID NO: 23), ATTGGAAGGAAAGTA (SEQ ID NO:24), ATTGTATTAGCACTC (SEQ ID NO: 26), au sein du marqueur H.P.M.E. *tkt-hrcA* , pour le genre *Bifidobacterium* ;
- les marqueurs cibles consistant en la séquence TTTGTTTCTTCT, TGTGTTCACCTCCCT à l'intérieur du marqueur H.P.M.E. *hrcA-grpE* ; pour le genre *Bacillus.*

10. Utilisation selon la revendication 9, dans laquelle le micro-organisme est une bactérie choisie dans le groupe constitué des genres *Bifidobacterium* et *Bacillus.*
